# EUROPÄISCHE PATENTSCHRIFT

(11) **EP 0 527 736 B1**
(45) Veröffentlichungstag und Bekanntmachung des Hinweises auf die Patenterteilung: **16.04.1997**
(21) Anmeldenummer: 90915462.7
(22) Anmeldetag: 24.10.1990
(51) Int. Cl.: A61K 31/275, A61K 31/42, C07C 255/21, C07C 255/23, C07D 261/18, C07D 317/66, C07D 413/12

(54) **ISOXAZOL-4-CARBONSÄUREAMIDE UND HYDROXYALKYLIDEN-CYANESSIGSÄUREAMIDE, DIESE VERBINDUNGEN ENTHALTENDE ARZNEIMITTEL UND DEREN VERWENDUNG**
ISOXAZOLE-4-CARBOXAMIDES AND HYDROXYALKYLIDENE-CYANOACETAMIDES, DRUGS CONTAINING THESE COMPOUNDS AND USE OF SUCH DRUGS
AMIDES D'ACIDE CARBOXYLIQUE-4 D'ISOXAZOL ET CYANAMIDES ACETIQUES D'HYDROXYALKYLIDENE, MEDICAMENTS CONTENANT CES COMPOSES ET LEUR APPLICATION

(30) Priorität: 18.05.1990 DE 4016178; 26.05.1990 DE 4017020; 26.05.1990 DE 3017043
(43) Veröffentlichungstag der Anmeldung: 24.02.1993
(73) Patentinhaber: HOECHST AKTIENGESELLSCHAFT, 65926 Frankfurt am Main (DE)
(72) Erfinder: BARTLETT, Robert, R., D-6100 Darmstadt (DE); KÄMMERER, Friedrich-Johannes, D-6203 Hochheim am Main (DE)
(86) Internationale Anmeldenummer: EP9001800
(87) Internationale Veröffentlichungsnummer: WO9117748

(56) Entgegenhaltungen:
- EP-A- 0 013 376
- EP-A- 0 217 206
- EP-A- 0 257 882
- EP-A- 0 274 443
- DE-A- 2 524 929
- DE-A- 2 854 439
- NL-A- 7 605 841
- Int. J. Immunopharmac., Band 8, Nr. 2, 1986, International Society for Immunopharmacology, GB; R.R. Bartlett: "Immunopharmacological profile of HWA 486, a novel isoxazol derivate - in vivo immunomodulating effects differ from those of cyclophosphamide, prednisolone, or cyclosporin A", Seiten 199-204
- Scand J. Rheumatology, supplement 75, 1988, SE; R.R. Bartlett et al.: "Development of auto immunit in MRL/Ipr mice and the effects of drugs on this murine disease", Seiten 290-299

## Beschreibung

In der Literatur sind eine Reihe von Verfahren zur Herstellung von Isoxazol-4-carbonsäureamide beschrieben worden (DE 25 24 959; DE 26 55 009; DE 34 05 727).

Aus der Europäischen Patentschrift 13 376 ist bekannt, daß 5-Methylisoxazol-4-carbonsäure-(4-trifluormethyl)-anilid aufgrund seiner pharmakologischen Eigenschaften als Antirheumatikum, Antiphlogistikum, Antipyretikum und Analgeticum eingesetzt werden kann, sowie zur Behandlung der multiplen Sklerose Verwendung findet. Dort sind ebenfalls Verfahren zur Herstellung dieser Verbindung beschrieben.

Es wurde nun gefunden, daß Isoxazol-4-carbonsäureamide der Formel I und Hydroxyalkylidencyanessigsäureamide der Formel Ia und ihrer tautomeren Form Ib Antitumoraktivität aufweisen. Viele der bekannten Antitumormittel erzeugen während der Therapie als Nebenwirkungen Übelkeit, Erbrechen oder Durchfall, die auch eine ärztliche Behandlung im Krankenhaus nötig machen. Ferner verändern diese Arzneimittel auch die Wachstumsgeschwindigkeit von anderen körpereigenen Zellen, welche dann zu Symptomen wie beispielsweise Haarausfall oder Blutarmut (Anämie) fuhrt. Diese Symptome konnten bei der Behandlung von Menschen und Tieren mit den Verbindungen der Formel I nicht beobachtet werden. Diese Wirkstoffe haben im Gegensatz zu den bisher bekannten cytotoxischen Antikrebsmitteln nicht die Eigenschaft das Immunsystem zu beeinträchtigen (Bartlett, Int. J. Immunopharmac., 1986, 8: 199-204). Damit eröffnen sich neue Wege der Tumortherapie, denn das körpereigene Abwehrsystem wird nicht beeinträchtigt, während Tumorzellen am Wachstum gehindert werden. Überraschenderweise werden eine Vielzahl von Tumorzellen durch diese Wirkstoffe gehemmt, während Zellen des Immunsystems, wie z.B. T-Lymphozyten nur bei einer bis zu 50-fach höheren Konzentration gehemmt werden.

Die Erfindung betrifft daher die Verwendung von mindestens einer Verbindung der Formel I, Ia oder Ib 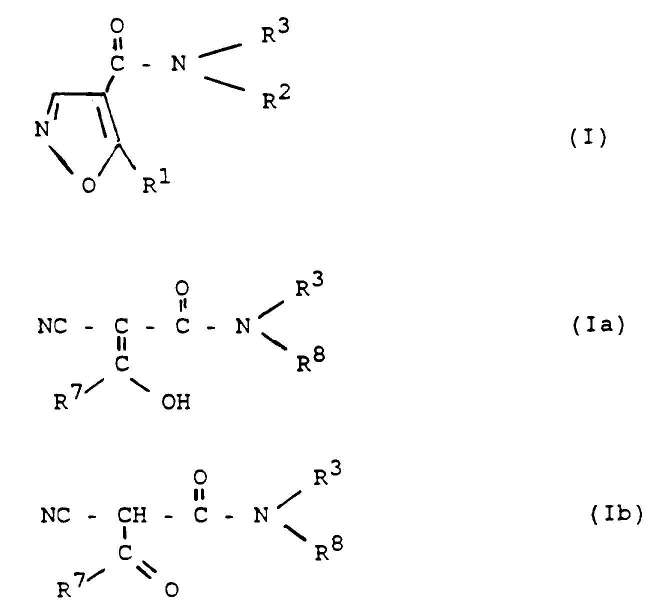 ihre gegebenenfalls stereoisomeren Formen und/oder gegebenenfalls mindestens einem von deren physiologisch verträglichen Salzen, wobei
R¹ für
a) Wasserstoff,
b) Alkyl mit 1 bis 6 C-Atomen,
c) Alkyl mit 1 bis 4 C-Atomen, ein- oder mehrfach substituiert durch
   1) Halogen, wie Fluor, Chlor, Brom oder Jod,
d) Phenyl,
R² für
a) Wasserstoff,
b) Alkyl mit 1 bis 4 C-Atomen,
c) Phenyl-(C₁-C₂)-alkyl, insbesondere Benzyl,
d) Alkenyl mit 2 bis 3 C-Atomen,
R³ für
a) einen ein-, zwei- oder dreikernigen, ungesättigten heterocyclischen Rest mit 3 bis 13 C-Atomen und 1 bis 4 Heteroatomen aus der Gruppe Sauerstoff, Schwefel und Stickstoff, wovon höchsten eines von Stickstoff verschieden ist, im Ringsystem bedeutet, unsubstituiert oder ein- oder mehrfach substituiert durch,
   1) Halogen, wie Fluor, Chlor, Brom oder Jod,
   2) Alkyl mit 1 bis 3 C-Atomen,
   3) Alkyl mit 1 bis 3 C-Atomen, ein- oder mehrfach substituiert durch
      3.1 Halogen, wie Fluor, Chlor, Brom oder Jod,
   4) Alkoxy mit 1 bis 3 C-Atomen,
   5) Alkoxy mit 1 bis 3 C-Atomen, ein- oder mehrfach substituiert durch
      5.1 Halogen, wie Fluor, Chlor, Brom oder Jod,
   6) Nitro,
   7) Hydroxy,
   8) Carboxy,
   9) Carbamoyl,
   10) Oxogruppe,
b) einen Rest der Formel II, 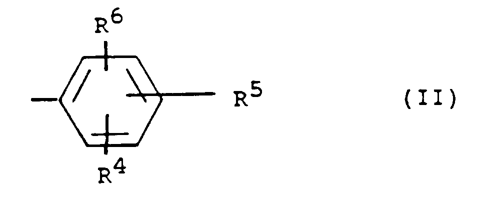 in der R⁴, R⁵ und R⁶ gleich oder verschieden sein können und für
   1) Wasserstoff,
   2) Phenoxy,
   3) Alkyl mit 1 bis 3 C-Atomen, ein- oder mehrfach substituiert durch
      3.1 Halogen, wie Fluor, Chlor, Brom oder Jod,
   4) in der R⁴ für Wasserstoff und R⁵ und R⁶ gemeinsam mit dem Phenylring der Formel II einen Naphthalinring bilden,
   5) in der R⁴ für Wasserstoff und R⁵ und R⁶ einen Methylendioxyrest bilden,
   6) Alkoxy mit 1 bis 3 C-Atomen,
   7) Alkoxy mit 1 bis 3 C-Atomen, ein- oder mehrfach substituiert durch
      7.1 Halogen, wie Fluor, Chlor, Brom oder Jod,
   8) (C₁-C₃)-Alkylmercapto,
   9) (C₁-C₃)-Alkylmercapto, ein- oder mehrfach substituiert durch
      9.1 Halogen, wie Fluor, Chlor, Brom oder Jod,
   10) Halogen, wie Fluor, Chlor, Brom oder Jod,
   11) Nitro,
   12) Cyano,
   13) Hydroxy,
   14) Carboxy,
   15) (C₁-C₃)-Alkylsulfonyl,
   16) Carbalkoxy, mit 1 bis 3 C-Atomen in der Alkylkette,
   17) Benzoyl,
   18) Benzoyl, ein- oder mehrfach substituiert durch
      18.1 Halogen, wie Fluor, Chlor, Brom oder Jod,
      18.2 (C₁-C₃)-Alkyl,
      18.3 (C₁-C₃)-Alkoxy,
   19) Phenyl,
   20) Phenyl, ein- oder mehrfach substituiert durch
      20.1 (C₁-C₃)-Alkoxy,
      20.2 Halogen, wie Fluor, Chlor, Brom oder Jod,
      20.3 (C₁-C₃)-Alkyl,
   21) Phenoxy, ein- oder mehrfach substituiert durch
      21.1 (C₁-C₃)-Alkoxy, ein- oder mehrfach substituiert durch
         21.1.1 Halogen, wie Fluor, Chlor, Brom oder Jod,
      21.2 Halogen, wie Fluor, Chlor, Brom oder Jod,
      21.3 (C₁-C₃)-Alkyl, ein- oder mehrfach substituiert durch
         21.3.1 Halogen, wie Fluor, Chlor, Brom oder Jod,
c) einen Rest der Formel III,

   -(CH₂)ₙ-COOR¹⁰ (III)

   in der R¹⁰ für
   1) Wasserstoff
   2) Alkyl mit 1 bis 4 C-Atomen,
      n für eine ganze Zahl von 1 bis 12,
d) R² und R³ zusammen mit dem Stickstoff, an das sie gebunden sind, einen 4- bis 9-gliedrigen Ring bilden, substituiert durch,
   1) Carbonyl am N-Atom benachbarten C-Atom,
e) R² und R³ zusammen mit dem Stickstoff, an das sie gebunden sind, einen 5- bis 6-gliedrigen Ring der Formel IV bilden, 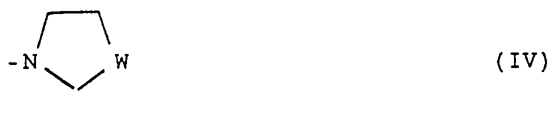 in der W für
   1) - CH₂-,
   2) -CH₂-CH₂-,
   3) 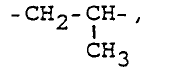
   4) 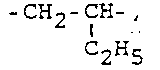
   5) 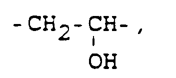
   6) -CH₂-O- oder
   7) -CH₂-S-
R⁷ für
a) Wasserstoff,
b) Alkyl mit 1 bis 17 C-Atomen,
c) Alkyl mit 1 bis 3 C-Atomen, ein- oder mehrfach substituiert durch Halogen wie Fluor, Chlor, Brom oder Jod,
d) Phenyl-(C₁-C₂)-alkyl, insbesondere Benzyl;
R⁸ für
a) Wasserstoff,
b) Methyl,
c) Alkenyl mit 2 bis 3 C-Atomen,
steht zur Herstellung von Arzneimitteln zur Behandlung von Krebserkrankungen.

Unter diesen Arzneimitteln sind bevorzugt die Verbindung 5-Methylisoxazol-4-carbonsäure-(4-trifluormethyl)anilid (Verbindung 1) und N-(4-Trifluormethyl)-2-cyan-3-hydroxycrotonsäureamid (Verbindung 2).

Geeignete physiologisch verträgliche Salze der Verbindung der Formel I sind beispielsweise Alkali-, Erdalkali- und Ammoniumsalze einschließlich solcher von organischen Ammoniumbasen.

Zu den ein-, zwei- oder dreikernigen, ungesättigten, heterocyclischen Resten mit 3 bis 13 C-Atomen gehören beispielsweise Thienyl, Pyridyl, Pyrimidinyl, Pyrazinyl, Pyridazinyl, Imidazolyl, Thiazolyl, Thiazolinyl, Oxazolyl, Thiadiazolyl, Benzoxazolyl, Benzimidazolyl, Chinolyl, Pyrazolyl, Acridinyl, Indolyl, Tetrazolyl oder Indazolyl.

Die Verbindung der Formel I und deren physiologisch verträgliche Salze eignen sich in besonderer Weise zur Behandlung von einer Vielzahl von Krebserkrankungen. Zu den Krebsarten die besonders durch diese Verbindungen gehemmt werden, gehören beispielsweise Leukämie, insbesondere chronische Leukämie des T- und B-Zelltyps, Lymphknotenkrebs, z.B. Hodgkin's oder non-Hodgkin's Lymphom, Karzinome, Sarkome oder Hautkrebs. Die Wirkstoffe können entweder für sich alleine, beispielsweise in Form von Mikrokapseln, in Mischungen miteinander oder in Kombination mit geeigneten Hilfs-und/oder Trägerstoffen verabreicht werden.

Die Herstellung der Verbindung der Formel I, Ia oder Ib erfolgt auf bekannte Weise (DE 2 524 959; DE 2 655 009; DE 3 405 727; DE 2 524 929; DE 2 555 789; DE 2 557 003).

Die Verbindungen der Formel I, Ia oder Ib lassen sich herstellen, indem man
a) eine Verbindung der Formel V, 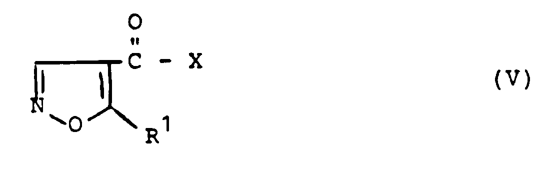 in der X für ein Halogenatom, vorzugsweise Chlor oder Brom steht und R¹ die in Formel I angegebene Bedeutung hat,
   mit dem Amin der Formel VI 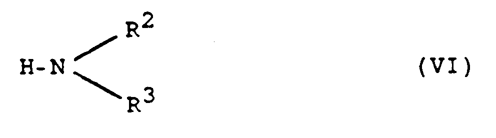 worin R² und R³ die in Formel I angegebene Bedeutung hat, umsetzt, oder
b) eine Verbindung der Formel VI 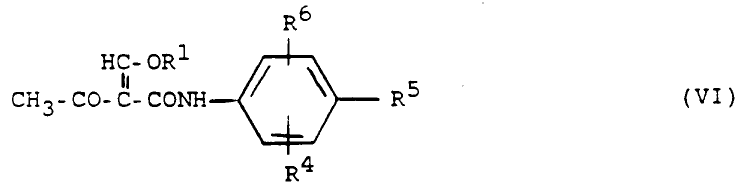 worin R¹ für (C₁-C₄)-Alkyl steht und R⁴, R⁵ und R⁶ die in Formel I angegebene Bedeutung haben, mit einer zweckmäßig mindestens äquimolaren Menge Hydroxylamin in einem organischen Lösungsmittel behandelt, oder
c) eine Verbindung der Formel V,
   in der X und R¹ die obengenannte Bedeutung haben, mit einem primären aliphatischen Amin der Formel VII

   H₂N - (CH₂)ₙ - COOR¹⁰ (VII)

   worin n und R¹⁰ die in Formel I angegebene Bedeutung haben, umsetzt, oder
d) eine Verbindung der Formel V,
   in der X und R¹ die obengenannte Bedeutung haben, mit einem Lactam der Formel VIII, 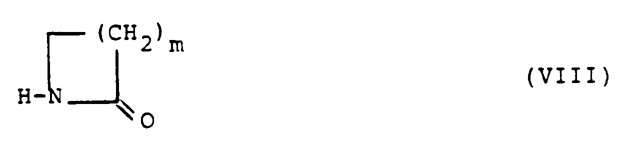 in der m eine ganze Zahl von 1 bis 6 bedeutet, umsetzt, oder
e) eine Verbindung der Formel V,
   in der X und R¹ die obengenannte Bedeutung haben, mit einem Amin der Formel IX, 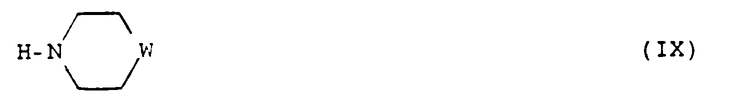 in der W die in Formel I angegebene Bedeutung hat, umsetzt, oder
f) die Verbindung der Formel I in Gegenwart eines basischen Mittels in die entsprechende Verbindung der Formel Ia oder Ib umsetzt.

Ein weiterer Gegenstand der Erfindung sind neue Verbindungen der Formel I, 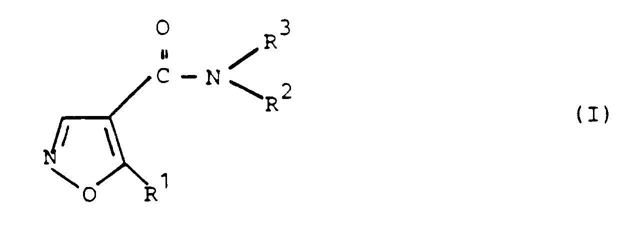 ihre gegebenenfalls stereoisomeren Formen und/oder gegebenenfalls ihre physiologisch verträglichen Salze, wobei
R¹ für
a) Wasserstoff,
b) Alkyl, mit 2 bis 6 C-Atomen,
c) Alkyl mit 1 bis 4 C-Atomen, ein- oder mehrfach substituiert durch
   1) Halogen, wie Fluor, Chlor, Brom oder Jod,
d) Phenyl,
R² für
a) Wasserstoff,
b) Alkenyl mit 2 bis 3 C-Atomen,
c) Benzyl,
R³ für
a) Pyridyl ein- oder mehrfach substituiert durch,
   1) Wasserstoff,
   2) Halogen, wie Fluor, Chlor, Brom oder Jod,
   3) Nitro,
   4) Alkyl mit 1 bis 3 C-Atomen,
   5) Alkoxy mit 1 bis 3 C-Atomen,
b) einen Rest der Formel II, 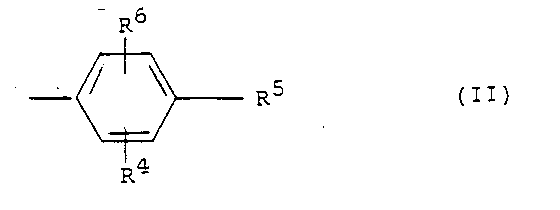 in der R⁴, R⁵, R⁶ gleich oder verscheiden sein können und für
   1) Halogen, wie Fluor, Chlor, Brom oder Jod,
   2) Nitro,
   3) Wasserstoff,
   4) Benzoyl ein- oder mehrfach substituiert durch
      4.1 Halogen, wie Fluor, Chlor, Brom oder Jod,
      4.2 Methyl,
      4.3 Methoxy,
      4.4 Benzoyl,
   5) (C₁-C₃)-Alkoxy, ein- oder mehrfach substituiert durch
      5.1 Halogen, wie Fluor, Chlor, Brom oder Jod,
   6) (C₁-C₃)-Alkyl, ein- oder mehrfach substituiert durch
      6.1 Halogen, wie Fluor, Chlor, Brom oder Jod,
   7) Hydroxy,
   8) Alkylsulfonyl, mit 1 bis 3 C-Atomen in der Alkylkette,
   9) in der R⁴ für Wasserstoff und R⁵ und R⁶ gemeinsam einen Methylendioxyrest bilden,
   10) Cyano,
   11) (C₁-C₃)-Alkylmercapto,
   12) Benzoyl,
c) Pyrimidinyl ein- oder mehrfach substituiert durch 1) Alkyl mit 1 bis 3 C-Atomen,
d) Indolyl
e) Indazolinyl
steht.

Ein weiterer Gegenstand der Erfindung sind neue Verbindungen der Formel Ia oder der Formel Ib, 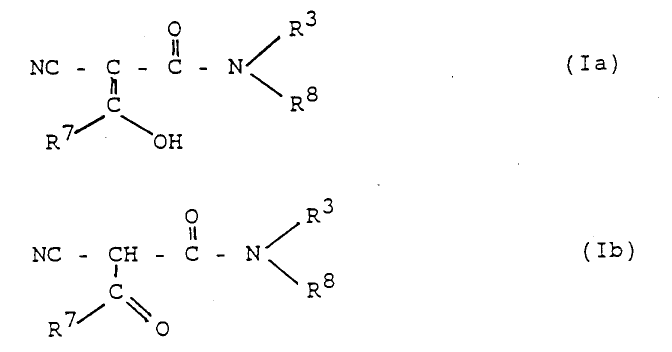 ihre gegebenenfalls stereoisomeren Formen und/oder gegebenenfalls ihre physiologisch verträglichen Salze, wobei die Reste R³, R⁷ und R⁸ unter
a) mit R⁷ für
   1) Wasserstoff,
   2) Alkyl mit 2 bis 4 C-Atomen
   mit R⁸ für
   1) Wasserstoff,
   2) Methyl
   mit R³ für
   1) Phenyl
   2) Phenyl, ein- oder mehrfach substituiert durch
      2.1 Halogen,
      2.2 Alkyl mit 1 bis 3 C-Atomen, ein- oder mehrfach substituiert durch Halogen, wie Fluor, Chlor, Brom oder Jod, steht
b) mit R⁷ für
   1) Alkyl mit 1 bis 4 C-Atomen,
   2) Wasserstoff,
   3) CF₃ ,
   mit R⁸ für
   1) Wasserstoff,
   2) Methyl,
   3) Alkenyl mit 2 bis 3 C-Atomen,
   mit R³ für
   1) Pyridyl,
   2) Pyridyl, ein- oder mehrfach substituiert durch
      2.1 Halogen, wie Fluor, Chlor, Brom oder Jod,
      2.2 Alkyl mit 1 bis 3 C-Atomen,
   3) Pyrimidinyl, substituiert wie bei 2)
   4) Thiazolyl, substituiert wie bei 2) und
      4.1 Alkoxycarbonyl, mit 1 bis 3 C-Atomen in der Alkylkette,
   5) Benzothiazolyl, substituiert wie bei 2),
   6) Benzimidazolyl, substituiert wie bei 2),
   7) Indazolyl, substituiert wie bei 2),
   8) Phenyl, ein- oder mehrfach substituiert durch
      8.1 Benzoyl,
      8.2 Benzoyl, ein- oder mehrfach substituiert durch
         8.2.1 Halogen, wie Fluor, Chlor, Brom oder Jod,
         8.2.2 Alkyl mit 1 bis 3 C-Atomen,
         8.2.3 Alkoxy mit 1 bis 3 C-Atomen,
         8.2.4. Alkoxy mit 1 bis 3 C-Atomen, ein- oder mehrfach substituiert durch Halogen wie Fluor, Chlor, Brom oder Jod, steht,
      8.3 Carboxy oder
      8.4 Hydroxy,
c) mit R⁷ für
   1) Wasserstoff,
   2) Alkyl mit 1 bis 4 C-Atomen,
   mit R⁸ für
   1) Wasserstoff,
   2) Methyl,
   mit R³ für
   1) einen Rest der Formel III,

      -(CH₂)₄-COOR¹⁰ ,

      in der R¹⁰ für
      1.1 Wasserstoff,
      1.2. Alkyl mit 1 bis 4 C-Atomen, steht,
d) mit R⁷ für
   1) Wasserstoff,
   2) Alkyl mit 1 bis 4 C-Atomen, steht,
      R⁸ und R³ zusammen mit dem Stickstoff, an das sie gebunden sind, einen 4- bis 9-gliedrigen Ring bilden, substituiert durch
      2.1 Carbonyl am N-Atom benachbarten C-Atom, oder
      R⁸ und R³ zusammen mit dem Stickstoff, an das sie gebunden sind, einen Piperidinring bilden gegebenenfalls substituiert durch Alkyl mit 1 bis 3 C-Atomen,
bedeuten.

Ein weiterer Gegenstand der Erfindung ist auch die Verwendung der neuen Verbindungen der Formel I, Ia oder Ib und/oder mindestens eines von deren physiologisch verträglichen Salzen zur Herstellung von Arzneimitteln zur Prophylaxe und/oder Behandlung von Rheumaerkrankungen.

Gegenstand der Erfindung sind auch Arzneimittel, die aus mindestens einer Verbindung der Formel I und/oder mindestens eines von deren physiologisch verträglichen Salzen bestehen oder mindestens einen dieser Wirkstoffe neben pharmazeutisch geeigneten und physiologisch verträglichen Trägerstoffen, Verdünnungsmitteln und/oder anderen Hilfsstoffen enthalten.

Die erfindungsgemäßen Arzneimittel können oral, topisch, rektal oder gegebenenfalls auch parenteral appliziert werden, wobei die orale Anwendung bevorzugt ist.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyäthylenglykole und Lösungsmittel, wie etwa steriles Wasser und ein oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis mindestens einer der Verbindung der Formel I und/oder mindestens einem von deren physiologisch verträglichen Salze enthält. Bei festen Dosierungseinheiten, wie Tabletten, Kapseln, Dragees oder Suppositorien, kann diese Dosis bis zu etwa 300 mg, bevorzugt jedoch etwa 10 bis 50 mg betragen.

Für die Behandlung eines an Leukämie erkrankten erwachsenen Patienten (70 kg) sind - je nach Wirksamkeit der Verbindungen der Formel I und/oder deren physiologisch verträglichen Salze am Menschen - Tagesdosen von etwa 5 bis 300 mg Wirkstoff, vorzugsweise etwa 25 bis 100 mg, bei oraler Verabreichung indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Verbindungen der Formel I und/oder mindestens eines von deren physiologisch verträglichen Salzen bei der Herstellung der vorgenannten galenischen Zubereitungsformen auch zusammen mit anderen geeigneten Wirkstoffen, beispielsweise anderen Antitumormitteln, Immunglobulinen, monoklonalen Antikörpern, immunstimulierenden Agenzien oder Antibiotika, formuliert werden. Diese Verbindungen können auch begleitend zu einer Strahlentherapie verabreicht werden.

Pharmakologische Prüfungen und Ergebnisse

Als Wirksamkeitstest von Chemotherapeutika wurde der in vitro Proliferationstest von Zellkulturen herangezogen.

### Beispiel 1

### Proliferations-Versuch

Clicks-/RPMI 1640 Medium (50:50) mit L-Glutamin ohne NaHCO₃ in Pulverform für 10 l (Seromed, Biochrom, Berlin, FRG), wird in 9 l Aqua bidest gelöst, und steril in Flaschen a 900 ml filtriert.

### Waschmedium

900 ml Grundmedium werden mit 9,5 ml 7,5%iger Natriumhydrogencarbonat-Lösung und 5 ml HEPES (N-2-Hydroxyethyl-Piperazin-N-2 Ethanolsulfonsäure) (Gibco, Eggenstein, FRG) abgepuffert.

### Gebrauchsmedium

900 ml Grundmedium plus 19 ml NaHCO₃-Lösung (7,5 %; 10 ml HEPES-Lösung und 10 ml L-Glutamin-Lösung (200 mM)).

Medium für die Mitogeninduzierte Lymphozytenproliferation Gebrauchsmedium wird mit 1 % hitzeinaktiviertem (30 min, 56°C) foetalem Kälberserum (FCS) angesetzt.

### Tumorzellmedium

Für die Haltung der Tumorzellen und Hybridomzellen wird Gebrauchsmedium mit 5 % FCS angesetzt.

### Kulturmedium für Zellinien

Für die Haltung der Zellinien werden 900 ml Gebrauchsmedium mit 10 % FCS, 10 ml NEA (non-essential amino acids)-Lösung (Gibco), 10 ml Natrium-Pyruvat-Lösung (100 mM, Gibco) und 5 ml 10⁻²M Mercaptoethanol gemischt.

### Gewinnung und Aufarbeitung der Milzzellen für die Mitogeninduzierte Lymphozytenproliferation

Die Mäuse werden durch Zervikaldislokation getötet und die Milzen steril entnommen. Auf einem sterilen Sieb mit einer Maschenweite von 80 "mesh" werden die Milzen zerschnitten und mit dem Stempel einer Plastikspritze (10 ml) vorsichtig in eine Petrischale mit Gebrauchsmedium passiert. Zur Entfernung der Erythrozyten aus der Milzzellsuspension wird das Gemisch etwa 1 min, unter gelegentlichem Aufschütteln in hypotonischer, 0,17 M Ammoniumchloridlösung bei Raumtemperatur inkubiert. Die Erythrozyten werden dabei lysiert, während die Vitalität und Reaktivität der Lymphozyten nicht beeinflußt wird. Nach Zentrifugation (7 min/ 340 g) wird das Lysat verworfen, die Zellen zweimal gewaschen und dann im jeweiligen Testmedium aufgenommen.

### Mitogeninduzierte Lymphozytenproliferation

5x10⁵ aufgearbeitete Milzzellen aus weiblichen NMRI-Mäusen wurden zusammen mit verschiedenen Mitogenen und Präparat in 200 µl Testmedium pro Vertiefung in Flachboden-Mikrotiterplatten pipettiert. Folgende Mitogen- und Präparatkonzentrationen wurden verwendet:

Concanavalin A [Serva]: 0,5 - 0,25 - 0,12 µg/ml Lipopolysaccharid [Calbiochem]: 1,0 - 0,5 - 0,1 µg/ml Phytohämagglutinin [Gibco]: 0,5 - 0,25 - 0,12 % Stammlösung Pokeweed mitogen [Gibco] Verbindung 1 oder 2: 50, 25, 10, 7,5, 5, 2,5, 1, 0,5, 0,1 µMol

Als Positivkontrollen wurden die Gruppe mit Mitogenzusätzen, ohne Präparat definiert. Bei den Negativkontrollen handelte es sich um Zellen in Kulturmedium mit Präparat ohne Mitogenzusätze. Jede Mitogenkonzentration wurde mit allen Präparatkonzentrationen vierfach getestet.
Nach 48 h Inkubation bei 37°C/5 % CO₂ wird den Zellen 25 µl/Vertiefung Tritium-Thymidin (Amersham) mit einer Aktivität von 0,25 µCi/Vertiefung (9,25x10³ Bq) hinzugefügt. Es schließt sich eine weitere Inkubation, unter den gleichen Bedingungen, für einen Zeitraum von 16 h, an. Zur Auswertung des Testansatzes werden die Zellen über ein Zellerntegerät (Flow Laboratories) auf Filterpapier geerntet, wobei nicht eingebautes Thymidin in einer gesonderten Abfallflasche gesammelt wird. Das Filterpapier wird getrocknet, ausgestanzt und zusammen mit 2 ml Szintillator (Rotiszint 22, Firma Roth) in Vials gegeben, die dann noch 2 h bei 4°C gekühlt werden. Die Menge, der von den Zellen eingebauten Radioaktivität wird in einem Beta-Counter (Firma Packard, Tricarb-460c) gemessen.

### Aufbereitung der Tumorzellen und Zellinien für den Proliferations-Test

Die im Test verwendeten Tumorzellen oder Zellinien werden in der logarithmischen Wachstumsphase der Stammhaltung entnommen, zweimal mit Waschmedium gewaschen und im entsprechenden Medium suspendiert.

### Durchführung und Auswertung der Proliferations-Tests

Der Proliferations-Test wurde in Rundboden-Mikrotiterplatten durchgeführt. Verbindung 1 und Interleukine wurden in je 50 µl/Vertiefung des entsprechenden Mediums gelöst und die Zellzahl (5x10⁵) wurde mit 100 µl/Vertiefung eingestellt, so daß sich ein Endvolumen von 200 µl/Vertiefung ergibt. In allen Tests wurden die Werte vierfach bestimmt. Zellen ohne Präparat und ohne Wachstumsfaktor wurden als Negativkontrolle definiert und Zellen ohne Präparat mit Wachstumsfaktor ergaben die Werte für die Positivkontrolle. Der Wert der Negativkontrolle wurde von allen ermittelten Werten abgezogen und die Differenz aus Positivkontrolle minus Negativkontrolle wurde 100 % gesetzt.
Die Platten wurden 72 h bei 37°C/5 % CO₂ inkubiert und die Proliferationsrate wurde entsprechend wie bei der Mitogeninduzierten Lymphozytenproliferation bestimmt.

Die Zellinien wurden von der Stammsammlung, American Type Culture Collection (ATCC), entnommen.

Die Tabelle 1 zeigt die Konzentrationen bei denen eine 50 %ige Hemmung auftritt:

**Tabelle 1**

| Zellinie | Ursprung | ED₅₀ |
|---|---|---|
| CTLL | Maus T Zellinie (T_{c}-Clon IL-2) | 40-50 µM |
| HT-2 | Maus T Zellinie (IL-2) | 40-50 µM |
| CTL-J-K | Maus T Zellinie (T_{c}, IL-2) | 40-50 µM |
| Cl 9/4 | Maus T Zellinie (IL-4 dep.) | 25 µM |
| K III 5 | Maus T Zellinie (Tₕ, IL-2) | 1-3 µM |
| Spleen T | Maus (Con A und PWM) | 10 µM |
| Spleen B | Maus (LPS) | 10 µM |
| A20 2J | Maus B Zelltumor (BALB/c) | 1-3 µM |
| TRK 4 | Maus B Zellhybridoma | 5 µM |
| TRK 5 | Maus B Zellhybridoma | 5 µM |
| Bone Marrow | Maus (M-CSF, GM CSF) | 5 µM |
| WEHI 279 | Maus B Zelllymphoma | ≤ 1 µM |
| P 388 D1 | Maus MØ Tumor | 10 µM |
| 7TD1 | Maus B Zellhybridoma (IL-6) | 10 µM |
| G53 | Maus T Zellklon | |
| PB-3C | Maus Mast Zellinie (IL-3) | 20 µM |
| DA-1 | Maus Tumor (IL-3) | 5 µM |
| 7D4 | Ratten hybridoma | ≤ 1 µM |
| A431 | Human epidermoid Carcinoma | 15 µM |
| KB | Human epidermoid Carcinoma | 15 µM |
| HFF | Human Foreskin Fibroblast | 40 µM |
| HL-60 | Human Promyelomonocytic Leukämia | 25 µM |

### Beispiel 2

### Akute Toxizität bei oraler Verabreichung

Verbindung 1 wurde zur Bestimmung der akuten Toxizität den Mäusen oder Ratten oral verabreicht.
Die LD₅₀-Werte wurden nach Litchfield und Wilcoxon bestimmt.

Das Gewicht der NMRI-Mäuse (NMRI : Naval Medical Research Institute) beträgt 20 bis 25 g und das der SD-Ratten (SD: Sprague-Dawley) beträgt 120 bis 165 g. Vor dem Versuch hungerten die Mäuse für etwa 18 Stunden. 5 Stunden nach der Verabreichung der getesteten Substanzen wird wieder normal gefüttert. Nach 3 Wochen wurden die Tiere durch Chloroform getötet und seziert. Pro Dosierung wurden 6 Tiere verwendet. Die Ergebnisse sind in Tabelle 2 zusammengefaßt.

**Tabelle 2**

| | Verbindung 1 akute Toxizität per OS LD₅₀ (mg/kg) | | Verbindung 2 akute Toxizität per OS LD₅₀ (mg/kg) |
|---|---|---|---|
| NMRI-Maus | 445 (362 - 546) | SD-Ratte | 160 (133 - 193) |
| SD-Ratte | 235 (167 - 332) | | |

### Beispiel 3

### Akute Toxizität nach intraperitonealer Verabreichung

Die akute Toxizität nach intraperitonealer Verabreichung der Testsubstanzen wurde mit NMRI-Mäusen (20 bis 25 g) und SD-Ratten (120 bis 195 g) durchgeführt. Die Testsubstanz wurde in einer 1 %igen Natrium-Carboxymethylcellulose-Lösung suspendiert. Die verschiedenen Dosierungen der Testsubstanz wurden den Mäusen in einem Volumen von 10 ml/kg Körpergewicht und den Ratten in einem Volumen von 5 ml/kg Körpergewicht verabreicht. Pro Dosierung wurden 10 Tiere verwendet. Nach 3 Wochen wurde die akute Toxizität nach der Methode von Litchfield und Wilcoxon bestimmt. Die Ergebnisse sind in Tabelle 3 zusammengefaßt.

**Tabelle 3**

| | Verbindung 1 akute Toxizität intraperitoneal LD₅₀ (mg/kg) | | Verbindung 2 akute Toxizität intraperitoneal LD₅₀ (mg/kg) |
|---|---|---|---|
| NMRI-Maus | 185 (163 - 210) | NMRI-Maus | (100 - 200) |
| SD-Ratte | 170 (153 - 189) | | |

### Beispiel 4

### A Herstellung von 5-Methylisoxazol-4-carbonsäure-(4-trifluormethyl)-anilid

Eine Lösung von 0,05 Mol 5-Methylisoxazol-4-carbonsäurechlorid (7,3 g) in 20 ml Acetonitril werden tropfenweise bei Raumtemperatur in eine Lösung von 0,1 Mol 4-Trifluormethylanilin (16,1 g) in 150 ml Acetonitril gegeben. Nach 20 Minuten Rühren wird das ausgefallene 4-Trifluormethylanilin-hydrochlorid abgesaugt, zweimal mit je 20 ml Acetonitril gewaschen und die vereinigten Filtrate unter vermindertem Druck eingeengt. Man erhält so 12,8 g weißes, kristallines 5-Methylisoxazol-4-carbonsäure-(4-trifluormethyl)-anilid (Verbindung 1).

### B Herstellung von N-(4-Trifluormethylphenyl)-2-cyano-3-hydroxycrotonsäureamid

Es werden 0,1 Mol 5-Methylisoxazol-4-carbonsäure-(4-trifluormethyl)-anilid in 100 ml Methanol gelöst und bei +10°C mit einer Lösung von 0,11 Mol (4,4 g) Natronlauge in 100 ml Wasser versetzt. Es wird 30 Minuten gerührt und nach Verdünnen mit Wasser mit konzentrierter Salzsäure angesäuert. Der ausgefallene Kristallbrei wird abgesaugt, mit Wasser gewaschen und an der Luft getrocknet. Die Ausbeute beträgt 24,4 g N-(4-Trifluormethylphenyl)-2-cyano-3-hydroxycrotonsäureamid (Verbindung 2). Schmelzpunkt aus Methanol 205 bis 206°C.

### Herstellungsbeispiele

Die Struktur aller nachstehend beschriebenen Verbindungen wurde durch Elementaranalyse und IR- sowie ¹H-NMR-Spektren gesichert.
5. N-(4-Chlordifluormethoxy)phenyl-5-ethylisoxazol-4-carboxamid
   0,1 Mol (19,4 g) 4-Chlordifluormethoxyanilin, gelöst in 180 ml Acetonitril, werden bei Raumtemperatur tropfenweise mit einer Lösung von 0,05 Mol (7,3 g) 5-Methylisoxazol-4-carbonsäurechlorid in 30 ml Acetonitril unter Rühren versetzt. Man rührt weitere 20 Minuten und filtriert die Flüssigkeit vom ausgefallenen Salz ab. Das Filtrat wird unter vermindertem Druck zur Trockne gebracht. Man erhält so 28,5 g (94,2 % d. Th.) kristallines Produkt.
   Schmelzpunkt [aus Cyclohexan-Aceton 20 : 1 (v/v)]: 112° -113°C.
   Analog dem vorstehend beschriebenen Beispiel werden die folgenden Verbindungen der Formel I dargestellt.
6. N-(4-Fluorphenyl)-isoxazol-4-carboxamid (vom Schmelzpunkt 162° bis 164° C) aus Isoxazol-4-carbonsäurechlorid und 4-Fluoranilin.
7. N-(4-Chlorphenyl)-isoxazol-4-carboxamid [vom Schmelzpunkt 175° bis 177° C (Zers.)] aus Isoxazol-4-carbonsäurechlorid und 4-Chloranilin.
8. N-(4-Bromphenyl)-isoxazol-4-carboxamid [vom Schmelzpunkt 184° bis 186° C (Zers.)] aus Isoxazol-4-carbonsäurechlorid und 4-Bromanilin.
9. N-(4-Jodphenyl)-isoxazol-4-carboxamid (vom Schmelzpunkt 207° bis 208° C) aus Isoxazol-4-carbonsäurechlorid und 4-Jodanilin.
10. N-(4-Nitrophenyl)-isoxazol-4-carboxamid [vom Schmelzpunkt 208° bis 210° C (Zers.)] aus Isoxazol-4-carbonsäurechlorid und 4-Nitroanilin.
11. N-(3,4-Methylendioxiphenyl)-isoxazol-4-carboxamid (vom Schmelzpunkt 168° bis 169° C) aus Isoxazol-4-carbonsäurechlorid und 3,4-Methylendioxianilin.
12. N-(4-Benzoylphenyl)-isoxazol-4-carboxamid [vom Schmelzpunkt 197° bis 199° C (Zers.)] aus Isoxazol-4-carbonsäurechlorid und 4-Aminobenzophenon.
13. N-(4-Fluorphenyl)-5-ethylisoxazol-4-carboxamid (vom Schmelzpunkt 75° bis 77° C) aus 5-Ethylisoxazol-4-carbonsäurechlorid und 4-Fluoranilin.
14. N-(4-Chlorphenyl)-5-ethylisoxazol-4-carboxamid (vom Schmelzpunkt 103° bis 105° C) aus 5-Ethylisoxazol-4-carbonsäurechlorid und 4-Chloranilin.
15. N-(4-Bromphenyl)-5-ethylisoxazol-4-carboxamid (vom Schmelzpunkt 117° bis 118° C) aus 5-Ethylisoxazol-4-carbonsäurechlorid und 4-Bromanilin.
16. N-(4-Nitrophenyl)-5-ethylisoxazol-4-carboxamid (vom Schmelzpunkt 139° bis 141° C) aus 5-Ethylisoxazol-4-carbonsäurechlorid und 4-Nitroanilin.
17. N-(3,4-Methylendioxiphenyl)-5-ethylisoxazol-4-carboxamid (vom Schmelzpunkt 105° bis 106° C) aus 5-Ethylisoxazol-4-carbonsäurechlorid und 3,4-Methylendioxianilin.
18. N-(4-Trifluormethoxiphenyl)-5-ethylisoxazol-4-carboxamid (vom Schmelzpunkt 52° bis 54° C) aus 5-Ethylisoxazol-4-carboxamid und 4-Trifluormethoxianilin.
19. N-(4-Benzoylphenyl)-5-ethylisoxazol-4-carboxamid (vom Schmelzpunkt 168° bis 170° C) aus 5-Ethylisoxazol-4-carbonsäurechlorid und 4-Aminobenzophenon.
20. N-[4-(4-Fluorbenzoyl)phenyl]-5-ethylisoxazol-4-carboxamid (vom Schmelzpunkt 153° bis 155° C) aus 5-Ethylisoxazol-4-carbonsäurechlorid und 4-(4-Fluorbenzoyl)anilin.
21. N-[4-(4Chlorbenzoyl)phenyl]-5-ethylisoxazol-4-carboxamid (vom Schmelzpunkt 159° bis 161° C) aus 5-Ethylisoxazol-4-carbonsäurechlorid und 4-(4-Chlorbenzoyl)anilin.
22. N-[4-(4-Brombenzoyl)phenyl]-5-ethylisoxazol-4-carboxamid (vom Schmelzpunkt 178° bis 181° C) aus 5-Ethylisoxazol-4-carbonsäurechlorid und 4-(4-Brombenzoyl)anilin.
23. N-(4-Benzoylphenyl]-5-propylisoxazol-4-carboxamid (vom Schmelzpunkt 134° bis 135° C) aus 5-Propylisoxazol-4-carbonsäurechlorid und 4-Aminobenzophenon.
24. N-(4-Chlorphenyl)-5-butylisoxazol-4-carboxamid (vom Schmelzpunkt 91° bis 92° C) aus 5-Butylisoxazol-4-carbonsäurechlorid und 4-Chloranilin.
25. N-(4-Benzoylphenyl-5-butylisoxazol-4-carboxamid (vom Schmelzpunkt 108° bis 110° C) aus 5-Butylisoxazol-4-carbonsäurechlorid und 4-Aminobenzophenon.
26. N-(4-Fluorphenyl)-5-trifluormethylisoxazol-4-carboxamid (vom Schmelzpunkt 97° C) aus 5-Trifluormethylisoxazol-4-carbonsäurechlorid und 4-Fluoranilin.
27. N-(4-Chlorphenyl)-5-trifluormethylisoxazol-4-carboxamid (vom Schmelzpunkt 90° bis 92° C) aus 5-Trifluormethylisoxazol-4-carbonsäurechlorid und 4-Chloranilin.
28. N-(4-Nitrophenyl)-5-trifluormethylisoxazol-4-carboxamid (vom Schmelzpunkt 136° bis 138° C) aus 5-Trifluormethylisoxazol-4-carbonsäurechlorid und 4-Nitroanilin.
29. N-(3,4-Methylendioxiphenyl)-5-trifluormethylisoxazol-4-carboxamid (vom Schmelzpunkt 114° bis 116° C) aus 5-Trifluormethylisoxazol-4-carbonsäurechlorid und 3,4-Methylendioxianilin.
30. N-(4-Trifluormethylphenyl)-5-chlormethylisoxazol-4-carboxamid (vom Schmelzpunkt 136° bis 137° C) aus 5-Chlormethylisoxazol4-carbonsäurechlorid und 4-Trifluormethylanilin.
31. N-(4-Trifluormethylphenyl)-5-phenylisoxazol-4-carboxamid (vom Schmelzpunkt 159° bis 160° C) aus 5-Phenylisoxazol4-carbonsäurechlorid und 4-Trifluormethylanilin.
32. N-(4-Fluorphenyl)-5-phenylisoxazol-4-carboxamid (vom Schmelzpunkt 151° bis 153° C) aus 5-Phenylisoxazol-4-carbonsäurechlorid und 4-Fluoranilin.
33. N-(4-Methylsulfonylphenyl)-5-methylisoxazol-4-carboxamid (vom Schmelzpunkt 170° bis 172° C) aus 5-Methylisoxazol-4-carbonsäurechlorid und 4-Methylsulfonylanilin.
34. N-Benzyl-N-(4-trifluormethylphenyl)-5-methylisoxazol-4-carboxamid (vom Schmelzpunkt 87° bis, 89° C) aus 5-Methylisoxazol-4-carbonsäurechlorid und N-Benzyl-4-trifluormethylanilin.
36. N-(5-Chlor-2-pyridyl)-isoxazol-4-carboxamid (vom Schmelzpunkt 254° bis 255° C) aus Isoxazol-4-carbonsäurechlorid und 2-Amino-5-chlorpyridin.
37. N-(5-Chlor-2-pyridyl)-5-ethylisoxazol-4-carboxamid (vom Schmelzpunkt 133° bis 136° C) aus 5-Ethylisoxazol4-carbonsäurechlorid und 2-Amino-5-chlorpyridin.
38. N-(5-Brom-2-pyridyl)-5-ethylisoxazol-4-carboxamid (vom Schmelzpunkt 144° bis 145° C) aus 5-Ethylisoxazol-4-carbonsäurechlorid und 2-Amino-5-brompyridin.
39. N-(5-Nitro-2-pyridyl)-5-ethylisoxazol-4-carboxamid (vom Schmelzpunkt 236° bis 237° C) aus 5-Ethylisoxazol-4-carbonsäurechlorid und 2-Amino-5-nitropyridin.
40. N-(5-Chlor-2-pyridyl)-5-phenylisoxazol-4-carboxamid (vom Schmelzpunkt 160° bis 161° C) aus 5-Phenylisoxazol-4-carbonsäurechlorid und 2-Amino-5-chlorpyridin.
41. N-(5-Indolyl)-5-methylisoxazol-4-carboxamid (vom Schmelzpunkt 155° bis 157° C) aus 5-Methylisoxazol-4-carbonsäurechlorid und 5-Aminoindol, .
42. N-(6-Indazolyl)-5-methylisoxazol-4-carboxamid (vom Schmelzpunkt 198° bis 202° C) aus 5-Methylisoxazol-4-carbonsäurechlorid und 6-Aminoindazol.
43. N-(5-Indazolyl)-5-methylisoxazol-4-carboxamid (vom Schmelzpunkt 218° bis 220° C) aus 5-Methylisoxazol-4-carbonsäurechlorid und 5-Aminoindazol.
44. N-Allyl-N-phenyl-5-methylisoxazol-4-carboxamid (vom Schmelzpunkt 79° bis 85° C) aus 5-Methylisoxazol-4-carbonsäurechlorid und N-Allylanilin.
45. N-[3-(1,1,2,2-Tetrafluorethoxi)phenyl]-5-methylisoxazol-4-carboxamid (vom Schmelzpunkt 97° C) aus 5-Methylisoxazol-4-carbonsäurechlorid und 3-(1,1,2,2-Tetrafluorethoxi)anilin.
46. N-(4-Cyanophenyl)-5-methylisoxazol-4-carboxamid (vom Schmelzpunkt 197° bis 200° C) aus 5-Methylisoxazol-4-carbonsäurechlorid und 4-Cyanoanilan.
47. N-(4-Methylmercaptophenyl)-5-methylisoxazol-4-carboxamid (vom Schmelzpunkt 134° bis 136° C) aus 5-Methylisoxazol-4-carbonsäurechlorid und 4-Methylmercaptoanilin.
48. N-(4,6-Dimethyl-2-pyridyl)-5-methylisoxazol-4-carboxamid (vom Schmelzpunkt 210° C) aus 5-Methylisoxazol-4-carbonsäurechlorid und 2-Amino-4,6-dimethylpyridin.
49. N-(4,6-Dimethyl-2-pyrazinyl)-5-methylisoxazol-4-carboxamid (vom Schmelzpunkt 222° bis 226° C) aus 5-Methylisoxazol-4-carbonsäurechlorid und 2-Amino-4,6-dimethylpyrazin.
50. N-(4-Trifluormethoxyphenyl)-2-cyano-3-hydroxycrotonsäureamid
   Es wurden 0,1 Mol (28,6 g) N-(4-Trifluormethoxyphenyl)-5-methylisoxazol-4-carboxamid in 100 ml Ethanol gelöst und bei 20° C mit einer Lösung von 0,11 Mol(4,4 g) Natronlauge in 100 ml Wasser versetzt. Es wie 30 Minutan gerührt und nach Verdünnung mit Wasser mit konzentrierter Salzsäure angesäuert. Der ausgefallene Kristallbrei wird abgesaugt, mit Wasser gewaschen und an der Luft getrocknet. Man erhält so 27,7 g (97,1 % der Theorie) N-(4-Trifluormethoxyphenyl)-2-cyano-3-hydroxycrotonsäureamid vom Schmelzpunkt 171° -176° C (aus Ethanol).
   Analog dem vorstehend beschriebenen Beispiel werden die folgenden Verbindungen der Formel Ia oder Ib dargestellt.
51. 2-Cyano-3-hydroxy-N-[4-(1,1,2,2-tetrafluorethoxy)phenyl] crotonsäureamid (vom Schmelzpunkt 166°-164° C) aus N-[4-(1,1,2,2,-Tetrafluorethoxy)phenyl]-5-methylisoxazol-4-carboxamid.
52. N-(5-Chlor-2-pyridyl)-2-cyano-3-hydroxycrotonsäureamid [vom Schmelzpunkt 213° bis 215° C (Zers.)] aus N-(5-Chlor-2-pyridyl)-5-methylisoxazol-4-carboxamid.
53. N-(2-Chlor-3-pyridyl)-2-cyano-3-hydroxycrotonsäureamid (vom Schmelzpunkt 128° bis 131° C) aus N-(2-Chlor-3-pyridyl)-5-methylicoxazol-4-carboxamid.
54. N-(4-Benzoylphenyl)-2-cyano-3-hydroxycrotonsäureamid (vom Schmelzpunkt 186° bis 188° C) aus N-(4-Benzoylphenyl)-5-methyl-isoxazol-4-carboxamid.
55. N-[4-(4-Chlorbenzoyl)phenyl]-2-cyano-3-hydroxy-4-methylcrotonsäureamid (vom Schmelzpunkt 157° bis 159° C) aus N-[4-(4-Chlorbenzoyl)phenyl]-5-ethylisoxazol-4-carboxamid.
56. N-[4-(4-Brombenzoyl)phenyl]-2-cyano-3-hydroxycrotonsäureamid (vom Schmelzpunkt 221° bis 223°C) aus N-[4-(4-Brombenzoyl) phenyl]-5-methylisoxazol-4-carboxamid.
57. N-[4-(4-Methoxybenzoyl)phenyl]-2-cyano-3-hydroxycrotonsäureamid (vom Schmelzpunkt 74° bis 75° C) aus N-[4-(4-Methoxybenzoyl)phenyl]-5-methylisoxazol-4-carboxamid.
58. N-[4-(4-Methylbenzoyl)phenyl]-2-cyano-3-hydroxycrotonsäureamid (vom Schmelzpunkt 177° bis 179° C) aus N-[4-(4-Methylbenzoyl)phenyl]-5-methylisoxazol-4-carboxamid.
59. N-(5-Chlor-2-pyridyl)-2-cyano-3-hydroxy-4-methylcrotonsäure amid (vom Schmelzpunkt 206° bis 208° C) aus N-(5-Chlor-2-pyridyl)-5-ethylisoxazol-4-carboxamid.
60. N-(5-Brom-2-pyridyl)-2-cyano-3-hydroxy-4-methylcrotonsäure amid [vom Schmelzpunkt 200° bis 202° C (Zers.)] aus N-(5-Brom-2-pyridyl)-5-ethylisoxazol-4-carboxamid.
61. 2-Cyano-3-hydroxy-4-methyl-N-(4-nitrophenyl)crotonsäureamid [vom Schmelzpunkt 202° bis 203° C (Zers.)] aus N-(4-Nitrophenyl)-5-ethylisoxazol-4-carboxamid.
62. N-(3,4-Methylendioxiphenyl)-2-cyano-3-hydroxy-4-methylcrotonsäureamid (vom Schmelzpunkt 99° bis 100° C) aus N-(3,4-Methylendioxiphanyl)-5-ethylisoxazol-4-carboxamid,.
63. N-(4-Benzoylphenyl)-2-cyano-3-hydroxy-4-propylcrotonsäureamid aus N-(4-Benzoylphenyl)-5-butylisoxazol-4-carboxamid.
64. N-(5-Brom-2-pyridyl)-2-cyano-3-hydroxycrotonsäureamid [vom Schmelzpunkt 220° bis 223° C (Zers.)] aus N-(5-Brom-2-pyridyl)-5-methylisoxazol-4-carboxamid.
65. N-[4-(4-Chlorbenzoyl)phenyl]-2-cyano-3-hydroxycrotonsäureamid [vom Schmelzpunkt 219° bis 223° C(Zers.)] aus N-[4-(4-Chlorbenzoyl)phenyl]-5-methylisoxazol-4-carboxamid.
66. N-[4-(4-Fluorbenzoyl)phenyl]-2-cyano-3-hydroxycrotonsäureamid [vom Schmelzpunkt 229° bis 231° C(Zers.)] aus N-[4-(4-Fluorbenzoyl)phenyl]-5-methylisoxazol-4-carboxamid.
67. N-[4-(4-Fluorbenzoyl)phenyl]-2-cyano-4-methyl-3-hydroxycrotonsäureamid (vom Schmelzpunkt 147° bis 148° C) aus N-[4-(4-Fluorbenzoyl)phenyl]-5-ethylisoxazol-4-carboxamid.
68. N-[4-(4-Brombenzoyl)phenyl]-2-cyano-3-hydroxy-4-methylcrotonsäureamid (vom Schmelzpunkt 153° bis 155° C) aus N-[4-(4-Brombenzoyl)phenyl]-5-ethylisoxazol-4-carboxamid.
69. N-(4-Trifluormethoxy)phenyl-2-cyano-3-hydroxy-4-methylcrotonsäureamid (vom Schmelzpunkt 166° bis 167° C) aus N-(4-Trifluormethoxy)phenyl-5-ethylisoxazol-4-carboxamid.
70. N-(4-Fluorphenyl)-2-cyano-3-hydroxy-4-methylcrotonsäureamid (vom Schmelzpunkt 145° C) aus N-(4-Fluorphenyl)-5-ethylisoxazol-4-carboxamid.
71. N-(3,4-Methylendioxyphenyl)-2-cyano-3-hydroxy-4-methylcrotonsäureamid (vom Schmelzpunkt 99° bis 100° C) aus N-(3,4-Methylendioxyphenyl)-5-ethylisoxazol-4-carboxamid.
72. N-(4-Methylsulfonyl)phenyl-2-cyano-3-hydroxycrotonsäureamid (vom Schmelzpunkt 196° bis 198° C) aus N-(4-Methylsulfonyl) phenyl-5-methylisoxazol-4-carboxamid.
73. N-Allyl-N-phenyl-2-cyano-3-hydroxycrotonsäureamide (vom Schmelzpunkt 57° bis 50° C) aus N-Allyl-N-phenyl-5-methylisoxazol-4-carboxamid.
74. N-(4-Ethoxycarbonylmethyl-2-thiazolyl)-2-cyano-3-hydroxycrotonsäureamid (vom Schmelzpunkt 147° C) aus N-(4-Ethoxy= carbonylmethyl-2-thiazolyl)-5-methylisoxazol-4-carboxamid.
75. N-(2-Benzimidazolyl)-2-cyano-3-hydroxycrotonsäureamid (vom Zersetzungspunkt > 300° C) aus N-(2-Benzimidazolyl)-5-methylisoxazol-4-carboxamid.
76. N-(4-Methyl-2-thiazolyl)-2-cyano-3-hydroxycrotonsäureamid [vom Schmelzpunkt 210° bis 212 ° C (Zers.)] aus N-(4-Methyl-2-thiazolyl)-5-methylisoxazol-4-carboxamid.
77. N-(4-Chlor-2-benzothiazolyl)-2-cyano-3-hydroxycrotonsäureamid [vom Schmelzpunkt 211 ° bis 213° C (Zers.)] aus N-(4-Chlor-2-benzothiazolyl)-5-methylisoxazol-4-carboxamid.
78. N-(3-Pyridyl)-2-cyano-3-hydroxycrotonsäureamid [von Schmelzpunkt 240° bis 250° C (Zers.)] aus N-(3-Pyridyl)-5-methylisoxazol-4-carboxamid.
79. N-(4,6-Dimethyl-2-pyridyl)-2-cyano-3-hydroxycrotonsäureamid (vom Schmelzpunkt 184° bis 186° C) aus N-(4,6-Dimethyl-2-pyridyl)-5-methylisoxazol-4-carboxamid
80. N-(4,6-Dimethyl-2-pyrimidyl)-2-cyano-3-hydroxycrotonsäureamid (von Schmelzpunkt 221° ) aus N-(4,6-Dimethyl-2-pyrimidyl)-5-methylisoxazol-4-carboxamid.
81. N-(6-Indazolyl)-2-cyano-3-hydroxycrotonsäureamid (vom Schmelzpunkt > 300° C) aus N-(6-Indazolyl)-5-methylisoxazol-4-carboxamid.
82. N-(5-Indazolyl)-2-cyano-3-hydroxycrotonsäureamid (vom Schmelzpunkt 220° bis 223° C) aus N-(5-Indazolyl)-5-methylisoxazol-4-carboxamid.
83. N-(4-Carboxy-3-hydroxypenyl)-2-cyano-3-hydroxycrotonsäureamid [vom Schmelzpunkt 242° bis 246° C (Zers.)] aus N-(4-Carboxy-3-hydroxyphenyl)-5-methylisoxazol-4-carboxamid
84. N-(3-Carboxy-4-hydroxyphenyl)-2-cyano-3-hydroxycrotonsäureamid [vom Schmelzpunkt 248° bis 252° C (Zers.)] aus N-(3-Carboxy-4-hydroxyphenyl)-5-methylisoxazol-4-carboxamid
85. N-(4-Carboxy-3-chlorphenyl)-2-cyano-3-hydroxycrotonsäureamid [vom Schmelzpunkt 218° bis 224° C(Zers.)] aus N-(4-Carboxy-3-chlorphenyl)-5-methylisoxazol-4-carboxamid.
86. N-(4-Hydroxyphenyl)-2-cyano-3-hydroxycrotonsäureamid [vom Zersetzungspunkt 184° bis 186° C (Zers.)] aus N-(4-Hydroxyphenyl)-5-methylisoxazol-4-carboxamid.
87. N-[4-(4-Trifluormethylphenoxy)phenyl]-2-cyano-3-hydroxy-4-methylcrotonsäureamid (vom Schmelzpunkt 147° bis 149° C) aus N-[4-(4-Trifluormethylphenoxy)phenyl]-5-ethylisoxazol-4-carboxamid.
88. N-[4-(4-Trifluormethylphenoxy)phenyl]-2-cyano-3-hydroxycrotonsäureamid (vom Schmelzpunkt 171° bis 173° C) aus N-[4-(4-Trifluormethylphenoxy)phenyl]-5-methylisoxazol-4-carboxamid.
89. N-Methyl-N-(4-trifluormethylphenyl)-2-cyano-3-hydroxycrotonsäureamid (vom Schmelzpunkt 69° bis 70°C) aus N-Methyl-N-(4-trifluormethylphenyl)-5-methylisoxazol-4-carboxamid.
90. 2-Hydroxyethylidencyanessigsäurepiperidid aus N-(5-Methyl-4-isoxazolylcarbonyl)piperidin.
91. 2-Hydroxyethylidencyanessigsäure-4-methylpiperidid aus N-(5-Methyl-4-isoxazolyl-carbonyl)-4-hydroxypiperidin.
92. N-(4-Carboxybutyl)-2-cyano-3-hydroxycrotonsäureamid (vom Schmelzpunkt 92° C) aus N-(5-Methyl-4-isoxazolylcarbonyl)-5-aminovaleriansäure.
93. N-(4-Ethoxycarbonylbutyl)-2-cyano-3-hydroxycrotonsäureamid aus N-(4-Ethoxycarbonylbutyl)-5-methylisoxazol-4-carboxamid.
94. N-(6-Carboxyhexyl)-2-cyano-3-hydroxycrotonsäureamid (vom Schmelzpunkt 93° bis 94° C) aus N-(5-Methyl-4-isoxazolyl carbonyl)-7-aminoheptansäure.

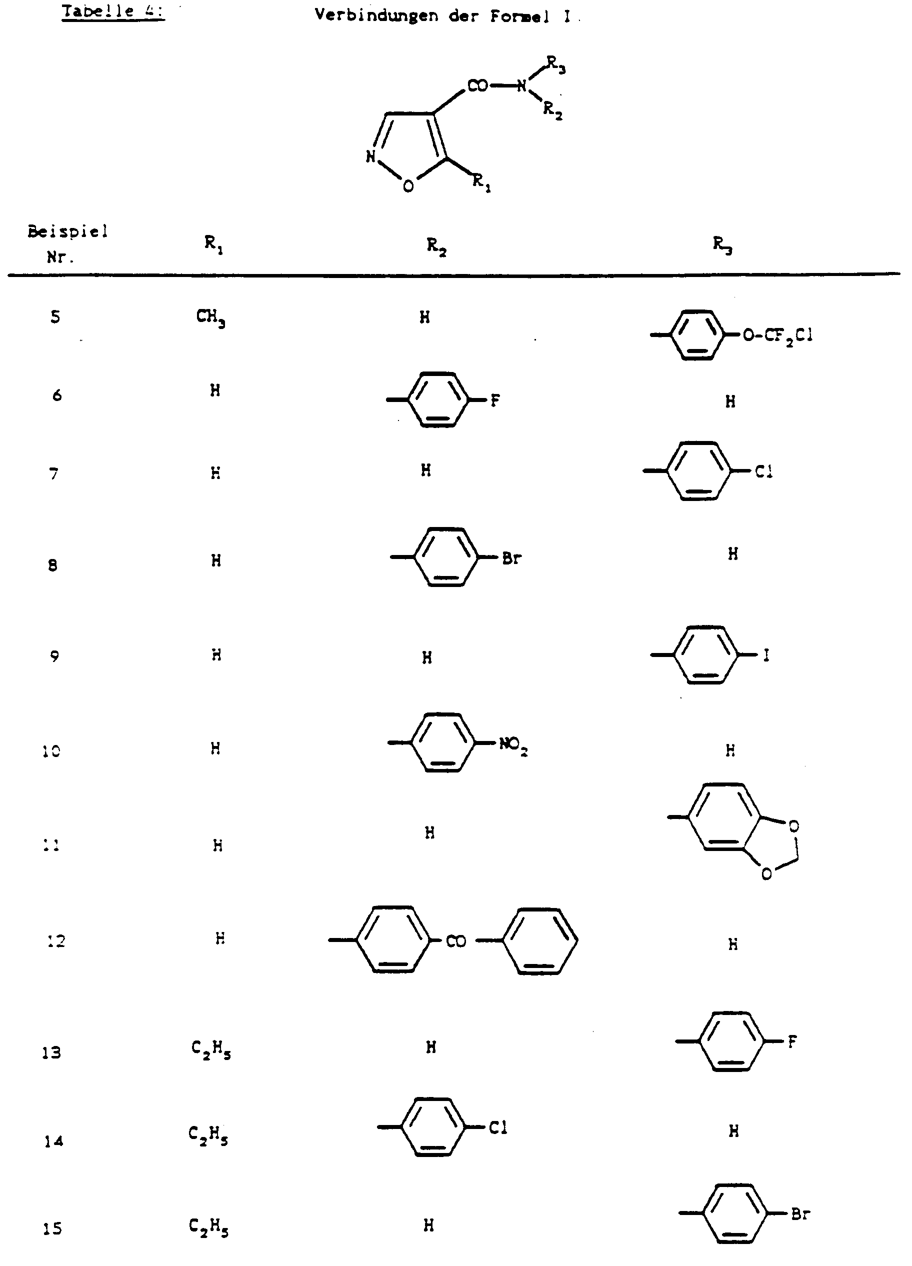

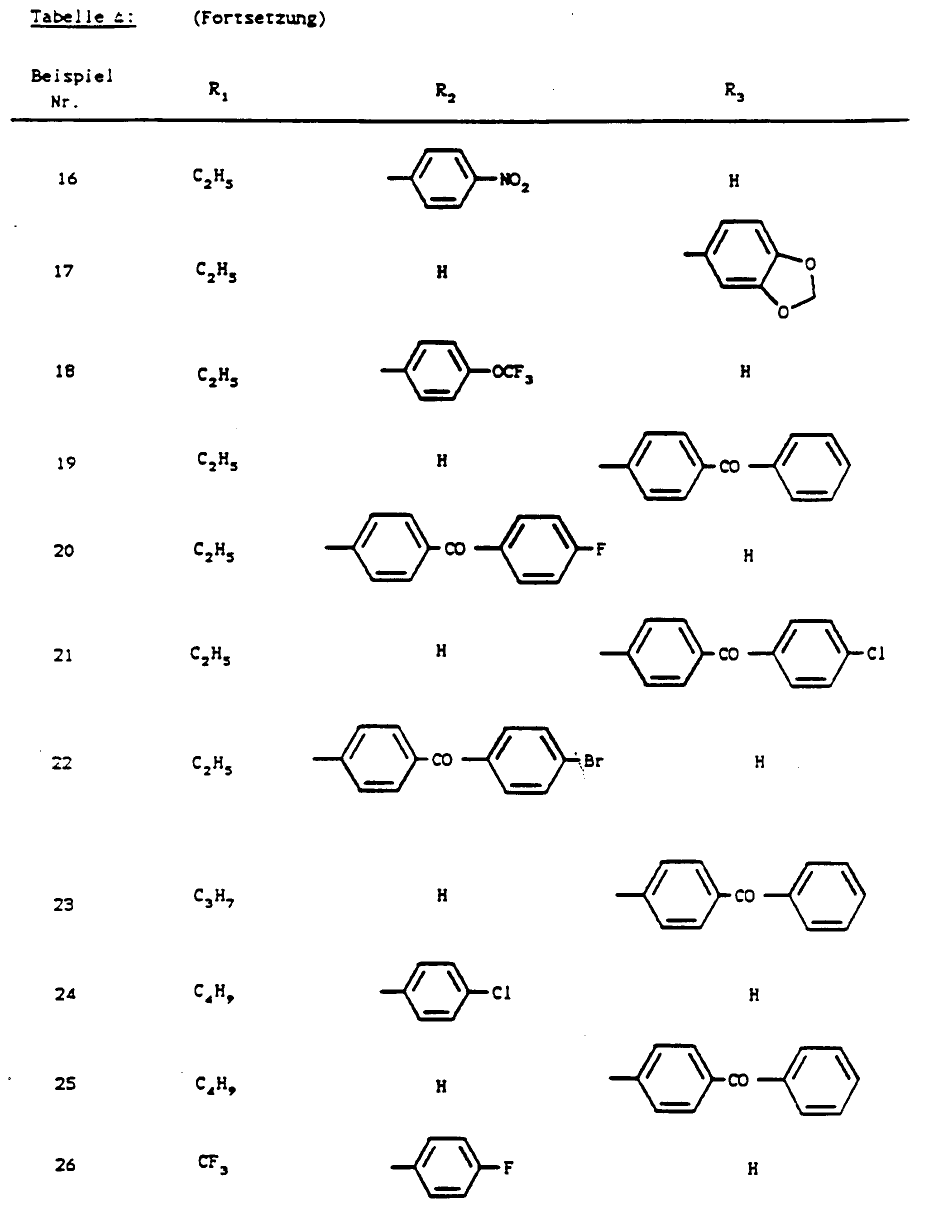

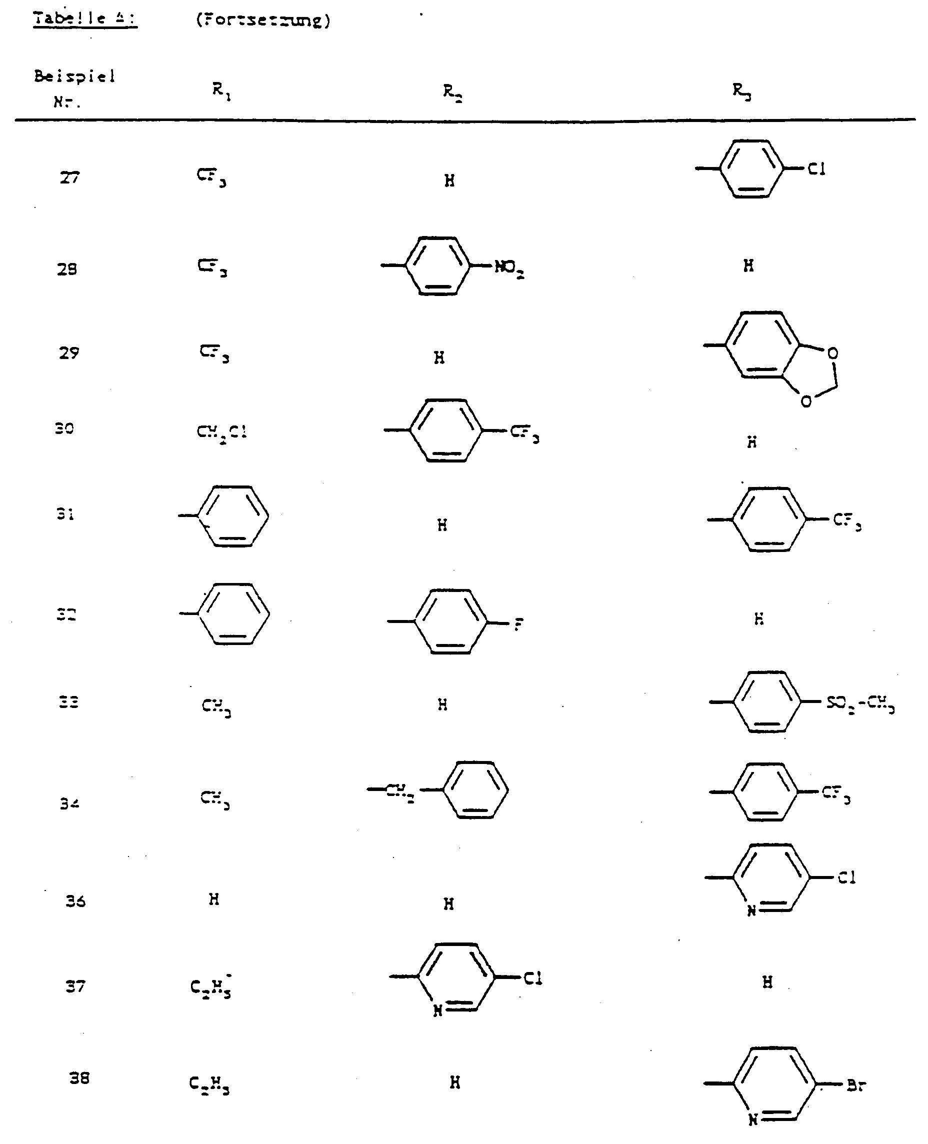

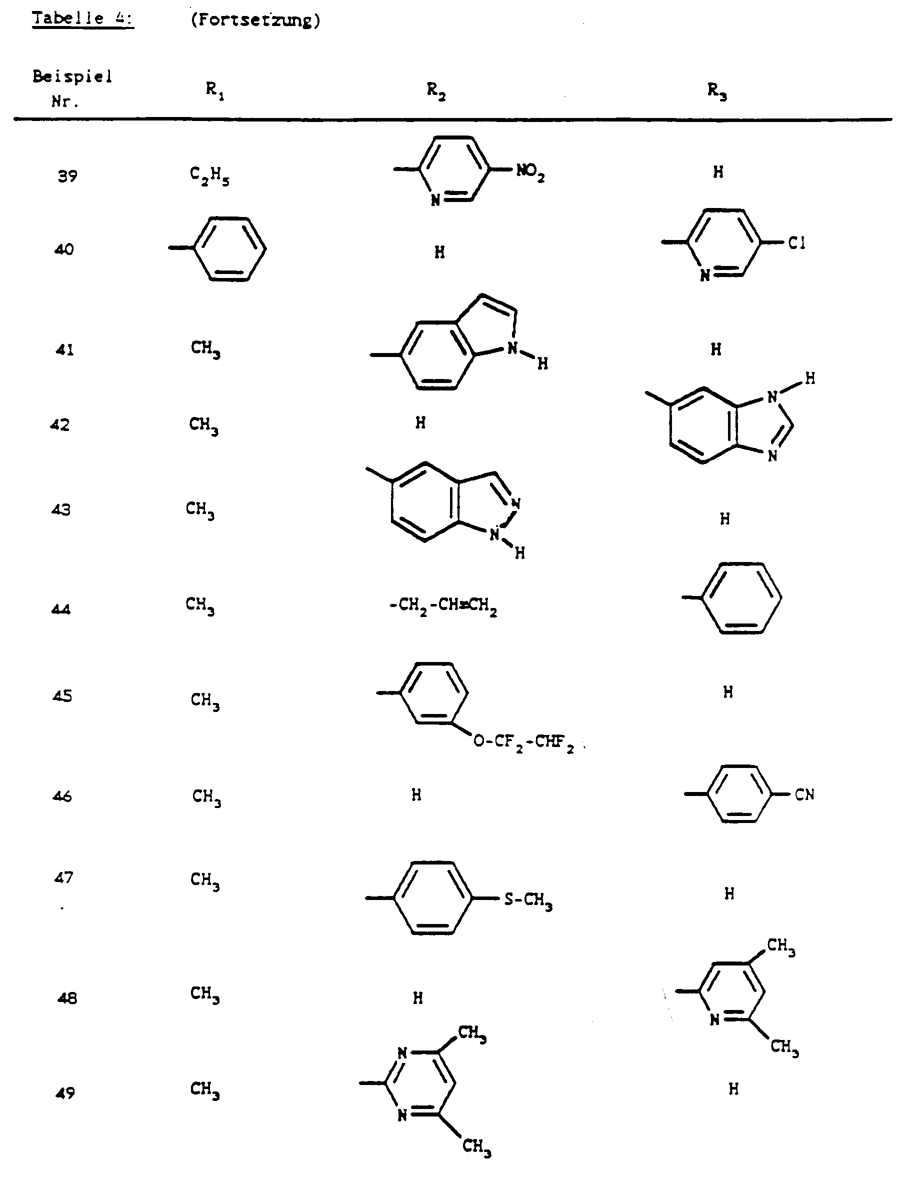

## Patentansprüche (Patentansprüche für folgende(n) Vertragsstaat(en): AT, BE, CH, DE, DK, FR, GB, IT, LI, LU, NL, SE)

1. Verwendung Von mindestens einer Verbindung der Formel I, Ia oder Ib 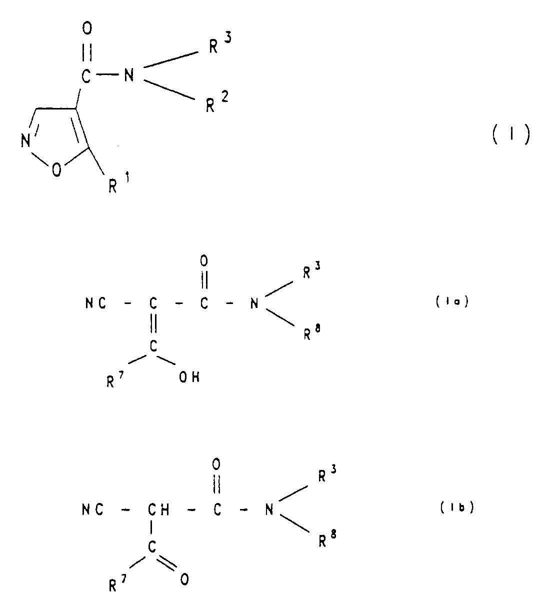 ihre gegebenenfalls stereoisomeren Formen und/oder gegebenenfalls mindestens einem von deren physiologisch verträglichen Salzen, wobei
R¹ für
a) Wasserstoff,
b) Alkyl mit 1 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl,
R² für
a) Wasserstoff,
b) Alkyl mit 1 bis 4 C-Atomen,
c) Phenyl-(C₁-C₂)-alkyl, insbesondere Benzyl,
d) Alkenyl mit 2 bis 3 C-Atomen,
R³ für
a) einen ein-, zwei- oder dreikernigen, ungesättigten heterocyclischen Rest mit 3 bis 13 C-Atomen und 1 bis 4 Heteroatomen aus der Gruppe Sauerstoff, Schwefel und Stickstoff, wovon höchstens eines von Stickstoff verschieden ist, im Ringsystem bedeutet, unsubstituiert oder ein- oder mehrfach substituiert durch
1) Halogen, wie Fluor, Chlor, Brom oder Jod,
2) Alkyl mit 1 bis 3 C-Atomen,
3) Alkyl mit 1 bis 3 C-Atomen ein- oder mehrfach substituiert durch
3.1 Halogen, wie Fluor, Chlor, Brom oder Jod,
4) Alkoxy mit 1 bis 3 C-Atomen,
5) Alkoxy mit 1 bis 3 C-Atomen, ein- oder mehrfach substituiert durch
5.1 Halogen, wie Fluor, Chlor, Brom oder Jod,
6) Nitro,
7) Hydroxy,
8) Carboxy,
9) Carbamoyl,
10) Oxogruppe,
b) einen Rest der Formel II, 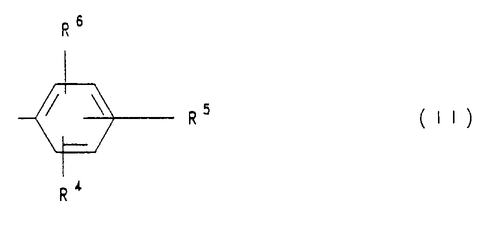 in der R⁴, R⁵ und R⁶ gleich oder verschieden sein können und für
1) Wasserstoff,
2) Phenoxy,
3) Alkyl mit 1 bis 3 C-Atomen, ein- oder mehrfach substituiert durch
3.1 Halogen, wie Fluor, Chlor, Brom oder Jod,
4) in der R⁴ für Wasserstoff und R⁵ und R⁶ gemeinsam mit dem Phenylring der Formel II einen Naphthalinring bilden,
5) in der R⁴ für Wasserstoff und R⁵ und R⁶ einen Methylendioxyrest bilden,
6) Alkoxy mit 1 bis 3 C-Atomen,
7) Alkoxy mit 1 bis 3 C-Atomen, ein- oder mehrfach substituiert durch
7.1 Halogen, wie Fluor, Chlor, Brom oder Jod,
8) (C₁-C₃)-Alkylmercapto,
9) (C₁-C₃)-Alkylmercapto, ein- oder mehrfach substituiert durch
9.1 Halogen, wie Fluor, Chlor, Brom oder Jod,
10) Halogen, wie Fluor, Chlor, Brom oder Jod,
11) Nitro,
12) Cyano,
13) Hydroxy,
14) Carboxy,
15) (C₁-C₃)-Alkylsulfonyl,
16) Carbalkoxy, mit 1 bis 3 C-Atomen in der Alkylkette,
17) Benzoyl,
18) Benzoyl, ein- oder mehrfach substituiert durch
18.1 Halogen, wie Fluor, Chlor, Brom oder Jod,
18.2 (C₁-C₃)-Alkyl,
18.3 (C₁-C₃)-Alkoxy,
19) Phenyl,
20) Phenyl, ein- oder mehrfach substituiert durch
20.1 (C₁-C₃)-Alkoxy,
20.2 Halogen, wie Fluor, Chlor, Brom oder Jod,
20.3 (C₁-C₃)-Alkyl,
21) Phenoxy, ein- oder mehrfach substituiert durch
21.1 (C₁-C₃)-Alkoxy, ein- oder mehrfach substituiert durch
21.1.1 Halogen, wie Fluor,Chlor,Brom oder Jod,
21.2 Halogen, wie Fluor, Chlor, Brom oder Jod,
21.3 (C₁-C₃)-Alkyl ein- oder mehrfach substituiert durch
21.3.1 Halogen, wie Fluor, Chlor, Brom oder Jod,
c) einen Rest der Formel III,
-(CH₂)ₙ-COOR¹⁰ (III)
in der R¹⁰ für
1) Wasserstoff
2) Alkyl mit 1 bis 4 C-Atomen,
n für eine ganze Zahl von 1 bis 12,
d) R² und R³ zusammen mit dem Stickstoff, an das sie gebunden sind, einen 4- bis 9-gliedrigen Ring bilden, substituiert durch,
1) Carbonyl am N-Atom benachbarten C-Atom,
e) R² und R³ zusammen mit dem Stickstoff, an das sie gebunden sind, einen 5- bis 6-gliedrigen Ring der Formel IV bilden, 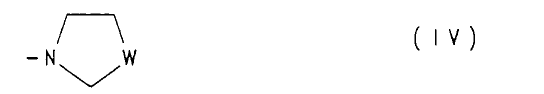 in der W für
1) -CH₂-,
2) -CH₂-CH₂-,
3) 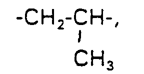
4) 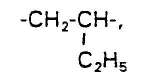
5) 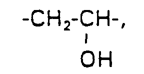
6) -CH₂-O- oder
7) -CH₂-S-
R⁷ für
a) Wasserstoff,
b) Alkyl mit 1 bis 17 C-Atomen,
c) Alkyl mit 1 bis 3 C-Atomen, ein- oder mehrfach substituiert durch Halogen wie Fluor, Chlor, Brom oder Jod,
d) Phenyl-(C₁-C₂)-alkyl, insbesondere Benzyl,
R⁸ für
a) Wasserstoff,
b) Methyl,
c) Alkenyl mit 2 bis 3 C-Atomen,
steht, zur Herstellung von Arzneimitteln zur Behandlung von Krebserkrankungen.

2. Verwendung gemäß Anspruch 1, dadurch gekennzeichnet, daß
R¹ für
a) Wasserstoff,
b) Alkyl mit 1 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl
R² für
a) Wasserstoff,
b) Alkyl mit 1 bis 4 C-Atomen,
c) Phenyl-(C₁-C₂)-alkyl, insbesondere Benzyl,
d) Alkenyl mit 2 bis 3 C-Atomen,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 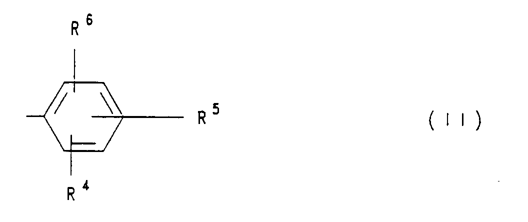 in der R⁴, R⁵ und R⁶ gleich oder verschieden sein können und für
1) Wasserstoff,
2) Phenoxy,
3) Alkyl mit 1 bis 3 C-Atomen, ein oder mehrfach substituiert durch
3.1 Halogen, wie Fluor Chlor, Brom oder Jod,
4) in der R⁴ für Wasserstoff und R⁵ und R⁶ einen Methylendioxyrest bilden,
5) Alkoxy mit 1 bis 3 C-Atomen,
6) Alkoxy mit 1 bis 3 C-Atomen, ein- oder mehrfach substituiert durch
6.1 Halogen, wie Fluor, Chlor, Brom oder Jod,
7) (C₁-C₃)-Alkylmercapto,
8) (C₁-C₃)-Alkylmercapto, ein- oder mehrfach substituiert durch
8.1 Halogen, wie Fluor, Chlor, Brom oder Jod,
9) Halogen, wie Fluor, Chlor, Brom oder Jod,
10) Nitro,
11) Cyano,
12) (C₁-C₃)-Alkylsulfonyl,
13) Benzoyl,
14) Benzoyl, ein- oder mehrfach substituiert durch
14.1 Halogen, wie Fluor, Chlor, Brom oder Jod,
14.2 (C₁-C₃)-Alkyl,
14.3 (C₁-C₃)-Alkoxy,
15) Phenoxy, ein- oder mehrfach substituiert durch
15.1 (C₁-C₃)-Alkoxy, ein- oder mehrfach substituiert durch
15.1.1 Halogen, wie Fluor,Chlor,Brom oder Jod,
15.2 Halogen, wie Fluor, Chlor, Brom oder Jod,
15.3 (C₁-C₃)-Alkyl, ein- oder mehrfach substituiert durch
15.3.1 Halogen, wie Fluor,Chlor,Brom oder Jod,
c) einen Rest der Formel III,
-(CH₂)ₙ-COOR¹⁰ (III)
in der R¹⁰ für
1) Wasserstoff
2) Alkyl mit 1 bis 4 C-Atomen,
n für eine ganze Zahl von 1 bis 12,
d) R² und R³ zusammen mit dem Stickstoff, an das sie gebunden sind, einen 4- bis 9-gliedrigen Ring bilden, substituiert durch,
1) Carbonyl am N-Atom benachbarten C-Atom,
R⁷ für
a) Wasserstoff,
b) Alkyl mit 1 bis 17 C-Atomen,
c) Alkyl mit 1 bis 3 C-Atomen, ein- oder mehrfach substituiert durch Halogen, wie Fluor Chlor, Brom oder Jod,
d) Phenyl-(C₁-C₂)-alkyl, insbesondere Benzyl,
R⁸ für
a) Wasserstoff,
b) Methyl,
c) Alkenyl mit 2 bis 3 C-Atomen,
steht.

3. Verwendung gemäß Anspruch 1, dadurch gekennzeichnet, daß
R¹ für Alkyl mit 1 bis 6 C-Atomen,
R² für Wasserstoff,
R³ für
a) Pyridyl, ein- oder mehrfach substituiert durch Halogen, wie Fluor, Chlor, Brom oder Jod,
b) einen Rest der Formel II, 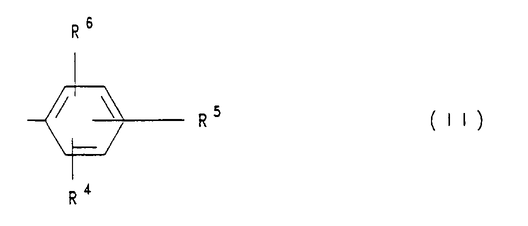 in der R⁴, R⁵ und R⁶ gleich oder verschieden sein können und für
1) Wasserstoff,
2) Phenoxy,
3) Alkyl mit 1 bis 3 C-Atomen, ein- oder mehrfach substituiert durch
3.1 Halogen, wie Fluor, Chlor, Brom oder Jod,
4) in der R⁴ für Wasserstoff und R⁵ und R⁶ einen Methylendioxyrest bilden,
5) Alkoxy mit 1 bis 3 C-Atomen,
6) Alkoxy mit 1 bis 3 C-Atomen, ein- oder mehrfach substituiert durch
6.1 Halogen, wie Fluor, Chlor, Brom oder Jod,
7) Halogen, wie Fluor, Chlor, Brom oder Jod,
8) Nitro,
9) Benzoyl,
10) Benzoyl, ein- oder mehrfach substituiert durch
10.1 Halogen, wie Fluor, Chlor, Brom oder Jod,
10.2 (C₁-C₃)-Alkyl,
10.3 (C₁-C₃)-Alkoxy,
11) Phenoxy, ein- oder mehrfach substituiert durch
11.1 (C₁-C₃)-Alkoxy, ein- oder mehrfach substituiert durch
11.1.1 Halogen, wie Fluor,Chlor,Brom oder Jod,
11.2 Halogen, wie Fluor, Chlor, Brom oder Jod,
11.3 (C₁-C₃)-Alkyl, ein- oder mehrfach substituiert durch
11.3.1 Halogen, wie Fluor,Chlor,Brom oder Jod,
R⁷ für Alkyl mit 1 bis 17 C-Atomen,
R⁸ für Wasserstoff
steht.

4. Verwendung von Methylisoxazol-4-carbonsäure-(4-trifluormethyl)-anilid oder N-(4-Trifluormethylphenyl)-2-cyano-3-hydroxy-crotonsäureamid zur Herstellung von Arzneimitteln zur Behandlung von Krebserkrankungen.

5. Verfahren zur Herstellung eines Arzneimittels zur Behandlung von Krebserkrankungen, dadurch gekennzeichnet, daß man mindestens eine Verbindung der Formel I, Ia oder Ib nach Anspruch 1, ihre gegebenenfalls stereoisomeren Formen und/oder mindestens einem von deren physiologisch verträglichen Salzen mit einem physiologisch annehmbaren Träger und gegebenenfalls weiteren Zusatz- und/oder Hilfsstoffen in eine geeignete Darreichungsform bringt.

6. Verwendung von einer oder mehreren der Verbindungen nach Anspruch 1 zur Herstellung von Arzneimitteln zur Behandlung von Leukämie-, Sarkom-, Lymphknotentumor- oder Hautkrebserkrankungen.

7. Verbindung der Formel I 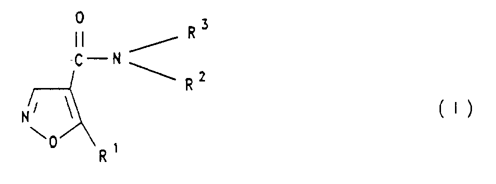 ihre gegebenenfalls stereoisomeren Formen und/oder gegebenenfalls ihre physiologisch verträglichen Salze, wobei
R¹ für
a) Wasserstoff,
b) Alkyl mit 2 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl,
R² für
a) Wasserstoff,
b) Alkenyl mit 2 bis 3 C-Atomen,
c) Benzyl,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 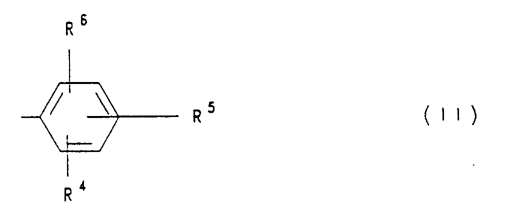 in der R⁴, R⁵, R⁶ gleich oder verschieden sein können und für
1) Halogen, wie Fluor, Chlor, Brom oder Jod,
2) Nitro,
3) Wasserstoff,
4) Benzoyl, ein- oder mehrfach substituiert durch
4.1 Halogen, wie Fluor, Chlor, Brom oder Jod,
4.2 Methyl,
4.3 Methoxy,
4.4 Benzoyl,
5) (C₁-C₃-)Alkoxy, ein- oder mehrfach substituiert durch
5.1 Halogen, wie Fluor, Chlor, Brom oder Jod,
6) (C₁-C₃)-Alkyl, ein- oder mehrfach substituiert durch
6.1 Halogen, wie Fluor, Chlor, Brom oder Jod,
7) Hydroxy,
8) Alkylsulfonyl, mit 1 bis 3 C-Atomen in der Alkylkette,
9) in der R⁴ für Wasserstoff und R⁵ und R⁶ gemeinsam einen Methylendioxyrest bilden,
10) Cyano,
11) (C₁-C₃)-Alkylmercapto,
12) Benzoyl,
c) Pyrimidinyl, ein- oder mehrfach substituiert durch
1) Alkyl mit 1 bis 3 C-Atomen,
d) Indolyl oder
e) Indazolinyl
steht.

8. Verbindung gemäß Anspruch 7, dadurch gekennzeichnet daß
R¹ für
a) Wasserstoff,
b) Alkyl mit 2 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl,
R² für
a) Wasserstoff,
b) Alkenyl mit 2 bis 3 C-Atomen,
c) Benzyl,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 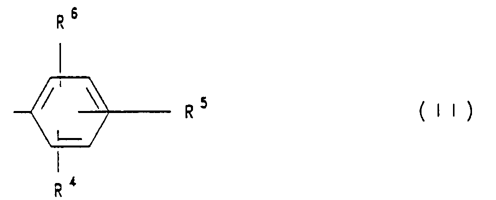 in der R⁴, R⁵, R⁶ gleich oder verschieden sein können und für
1) Halogen, wie Fluor, Chlor, Brom oder Jod,
2) Nitro,
3) Wasserstoff,
4) Benzoyl, ein- oder mehrfach substituiert durch
4.1 Halogen, wie Fluor, Chlor, Brom oder Jod,
4.2 Methyl,
4.3 Methoxy,
4.4 Benzoyl,
5) (C₁-C₃-)Alkoxy, ein- oder mehrfach substituiert durch
5.1 Halogen, wie Fluor, Chlor, Brom oder Jod,
6) (C₁-C₃)-Alkyl, ein- oder mehrfach substituiert durch
6.1 Halogen, wie Fluor, Chlor, Brom oder Jod,
7) Hydroxy,
8) Alkylsulfonyl, mit 1 bis 3 C-Atomen in der Alkylkette,
9) in der R⁴ für Wasserstoff und R⁵ und R⁶ gemeinsam einen Methylendioxyrest bilden,
10) Cyano,
11) (C₁-C₃)-Alkylmercapto,
12) Benzoyl,
c) Pyrimidinyl, ein- oder mehrfach substituiert durch
1) Alkyl mit 1 bis 3 C-Atomen,
d) Indolyl oder e) Indazolinyl
steht.

9. Verbindung gemäß Anspruch 7, dadurch gekennzeichnet, daß
R¹ für Alkyl mit 2 bis 6 C-Atomen,
R² für Wasserstoff,
R³ für
a) Pyridyl, ein- oder mehrfach substituiert durch
1) Wasserstoff,
2) Halogen, wie Fluor, Chlor, Brom oder Jod,
b) einen Rest der Formel II, 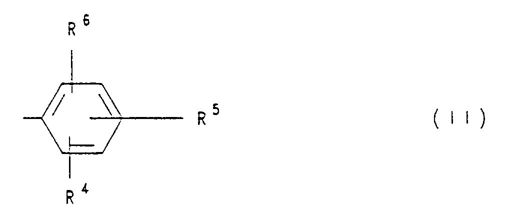 in der R⁴, R⁵, R⁶ gleich oder verschieden sein können und für
1) Halogen, wie Fluor, Chlor, Brom oder Jod,
2) Nitro,
3) Benzoyl,
4) Benzoyl, ein- oder mehrfach substituiert durch
4.1 Halogen, wie Fluor, Chlor, Brom oder Jod,
4.2 Methyl,
5) (C₁-C₃-)Alkoxy, ein- oder mehrfach substituiert durch
5.1 Halogen, wie Fluor, Chlor, Brom oder Jod,
6) (C₁-C₃)-Alkyl, ein- oder mehrfach substituiert durch
6.1 Halogen, wie Fluor, Chlor, Brom oder Jod,
7) Wasserstoff,
8) in der R⁴ für Wasserstoff und R⁵ und R⁶ gemeinsam einen Methylendioxyrest bilden,
steht.

10. Verbindung der Formel Ia oder der Formel Ib, 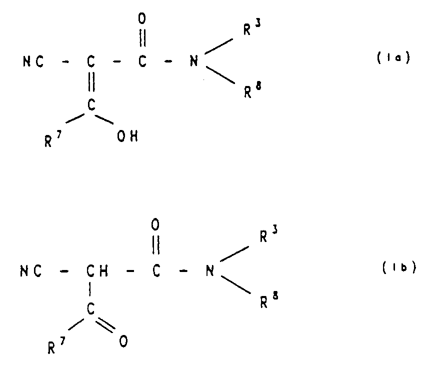 ihre gegebenenfalls stereoisomeren Formen und/oder gegebenenfalls ihre physiologisch verträglichen Salze, wobei die Reste R⁷, R⁸ und R³ unter
a) mit R⁷ für
1) Wasserstoff,
2) Alkyl mit 2 bis 4 C-Atomen,
mit R⁸ für
1) Wasserstoff,
2) Methyl,
mit R³ für
1) Phenyl,
2) Phenyl, ein- oder mehrfach substituiert durch
2.1 Halogen,
2.2. Alkyl mit 1 bis 3 C-Atomen, ein- oder mehrfach substituiert durch Halogen, wie Fluor, Chlor, Brom oder Jod steht
b) mit R⁷ für
1) Alkyl mit 1 bis 4 C-Atomen,
2) Wasserstoff,
3) CF₃,
mit R⁸ für
1) Wasserstoff,
2) Methyl,
3) Alkenyl mit 2 bis 3 C-Atomen,
mit R³ für
1) Pyridyl,
2) Pyridyl, ein- oder mehrfach substituiert durch
2.1 Halogen, wie Fluor, Chlor, Brom oder Jod,
2.2 Alkyl mit 1 bis 3 C-Atomen,
3) Pyrimidinyl, substituiert wie bei 2)
4) Thiazolyl, substituiert wie bei 2) und
4.1 Alkoxycarbonyl, mit 1 bis 3 C-Atomen in der Alkylkette,
5) Benzothiazolyl, substituiert wie bei 2),
6) Benzimidazolyl, substituiert wie bei 2),
7) Indazolyl, substituiert wie bei 2),
8) Phenyl, ein- oder mehrfach substituiert durch
8.1 Benzoyl,
8.2 Benzoyl, ein- oder mehrfach substituiert durch
8.2.1 Halogen, wie Fluor, Chlor, Brom oder Jod,
8.2.2 Alkyl mit 1 bis 3 C-Atomen,
8.2.3 Alkoxy mit 1 bis 3 C-Atomen,
8.2.4 Alkoxy mit 1 bis 3 C-Atomen, ein- oder mehrfach substituiert durch Halogen, wie Fluor, Chlor, Brom oder Jod,
8.3. Carboxy oder
8.4 Hydroxy
steht,
c) mit R⁷ für
1) Wasserstoff,
2) Alkyl mit 1 bis 4 C-Atomen,
mit R⁸ für
1) Wasserstoff,
2) Methyl,
mit R³ für
1) einen Rest der Formel III,
-(CH₂)₄-COOR¹⁰
in der R¹⁰ für
1.1. Wasserstoff,
1.2 Alkyl mit 1 bis 4 C-Atomen steht
d) mit R⁷ für
1) Wasserstoff,
2) Alkyl mit 1 bis 4 C-Atomen,
steht,
R⁸ und R³ zusammen mit dem Stickstoff, an das sie gebunden sind, einen 4- bis 9-gliedrigen Ring bilden, substituiert durch
2.1. Carbonyl am N-Atom benachbarten C-Atom, oder
R⁸ und R³ zusammen mit Stickstoff, an das sie gebunden sind, einen Piperidinring bilden
gegebenenfalls substituiert durch Alkyl mit 1 bis 3 C-Atomen, bedeuten.

11. Verbindung gemäß Anspruch 10, dadurch gekennzeichnet daß die Reste R⁷, R⁸ und R³ unter
a) mit R⁷ für Alkyl mit 2 bis 4 C-Atomen,
mit R⁸ für Wasserstoff,
mit R³ für
1) Phenyl,
2) Phenyl, ein- oder mehrfach substituiert durch
2.1 Halogen, wie Fluor, Chlor, Brom oder Jod,
2.2 Alkyl mit 1 bis 3 C-Atomen, ein- oder mehrfach substituiert durch Halogen, wie Fluor, Chlor, Brom oder Jod steht,
b) mit R⁷ für Alkyl mit 1 bis 4 C-Atomen,
mit R⁸ für Wasserstoff,
mit R³ für
1) Pyridyl,
2) Pyridyl, ein- oder mehrfach substituiert durch
2.1 Halogen, wie Fluor, Chlor, Brom oder Jod,
2.2 Alkyl mit 1 bis 3 C-Atomen,
3) Phenyl, ein- oder mehrfach substituiert durch
3.1 Benzoyl,
3.2 Benzoyl, ein- oder mehrfach subsitutiert durch
3.2.1 Halogen, wie Fluor, Chlor, Brom oder Jod,
3.2.2 Alkyl mit 1 bis 3 C-Atomen,
3.2.3 Alkoxy mit 1 bis 3 C-Atomen,
3.2.4 Alkoxy mit 1 bis 3 C-Atomen, ein- oder mehrfach substituiert durch Halogen, wie Fluor, Chlor, Brom oder Jod, steht,
bedeuten.

12. Verbindung N-(4-Chlordifluormethoxy)-phenyl-5-ethylisoxazol-4-carboxamid, N-(4-Methylsulfonylphenyl)-5-methylisoxazol-4-carboxamid, N-(5-Indolyl)-5-methylisoxazol-4-carboxamid, N-(6-Indazolyl)-5-methyl-isoxazol-4-carboxamid, N-(5-Indazolyl)-5-methylisoxazol-4-carboxamid, N-Allyl-N-phenyl-5-methyl-isoxazol-4-carboxamid, N-(4,6-Dimethyl-2-pyridyl)-5-methylisoxazol-4-carboxamid, N-(4,6-Dimethyl-2-pyrimidinyl)-5-methylisoxazol-4-carboxamid, N-(4-Trifluormethoxyphenyl)-2-cyano-3-hydroxy-crotonsäureamid, N-[4-(1,1,2,2-Tetrafluorethoxy)-phenyl]-2-cyano-3-hydroxycrotonsäureamid, N-[4-(4-Fluorbenzoyl)-phenyl]-2-cyano-3-hydroxycrotonsäureamid oder N-(3,4-Methylendioxyphenyl)-2-cyano-3-hydroxy-4-methyl-crotonsäureamid.

13. Verwendung von mindestens einer Verbindung der Formel I, Ia oder Ib nach Anspruch 7 bis 12, ihre gegebenenfalls stereoisomeren Formen und/oder mindestens einem von deren physiologisch verträglichen Salzen zur Herstellung von Arzneimitteln zur Behandlung von Rheumaerkrankungen.

14. Arzneimittel, gekennzeichnet durch einen Gehalt an -oder bestehend ausmindestens einer Verbindung der Formel I, Ia oder Ib nach Anspruch 7 bis 12, ihre gegebenenfalls stereoisomeren Formen und/oder mindestens einem von deren physiologisch verträglichen Salzen.

15. Verfahren zur Herstellung eines Arzneimittel, dadurch gekennzeichnet, daß man mindestens eine Verbindung der Formel I, Ia oder Ib nach Anspruch 7 bis 12, ihre gegebenenfalls stereoisomeren Formen und/oder mindestens einem von deren physiologisch verträglichen Salzen mit einem physiologisch annehmbaren Träger und gegebenenfalls weiteren Zusatz- und/oder Hilfsstoffen in eine geeignete Darreichungsform bringt.

16. Verwendung von mindestens einer Verbindung der Formel I, Ia oder Ib nach Anspruch, 7 bis 12, ihre gegebenenfalls stereoisomeren Formen und/oder mindestens einem von deren physiologisch verträglichen Salzen zur Herstellung von Arzneimitteln zur Behandlung von Krebserkrankungen.

17. Verwendung von einer oder mehreren Verbindungen nach Anspruch 16 zur Herstellung von Arzneimitteln zur Behandlung von Leukämie-, Sarkom-, Lymphknotentumor- und Hautkrebserkrankungen.

## Patentansprüche (Patentansprüche für folgende(n) Vertragsstaat(en): GR)

1. Verwendung von mindestens einer Verbindung der Formel I, Ia oder Ib 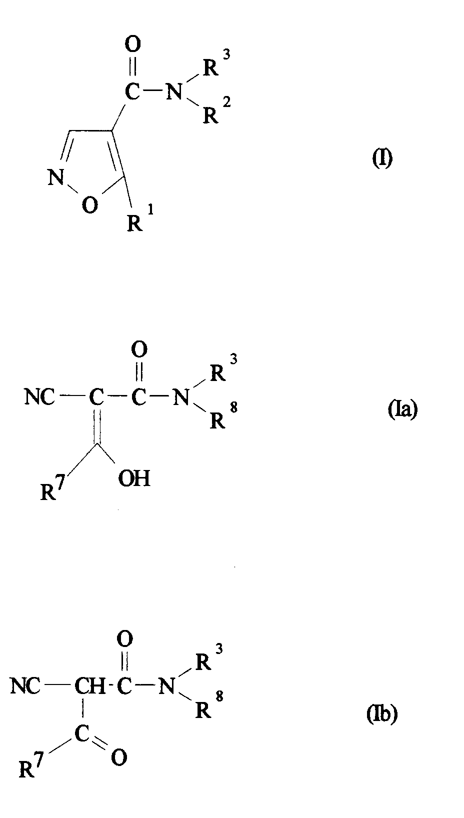 ihre gegebenenfalls stereoisomeren Formen und/oder gegebenenfalls mindestens einem von deren physiologisch verträglichen Salzen, wobei
R¹ für
a) Wasserstoff,
b) Alkyl mit 1 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl,
R² für
a) Wasserstoff,
b) Alkyl mit 1 bis 4 C-Atomen,
c) Phenyl-(C₁-C₂)-alkyl, insbesondere Benzyl,
d) Alkenyl mit 2 bis 3 C-Atomen,
R³ für
a) einen ein-, zwei- oder dreikernigen, ungesättigten heterocyclischen Rest mit 3 bis 13 C-Atomen und 1 bis 4 Heteroatomen aus der Gruppe Sauerstoff, Schwefel und Stickstoff, wovon höchstens eines von Stickstoff verschieden ist, im Ringsystem bedeutet, unsubstituiert oder ein- oder mehrfach substituiert durch
1) Halogen, wie Fluor, Chlor, Brom oder Jod,
2) Alkyl mit 1 bis 3 C-Atomen,
3) Alkyl mit 1 bis 3 C-Atomen ein- oder mehrfach substitutert durch
3.1 Halogen, wie Fluor, Chlor, Brom oder Jod,
4) Alkoxy mit 1 bis 3 C-Atomen,
5) Alkoxy mit 1 bis 3 C-Atomen, ein- oder mehrfach substituiert durch
5.1 Halogen, wie Fluor, Chlor, Brom oder Jod,
6) Nitro,
7) Hydroxy,
8) Carboxy,
9) Carbamoyl,
10) Oxogruppe,
b) einen Rest der Formel II, 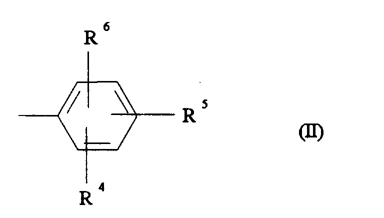 in der R⁴, R⁵ und R⁶ gleich oder verschieden sein können und für
1) Wasserstoff,
2) Phenoxy,
3) Alkyl mit 1 bis 3 C-Atomen, ein- oder mehrfach substituiert durch 3.1 Halogen, wie Fluor, Chlor, Brom oder Jod,
4) in der R⁴ für Wasserstoff und R⁵ und R⁶ gemeinsam mit dem Phenylring der Formel II einen Naphthalinring bilden,
5) in der R⁴ für Wasserstoff und R⁵ und R⁶ einen Methylendioxyrest bilden,
6) Alkoxy mit 1 bis 3 C-Atomen,
7) Alkoxy mit 1 bis 3 C-Atomen, ein- oder mehrfach substituiert durch 7.1 Halogen, wie Fluor, Chlor, Brom oder Jod,
8) (C₁-C₃)-Alkylmercapto,
9) (C₁-C₃)-Alkylmercapto, ein- oder mehrfach substituiert durch 9.1 Halogen, wie Fluor, Chlor, Brom oder Jod,
10) Halogen, wie Fluor, Chlor, Brom oder Jod,
11) Nitro,
12) Cyano,
13) Hydroxy,
14) Carboxy,
15) (C₁-C₃)-Alkylsulfonyl,
16) Carbalkoxy, mit 1 bis 3 C-Atomen in der Alkylkette,
17) Benzoyl,
18) Benzoyl, ein-oder mehrfach substituiert durch
18.1 Halogen, wie Fluor, Chlor, Brom oder Jod,
18.2 (C₁-C₃)-Alkyl,
18.3 (C₁-C₃)-Alkoxy,
19) Phenyl,
20) Phenyl, ein- oder mehrfach substituiert durch
20.1 (C₁-C₃)-Alkoxy,
20.2 Halogen, wie Fluor, Chlor, Brom oder Jod,
20.3 (C₁-C₃)-Alkyl,
21) Phenoxy, ein- oder mehrfach substituiert durch
21.1 (C₁-C₃)-Alkoxy, ein- oder mehrfach substituiert durch
21.1.1 Halogen, wie Fluor, Chlor, Brom oder Jod,
21.2 Halogen, wie Fluor, Chlor, Brom oder Jod,
21.3 (C₁-C₃)-Alkyl ein- oder mehrfach substituiert durch
21.3.1 Halogen, wie Fluor, Chlor, Brom oder Jod,
c) einen Rest der Formel III,
-(CH₂)ₙ-COOR¹⁰ (III)
in der R¹⁰ für
1) Wasserstoff,
2) Alkyl mit 1 bis 4 C-Atomen,
n für eine ganze Zahl von 1 bis 12,
d) R² und R³ zusammen mit dem Stickstoff, an das sie gebunden sind, einen 4- bis 9-gliedrigen Ring bilden, substituiert durch,
1) Carbonyl am N-Atom benachbarten C-Atom,
e) R² und R³ zusammen mit dem Stickstoff, an das sie gebunden sind, einen 5- bis 6-gliedrigen Ring der Formel IV bilden, 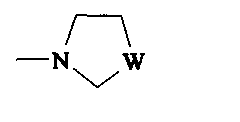 in der W für
1) -CH₂-,
2) -CH₂-CH₂-,
3) 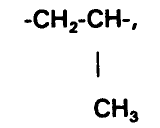
4) 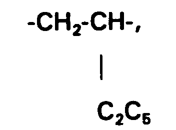
5) 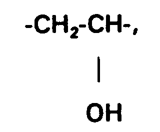
6) -CH₂-O- oder
7) -CH₂-S-
R⁷ für
a) Wasserstoff,
b) Alkyl mit 1 bis 17 C-Atomen,
c) Alkyl mit 1 bis 3 C-Atomen, ein- oder mehrfach substituiert durch Halogen wie Fluor, Chlor, Brom oder Jod,
d) Phenyl-(C₁-C₂)-alkyl, Insbesondere Benzyl,
R⁸ für
a) Wasserstoff,
b) Methyl,
c) Alkenyl mit 2 bis 3 C-Atomen,
steht, zur Herstellung von Arzneimitteln zur Behandlung von Krebserkrankungen.

2. Verwendung gemäß Anspruch 1, dadurch gekennzeichnet, daß
R¹ für
a) Wasserstoff,
b) Alkyl mit 1 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl
R² für
a) Wasserstoff,
b) Alkyl mit 1 bis 4 C-Atomen,
c) Phenyl-(C₁-C₂)-alkyl, insbesondere Benzyl,
d) Alkenyl mit 2 bis 3 C-Atomen,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 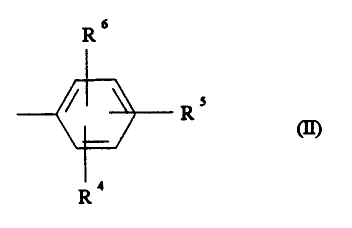 in der R⁴, R⁵ und R⁶ gleich oder verschieden sein können und für
1) Wasserstoff,
2) Phenoxy,
3) Alkyl mit 1 bis 3 C-Atomen, ein- oder mehrfach substituiert durch
3.1 Halogen, wie Fluor, Chlor, Brom oder Jod,
4) in der R⁴ für Wasserstoff und R⁵ und R⁶ einen Methylendioxyrest bilden,
5) Alkoxy mit 1 bis 3 C-Atomen,
6) Alkoxy mit 1 bis 3 C-Atomen, ein- oder mehrfach substituiert durch
6.1 Halogen, wie Fluor, Chlor, Brom oder Jod,
7) (C₁-C₃)-Alkylmercapto,
8) (C₁ -C₃)-Alkylmercapto, ein- oder mehrfach substituiert durch
8.1 Halogen, wie Fluor, Chlor, Brom oder Jod,
9) Halogen, wie Fluor, Chlor, Brom oder Jod,
10) Nitro,
11) Cyano,
12) (C₁-C₃)-Alkylsulfonyl,
13) Benzoyl,
14) Benzoyl, ein- oder mehrfach substituiert durch
14.1 Halogen, wie Fluor, Chlor, Brom oder Jod,
14.2 (C₁-C₃)-Alkyl,
14.3 (C₁-C₃)-Alkoxy,
15) Phenoxy, ein- oder mehrfach substituiert durch
15.1 (C₁-C₃)-Alkoxy, ein- oder mehrfach substituiert durch
15.1.1 Halogen, wie Fluor, Chlor, Brom oder Jod,
15.2 Halogen, wie Fluor, Chlor, Brom oder Jod,
15.3 (C₁-C₃)-Alkyl ein- oder mehrfach substituiert durch
15.3.1 Halogen, wie Fluor, Chlor, Brom oder Jod,
c) einen Rest der Formel III,
-(CH₂)ₙ-COOR¹⁰ (III)
in der R¹⁰ für
1) Wasserstoff,
2) Alkyl mit 1 bis 4 C-Atomen,
n für eine ganze Zahl von 1 bis 12,
d) R² und R³ zusammen mit dem Stickstoff, an das sie gebunden sind, einen 4- bis 9-gliedrigen Ring bilden, substituiert durch,
1) Carbonyl am N-Atom benachbarten C-Atom,
R⁷ für
a) Wasserstoff,
b) Alkyl mit 1 bis 17 C-Atomen,
c) Alkyl mit 1 bis 3 C-Atomen, ein- oder mehrfach substituiert durch Halogen wie Fluor, Chlor, Brom oder Jod,
d) Phenyl-(C₁-C₂)-alkyl, insbesondere Benzyl,
R⁸ für
a) Wasserstoff,
b) Methyl,
c) Alkenyl mit 2 bis 3 C-Atomen,
steht.

3. Verwendung gemäß Anspruch 1, dadurch gekennzeichnet, daß
R¹ für Alkyl mit 1 bis 6 C-Atomen,
R² für Wasserstoff,
R³ für
a) Pyridyl, ein- oder mehrfach substituiert durch Halogen, wie Fluor, Chlor, Brom oder Jod
b) einen Rest, der Formel II, 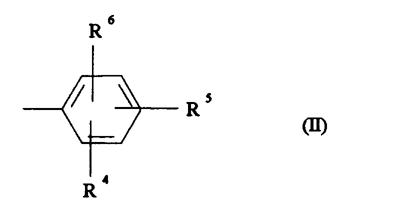 in der R⁴, R⁵ und R⁶ gleich oder verschieden sein können und für
1) Wasserstoff,
2) Phenoxy,
3) Alkyl mit 1 bis 3 C-Atomen, ein- oder mehrfach substituiert durch
3.1 Halogen, wie Fluor, Chlor, Brom oder Jod,
4) in der R⁴ für Wasserstoff und R⁵ und R⁶ einen Methylendioxyrest bilden,
5) Alkoxy mit 1 bis 3 C-Atomen,
6) Alkoxy mit 1 bis 3 C-Atomen, ein- oder mehrfach substituiert durch
6.1 Halogen, wie Fluor, Chlor, Brom oder Jod,
7) Halogen, wie Fluor, Chlor, Brom oder Jod,
8) Nitro,
9) Benzoyl,
10) Benzoyl, ein- oder mehrfach substituiert durch
10.1 Halogen, wie Fluor, Chlor, Brom oder Jod,
10.2 (C₁-C₃)-Alkyl,
10.3 (C₁-C₃)-Alkoxy,
11) Phenoxy, ein- oder mehrfach substituiert durch
11.1 (C₁-C₃)-Alkoxy, ein- oder mehrfach substituiert durch
11.1.1 Halogen, wie Fluor, Chlor, Brom oder Jod,
11.2 Halogen, wie Fluor, Chlor, Brom oder Jod,
11.3 (C₁-C₃)-Alkyl ein- oder mehrfach substituiert durch
11.3.1 Halogen, wie Fluor, Chlor, Brom oder Jod,
R⁷ für Alkyl mit 1 bis 17 C-Atomen,
R⁸ für Wasserstoff
steht.

4. Verwendung von Methylisoxazol-4-carbonsäure-(4-trifluormethyl)-anilid oder N-(4-Trifluormethylphenyl)-2-cyano-3-hydroxy-crotonsäureamid zur Herstellung von Arzneimitteln zur Behandlung von Krebserkrankungen.

5. Verwendung von einer oder mehreren der Verbindungen nach Anspruch 1 zur Herstellung von Arzneimitteln zur Behandlung von Leukämie-, Sarkom-, Lymphknotentumor- oder Hautkrebserkrankungen.

6. Verfahren zur Herstellung der Verbindung der Formel I 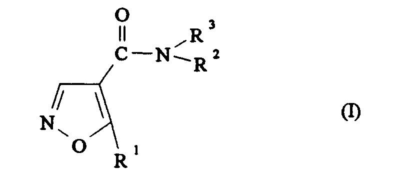 ihre gegebenenfalls stereoisomeren Formen und/oder gegebenenfalls ihre physiologisch verträglichen Salze, wobei
R¹ für
a) Wasserstoff,
b) Alkyl mit 2 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl
R² für
a) Wasserstoff,
b) Alkenyl mit 2 bis 3 C-Atomen,
c) Benzyl,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 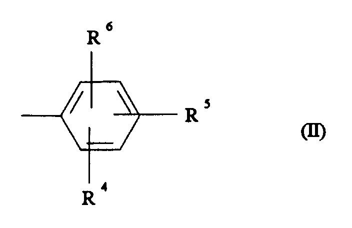 in der R⁴, R⁵ und R⁶ gleich oder verschieden sein können und für
1) Halogen, wie Fluor, Chlor, Brom oder Jod,
2) Nitro,
3) Wasserstoff,
4) Benzoyl, ein- oder mehrfach substituiert durch
4.1 Halogen, wie Fluor, Chlor, Brom oder Jod,
4.2 Methyl,
4.3 Methoxy,
4.4 Benzoyl,
5) (C₁-C₃)-Alkoxy, ein- oder mehrfach substituiert durch,
5.1 Halogen, wie Fluor, Chlor, Brom oder Jod
6) (C₁ -C₃)-Alkyl, ein- oder mehrfach substituiert durch
6.1 Halogen, wie Fluor, Chlor, Brom oder Jod,
7) Hydroxy,
8) Alkylsulfonyl, mit 1 bis 3 C-Atomen in der Alkylkette,
9) in der R⁴ für Wasserstoff und R⁵ und R⁶ gemeinsam einen Methylendioxyrest bilden,
10) Cyano,
11) (C₁-C₃)-Alkylmercapto,
12) Benzoyl,
c) Pyrimidinyl, ein- oder mehrfach substituiert durch
1) Alkyl mit 1 bis 3 C-Atomen,
d) Indolyl oder
e) Indazolinyl
steht, dadurch gekennzeichnet, daß man
a) eine Verbindung der Formel V, 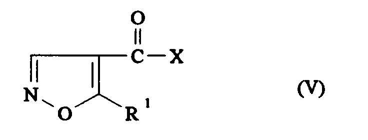 in der X für ein Halogenatom, vorzugsweise Chlor oder Brom, steht und R¹ die in Formel I angegebene Bedeutung hat,
mit dem Amin der Formel VI 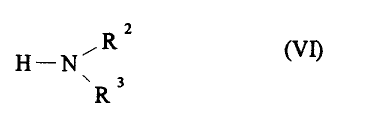 worin R² und R³ die in Formel I angegebene Bedeutung hat, umsetzt.

7. Verfahren gemäß Anspruch 6, dadurch gekennzeichnet, daß man eine Verbindung der Formel I herstellt, wobei
R¹ für
a) Wasserstoff,
b) Alkyi mit 2 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl,
R² für
a) Wasserstoff,
b) Alkenyl mit 2 bis 3 C-Atomen,
c) Benzyl,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 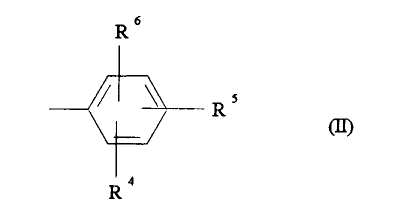 in der R⁴, R⁵ und R⁶ gleich oder verschieden sein können und für
1) Halogen, wie Fluor, Chlor, Brom oder Jod,
2) Nitro,
3) Wasserstoff,
4) Benzoyl, ein- oder mehrfach substituiert durch
4.1 Halogen, wie Fluor, Chlor, Brom oder Jod,
4.2 Methyl,
4.3 Methoxy,
4.4 Benzoyl,
5) (C₁-C₃)-Alkoxy, ein- oder mehrfach substituiert durch Halogen wie Fluor, Chlor, Brom oder Jod
6) (C₁ -C₃)-Alkyl, ein- oder mehrfach substituiert durch Halogen wie Fluor, Chlor, Brom oder Jod,
7) Hydroxy,
8) Alkylsulfonyl, mit 1 bis 3 C-Atomen in der Alkylkette,
9) in der R⁴ für Wasserstoff und R⁵ und R⁶ gemeinsam einen Methylendioxyrest bilden,
10) Cyano,
11) (C₁-C₃)-Alkylmercapto,
12) Benzoyl,
c) Pyrimidinyl, ein- oder mehrfach substituiert durch Alkyl mit 1 bis 3 C-Atomen,
d) Indolyl oder
e) Indazolinyl steht.

8. Verfahren gemäß Anspruch 6, dadurch gekennzeichnet, daß, man eine Verbindung der Formel I herstellt, wobei
R¹ für Alkyl mit 2 bis 6 C-Atomen,
R² für Wasserstoff,
R³ für
a) Pyridyl, ein- oder mehrfach substituiert durch
1) Wasserstoff,
2) Halogen, wie Fluor, Chlor, Brom oder Jod,
b) einen Rest der Formel II, 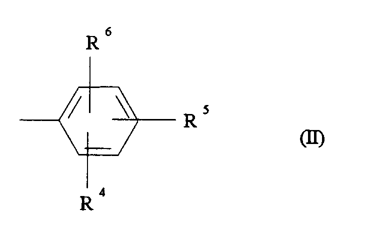 in der R⁴, R⁵ und R⁶ gleich oder verschieden sein können und für
1) Halogen, wie Fluor, Chlor, Brom oder Jod,
2) Nitro,
3) Benzoyl,
4) Benzoyl, ein- oder mehrfach substituiert durch
4.1 Halogen, wie Fluor, Chlor, Brom oder Jod,
4.2 Methyl,
5) (C₁-C₃)-Alkoxy, ein- oder mehrfach substituiert durch Halogen, wie Fluor, Chlor, Brom oder Jod
6) (C₁ -C₃)-Alkyl, ein- oder mehrfach substituiert durch Halogen wie Fluor, Chlor, Brom oder Jod,
7) Wasserstoff,
8) in der R⁴ für Wasserstoff und R⁵ und R⁶ gemeinsam einen Methylendioxyrest bilden, steht.

9. Verfahren zur Herstellung der Verbindung der Formel Ia oder der Formel Ib, 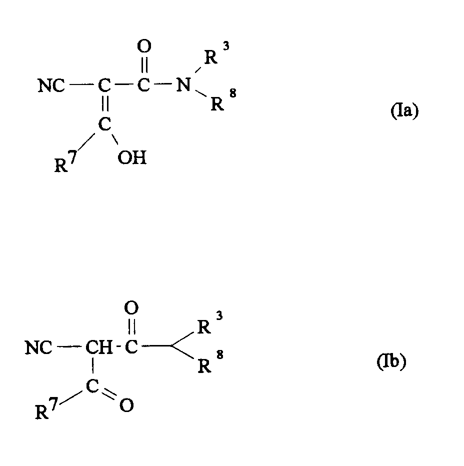 ihre gegebenenfalls stereoisomeren Formen und/oder gegebenenfalls ihre physiologisch verträglichen Salze, wobei die Reste R⁷, R⁸ und R⁹ unter
a) mit R⁷ für
1) Wasserstoff,
2) Alkyl mit 2 bis 4 C-Atomen,
mit R⁸ für
1) Wasserstoff,
2) Methyl,
mit R⁹ für
1) Phenyl,
2) Phenyl, ein- oder mehrfach substituiert durch
2.1 Halogen,
2.2. Alkyl mit 1 bis 3 C-Atomen, ein- oder mehrfach substituiert durch Halogen, wie Fluor, Chlor, Brom oder Jod, steht,
b) mit R⁷ für
1) Alkyl mit 1 bis 4 C-Atomen,
2) Wasserstoff,
3) CF₃,
mit R⁸ für
1) Wasserstoff,
2) Methyl,
3) Alkenyl mit 2 bis 3 C-Atomen,
mit R³ für
1) Pyridyl,
2) Pyridyl, ein- oder mehrfach substituiert durch
2.1 Halogen, wie Fluor, Chlor, Brom oder Jod,
2.2 Alkyl mit 1 bis 3 C-Atomen,
3) Pyrimidinyl, substituiert wie bei 2),
4) Thiazolyl, substituiert wie bei 2) und
4.1 Alkoxycarbonyl, mit 1 bis 3 C-Atomen in der Alkylkette,
5) Benzothiazolyl, substituiert wie bei 2),
6) Benzimidazolyl, substituiert wie bei 2),
7) Indazolyl, substituiert wie bei 2),
8) Phenyl, ein- oder mehrfach substituiert durch
8.1 Benzoyl,
8.2 Benzoyl, ein- oder mehrfach substituiert durch
8.2.1 Halogen, wie Fluor, Chlor, Brom oder Jod,
8.2.2 Alkyl mit 1 bis 3 C-Atomen,
8.2.3 Alkoxy mit 1 bis 3 C-Atomen,
8.2.4 Alkoxy mit 1 bis 3 C-Atomen, ein- oder mehrfach substituiert durch Halogen, wie Fluor, Chlor, Brom oder Jod,
8.3. Carboxy oder
8.4 Hydroxy, steht,
c) mit R⁷ für
1 ) Wasserstoff,
2) Alkyl mit 1 bis 4 C-Atomen,
mit R⁸ für
1) Wasserstoff,
2) Methyl,
mit R³ für
1) einen Rest der Formel III,
-(CH₂)₄-COOR¹⁰
in der R¹⁰ für
1.1 Wasserstoff,
1.2 Alkyl mit 1 bis 4 C-Atomen steht
d) mit R⁷ für
1) Wasserstoff,
2) Alkyl mit 1 bis 4 C-Atomen, steht,
R⁸ und R³ zusammen mit dem Stickstoff, an das sie gebunden sind, einen 4- bis 9-gliedrigen Ring bilden, substituiert durch
2.1. Carbonyl am N-Atom benachbarten C-Atom, oder
R⁸ und R³ zusammen mit Stickstoff, an das sie gebunden sind, einen Piperidinring bilden, der gegebenenfalls substituiert ist durch Alkyl mit 1 bis 3 C-Atomen,
dadurch gekennzeichnet, daß man
a) eine Verbindung der Formel V, 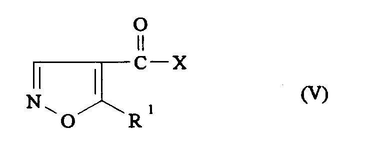 in der X für ein Halogenatom, vorzugsweise Chlor oder Brom, steht und R¹ die in Formel I angegebene Bedeutung hat,
mit dem Amin der Formel VI 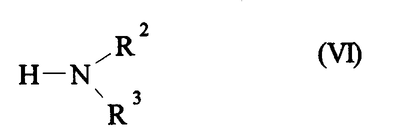 worin R² und R³ die in Formel Ia und Ib angegebene Bedeutung hat, umsetzt, oder
b) eine Verbindung der Formel VI 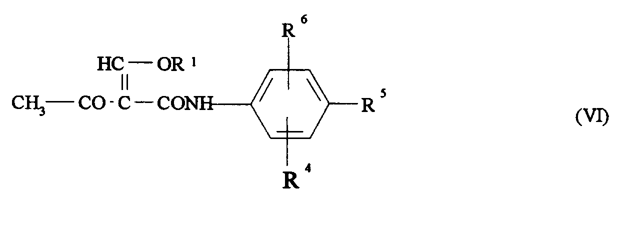 worin R¹ für (C₁-C₄)-Alkyl steht und R⁴, R⁵ und R⁶ die in Formel I angegebene Bedeutung haben, mit einer zweckmäßig mindestens äquimolaren Menge Hydroxylamin in einem organischen Lösungsmittel behandelt, oder
c) eine Verbindung der Formel V,
in der X und R¹ die obengenannte Bedeutung haben, mit einem primären aliphatischen Amin der Formel VII
H₂N - (CH₂)₄ - COOR¹⁰ (VII)
worin R¹⁰, die in Formel I angegebene Bedeutung hat, umsetzt, und
d) die gemäß den Verfahrensschritten a und c hergestellte Verbindung der Formel I in Gegenwart eines basischen Mittels in die entsprechende Verbindung der Formel Ia oder Ib umsetzt.

10. Verfahren gemäß Anspruch 9, dadurch gekennzeichnet, daß eine Verbindung der Formel Ia oder Ib hergestellt wird, wobei die Reste R⁷, R⁸ und R³ unter
a) mit R⁷ für Alkyl mit 2 bis 4 C-Atomen,
mit R⁸ für Wasserstoff,
mit R³ für
1) Phenyl,
2) Phenyl, ein- oder mehrfach substituiert durch
2.1 Halogen, wie Fluor, Chlor, Brom oder Jod,
2.2 Alkyl mit 1 bis 3 C-Atomen, ein- oder mehrfach substituiert durch Halogen, wie Fluor, Chlor, Brom oder Jod steht,
b) mit R⁷ für Alkyl mit 1 bis 4 C-Atomen,
mit R⁸ für Wasserstoff,
mit R³ für
1) Pyridyl,
2) Pyridyl, ein- oder mehrfach substituiert durch
2.1 Halogen, wie Fluor, Chlor, Brom oder Jod,
2.2 Alkyl mit 1 bis 3 C-Atomen,
3) Phenyl, ein- oder mehrfach substituiert durch
3.1 Benzoyl,
3.2 Benzoyl, ein- oder mehrfach subsitutiert durch
3.2.1 Halogen, wie Fluor, Chlor, Brom oder Jod,
3.2.2 Alkyl mit 1 bis 3 C-Atomen,
3.2.3 Alkoxy mit 1 bis 3 C-Atomen,
3.2.4 Alkoxy mit 1 bis 3 C-Atomen, ein- oder mehrfach substituiert durch Halogen, wie Fluor, Chlor, Brom oder Jod, steht,
bedeuten.

11. Verfahren gemäß der Ansprüche 6 bis 10, dadurch gekennzeichnet, daß man die Verbindung N-(4-Chlordifluormethoxy)-phenyl-5-ethylisoxazol-4-carboxamid, N-(4-Methylsulfonylphenyl)-5-methylisoxazol-4-carboxamid, N-(5-Indolyl)-5-methylisoxazol-4-carboxamid, N-(6-Indazolyl)-5-methylisoxazol-4-carboxamid, N-(5-Indazolyl)-5-methylisoxazol-4-carboxamid, N-Allyl-N-phenyl-5-methyl-isoxazol-4-carboxamid, N-(4,6-Dimethyl-2-pyridyl)-5-methylisoxazol-4-carboxamid, N-(4,6-Dimethyl-2-pyrimidinyl)-5-methylisoxazol-4-carboxamid, N-(4-Trifluormethoxyphenyl)-2-cyano-3-hydroxy-crotonsäureamid, N-[4-(1,1,2,2-Tetrafluorethoxy)-phenyl]-2-cyano-3-hydroxycrotonsäureamid, N-[4-(4-Fluorbenzoyl)-phenyl]-2-cyano-3-hydroxycrotonsäureamid oder N-(3,4-Methylendioxyphenyl)-2-cyano-3-hydroxy-4-methyl-crotonsäureamid herstellt.

12. Verwendung von mindestens einer Verbindung der Formel I, Ia oder Ib nach Anspruch 6 bis 11, ihre gegebenenfalls stereoisomeren Formen und/oder mindestens einem von deren physiologisch verträglichen Salzen zur Herstellung von Arzneimitteln zur Behandlung von Rheumaerkrankungen.

13. Verfahren zur Herstellung eines Arzneimittel, dadurch gekennzeichnet, daß man mindestens eine Verbindung der Formel I, Ia oder Ib hergestellt nach Anspruch 6 bis 11, ihre gegebenenfalls stereoisomeren Formen und/oder mindestens einem von deren physiologisch verträglichen Salzen mit einem physiologisch annehmbaren Träger und gegebenenfalls weiteren Zusatz- und/oder Hilfsstoffen in eine geeignete Darreichungsform bringt.

14. Verwendung von mindestens einer Verbindung der Formel I, Ia oder Ib nach Anspruch 6 bis 11, ihre gegebenenfalls stereoisomeren Formen und/oder mindestens einem von deren physiologisch verträglichen Salzen zur Herstellung von Arzneimitteln zur Behandlung von Krebserkrankungen.

15. Verwendung von einer oder mehreren Verbindungen nach Anspruch 14 zur Herstellung von Arzneimitteln zur Behandlung von Leukämie-, Sarkom-, Lymphknotentumor- und Hautkrebserkrankungen.

## Patentansprüche (Patentansprüche für folgende(n) Vertragsstaat(en): ES)

1. Verwendung von mindestens einer Verbindung der Formel I, Ia oder Ib 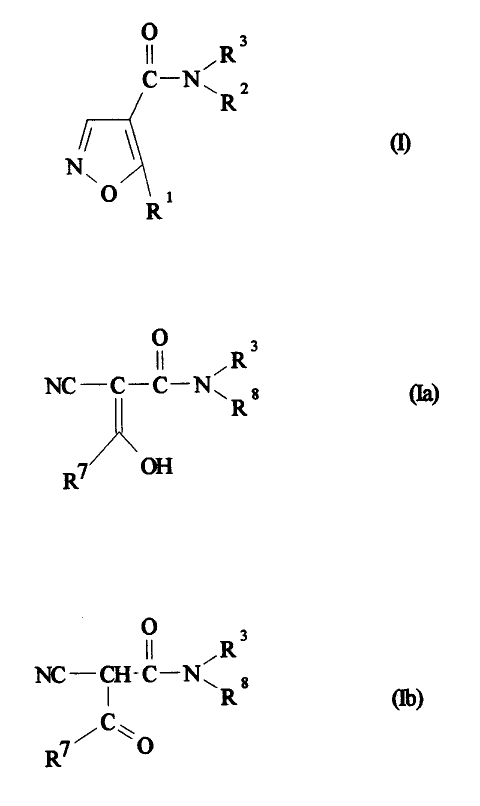 ihre gegebenenfalls stereoisomeren Formen und/oder gegebenenfalls mindestens einem von deren physiologisch verträglichen Salzen, wobei
R¹ für
a) Wasserstoff,
b) Alkyl mit 1 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl,
R² für
a) Wasserstoff,
b) Alkyl mit 1 bis 4 C-Atomen,
c) Phenyl-(C₁-C₂)-alkyl, insbesondere Benzyl,
d) Alkenyl mit 2 bis 3 C-Atomen,
R³ für
a) einen ein-, zwei- oder dreikernigen, ungesättigten heterocyclischen Rest mit 3 bis 13 C-Atomen und 1 bis 4 Heteroatomen aus der Gruppe Sauerstoff, Schwefel und Stickstoff, wovon höchstens eines von Stickstoff verschieden ist, im Ringsystem bedeutet, unsubstituiert oder ein- oder mehrfach substituiert durch
1) Halogen, wie Fluor, Chlor, Brom oder Jod,
2) Alkyl mit 1 bis 3 C-Atomen,
3) Alkyl mit 1 bis 3 C-Atomen ein- oder mehrfach substitutert durch
3.1 Halogen, wie Fluor, Chlor, Brom oder Jod,
4) Alkoxy mit 1 bis 3 C-Atomen,
5) Alkoxy mit 1 bis 3 C-Atomen, ein- oder mehrfach substituiert durch
5.1 Halogen, wie Fluor, Chlor, Brom oder Jod,
6) Nitro,
7) Hydroxy,
8) Carboxy,
9) Carbamoyl,
10) Oxogruppe,
b) einen Rest der Formel II, 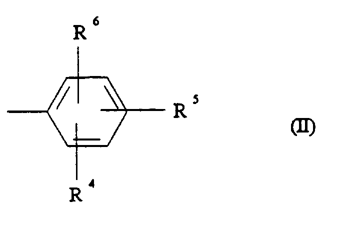 in der R⁴, R⁵ und R⁶ gleich oder verschieden sein können und für
1) Wasserstoff,
2) Phenoxy,
3) Alkyl mit 1 bis 3 C-Atomen, ein- oder mehrfach substituiert durch 3.1 Halogen, wie Fluor, Chlor, Brom oder Jod,
4) in der R⁴ für Wasserstoff und R⁵ und R⁶ gemeinsam mit dem Phenylring der Formel II einen Naphthalinring bilden,
5) in der R⁴ für Wasserstoff und R⁵ und R⁶ einen Methylendioxyrest bilden,
6) Alkoxy mit 1 bis 3 C-Atomen,
7) Alkoxy mit 1 bis 3 C-Atomen, ein- oder mehrfach substituiert durch 7.1 Halogen, wie Fluor, Chlor, Brom oder Jod,
8) (C₁-C₃)-Alkylmercapto,
9) (C₁ -C₃)-Alkylmercapto, ein- oder mehrfach substituiert durch 9.1 Halogen, wie Fluor, Chlor, Brom oder Jod,
10) Halogen, wie Fluor, Chlor, Brom oder Jod,
11) Nitro,
12) Cyano,
13) Hydroxy,
14) Carboxy,
15) (C₁-C₃)-Alkylsulfonyl,
16) Carbalkoxy, mit 1 bis 3 C-Atomen in der Alkylkette,
17) Benzoyl,
18) Benzoyl, ein- oder mehrfach substituiert durch
18.1 Halogen, wie Fluor, Chlor, Brom oder Jod,
18.2 (C₁-C₃)-Alkyl,
18.3 (C₁-C₃)-Alkoxy,
19) Phenyl,
20) Phenyl, ein- oder mehrfach substituiert durch
20.1 (C₁-C₃)-Alkoxy,
20.2 Halogen, wie Fluor, Chlor, Brom oder Jod,
20.3 (C₁-C₃)-Alkyl,
21) Phenoxy, ein- oder mehrfach substituiert durch
21.1 (C₁-C₃)-Alkoxy, ein- oder mehrfach substituiert durch
21.1.1 Halogen, wie Fluor, Chlor, Brom oder Jod,
21.2 Halogen, wie Fluor, Chlor, Brom oder Jod,
21.3 (C₁-C₃)-Alkyl ein- oder mehrfach substituiert durch
21.3.1 Halogen, wie Fluor, Chlor, Brom oder Jod,
c) einen Rest der Formel III,
-(CH₂)ₙ-COOR¹⁰ (III)
in der R¹⁰ für
1) Wasserstoff,
2) Alkyl mit 1 bis 4 C-Atomen,
n für eine ganze Zahl von 1 bis 12,
d) R² und R³ zusammen mit dem Stickstoff, an das sie gebunden sind, einen 4- bis 9-gliedrigen Ring bilden, substituiert durch,
1) Carbonyl am N-Atom benachbarten C-Atom,
e) R² und R³ zusammen mit dem Stickstoff, an das sie gebunden sind, einen 5- bis 6-gliedrigen Ring der Formel IV bilden, 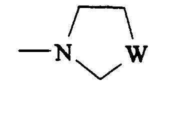 in der W für
1) -CH₂-,
2) -CH₂-CH₂-,
3) 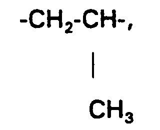
4) 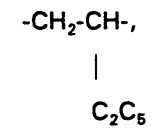
5) 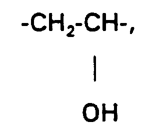
6) -CH₂-O- oder
7) -CH₂-S-
R⁷ für
a) Wasserstoff,
b) Alkyl mit 1 bis 17 C-Atomen,
c) Alkyl mit 1 bis 3 C-Atomen, ein- oder mehrfach substituiert durch Halogen wie Fluor, Chlor, Brom oder Jod,
d) Phenyl-(C₁-C₂)-alkyl, insbesondere Benzyl,
R⁸ für
a) Wasserstoff,
b) Methyl,
c) Alkenyl mit 2 bis 3 C-Atomen,
steht, zur Herstellung von Arzneimitteln zur Behandlung von Krebserkrankungen.

2. Verwendung gemäß Anspruch 1, dadurch gekennzeichnet, daß
R¹ für
a) Wasserstoff,
b) Alkyl mit 1 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl
R² für
a) Wasserstoff,
b) Alkyl mit 1 bis 4 C-Atomen,
c) Phenyl-(C₁-C₂)-alkyl, insbesondere Benzyl,
d) Alkenyl mit 2 bis 3 C-Atomen,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 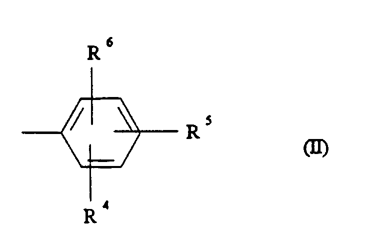 in der R⁴, R⁵ und R⁶ gleich oder verschieden sein können und für
1) Wasserstoff,
2) Phenoxy,
3) Alkyl mit 1 bis 3 C-Atomen, ein- oder mehrfach substituiert durch
3.1 Halogen, wie Fluor, Chlor, Brom oder Jod,
4) in der R⁴ für Wasserstoff und R⁵ und R⁶ einen Methylendioxyrest bilden,
5) Alkoxy mit 1 bis 3 C-Atomen,
6) Alkoxy mit 1 bis 3 C-Atomen, ein- oder mehrfach substituiert durch
6.1 Halogen, wie Fluor, Chlor, Brom oder Jod,
7) (C₁-C₃)-Alkylmercapto,
8) (C₁ -C₃)-Alkylmercapto, ein- oder mehrfach substituiert durch
8.1 Halogen, wie Fluor, Chlor, Brom oder Jod,
9) Halogen, wie Fluor, Chlor, Brom oder Jod,
10) Nitro,
11) Cyano,
12) (C₁-C₃)-Alkylsulfonyl,
13) Benzoyl,
14) Benzoyl, ein- oder mehrfach substituiert durch
14.1 Halogen, wie Fluor, Chlor, Brom oder Jod,
14.2 (C₁-C₃)-Alkyl,
14.3 (C₁-C₃)-Alkoxy,
15) Phenoxy, ein- oder mehrfach substituiert durch
15.1 (C₁-C₃)-Alkoxy, ein- oder mehrfach substituiert durch
15.1.1 Halogen, wie Fluor, Chlor, Brom oder Jod,
15.2 Halogen, wie Fluor, Chlor, Brom oder Jod,
15.3 (C₁-C₃)-Alkyl ein- oder mehrfach substituiert durch
15.3.1 Halogen, wie Fluor, Chlor, Brom oder Jod,
c) einen Rest der Formel III,
-(CH₂)ₙ-COOR¹⁰ (III)
in der R¹⁰ für
1) Wasserstoff,
2) Alkyl mit 1 bis 4 C-Atomen,
n für eine ganze Zahl von 1 bis 12,
d) R² und R³ zusammen mit dem Stickstoff, an das sie gebunden sind, einen 4- bis 9-gliedrigen Ring bilden, substituiert durch,
1) Carbonyl am N-Atom benachbarten C-Atom,
R⁷ für
a) Wasserstoff,
b) Alkyl mit 1 bis 17 C-Atomen,
c) Alkyl mit 1 bis 3 C-Atomen, ein- oder mehrfach substituiert durch Halogen wie Fluor, Chlor, Brom oder Jod,
d) Phenyl-(C₁-C₂)-alkyl, insbesondere Benzyl,
R⁸ für
a) Wasserstoff,
b) Methyl,
c) Alkenyl mit 2 bis 3 C-Atomen,
steht.

3. Verwendung gemäß Anspruch 1, dadurch gekennzeichnet, daß
R¹ für Alkyl mit 1 bis 6 C-Atomen,
R² für Wasserstoff,
R³ für
a) Pyridyl, ein- oder mehrfach substituiert durch Halogen, wie Fluor, Chlor, Brom oder Jod
b) einen Rest, der Formel II, 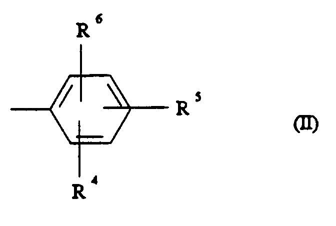 in der R⁴, R⁵ und R⁶ gleich oder verschieden sein können und für
1) Wasserstoff,
2) Phenoxy,
3) Alkyl mit 1 bis 3 C-Atomen, ein- oder mehrfach substituiert durch
3.1 Halogen, wie Fluor, Chlor, Brom oder Jod,
4) in der R⁴ für Wasserstoff und R⁵ und R⁶ einen Methylendioxyrest bilden,
5) Alkoxy mit 1 bis 3 C-Atomen,
6) Alkoxy mit 1 bis 3 C-Atomen, ein- oder mehrfach substituiert durch
6.1 Halogen, wie Fluor, Chlor, Brom oder Jod,
7) Halogen, wie Fluor, Chlor, Brom oder Jod,
8) Nitro,
9) Benzoyl,
10) Benzoyl, ein- oder mehrfach substituiert durch
10.1 Halogen, wie Fluor, Chlor, Brom oder Jod,
10.2 (C₁-C₃)-Alkyl,
10.3 (C₁-C₃)-Alkoxy,
11) Phenoxy, ein- oder mehrfach substituiert durch
11.1 (C₁-C₃)-Alkoxy, ein- oder mehrfach substituiert durch
11.1.1 Halogen, wie Fluor, Chlor, Brom oder Jod,
11.2 Halogen, wie Fluor, Chlor, Brom oder Jod,
11.3 (C₁-C₃)-Alkyl ein- oder mehrfach substituiert durch
11.3.1 Halogen, wie Fluor, Chlor, Brom oder Jod,
R⁷ für Alkyl mit 1 bis 17 C-Atomen,
R⁸ für Wasserstoff
steht.

4. Verwendung von Methylisoxazol-4-carbonsäure-(4-trifluormethyl)-anilid oder N-(4-Trifluormethylphenyl)-2-cyano-3-hydroxy-crotonsäureamid zur Herstellung von Arzneimitteln zur Behandlung von Krebserkrankungen.

5. Verwendung von einer oder mehreren der Verbindungen nach Anspruch 1 zur Herstellung von Arzneimitteln zur Behandlung von Leukämie-, Sarkom-, Lymphknotentumor- oder Hautkrebserkrankungen.

6. Verfahren zur Herstellung der Verbindung der Formel I 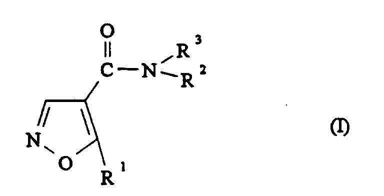 ihre gegebenenfalls stereoisomeren Formen und/oder gegebenenfalls ihre physiologisch verträglichen Salze, wobei
R¹ für
a) Wasserstoff,
b) Alkyl mit 2 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl
R² für
a) Wasserstoff,
b) Alkenyl mit 2 bis 3 C-Atomen,
c) Benzyl,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 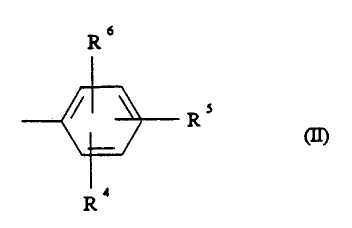 in der R⁴, R⁵ und R⁶ gleich oder verschieden sein können und für
1) Halogen, wie Fluor, Chlor, Brom oder Jod,
2) Nitro,
3) Wasserstoff,
4) Benzoyl, ein- oder mehrfach substituiert durch
4.1 Halogen, wie Fluor, Chlor, Brom oder Jod,
4.2 Methyl,
4.3 Methoxy,
4.4 Benzoyl,
5) (C₁-C₃)-Alkoxy, ein- oder mehrfach substituiert durch,
5.1 Halogen, wie Fluor, Chlor, Brom oder Jod
6) (C₁ -C₃)-Alkyl, ein- oder mehrfach substituiert durch
6.1 Halogen, wie Fluor, Chlor, Brom oder Jod,
7) Hydroxy,
8) Alkylsulfonyl, mit 1 bis 3 C-Atomen in der Alkylkette,
9) in der R⁴ für Wasserstoff und R⁵ und R⁶ gemeinsam einen Methylendioxyrest bilden,
10) Cyano,
11) (C₁-C₃)-Alkylmercapto,
12) Benzoyl,
c) Pyrimidinyl, ein- oder mehrfach substituiert durch
1) Alkyl mit 1 bis 3 C-Atomen,
d) Indolyl oder
e) Indazolinyl
steht, dadurch gekennzeichnet, daß man
a) eine Verbindung der Formel V, 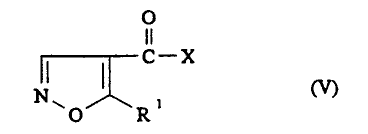 in der X für ein Halogenatom, vorzugsweise Chlor oder Brom, steht und R¹ die in Formel I angegebene Bedeutung hat,
mit dem Amin der Formel VI 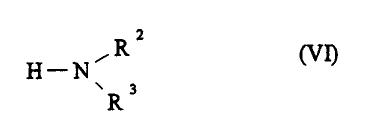 worin R² und R³ die in Formel I angegebene Bedeutung hat, umsetzt.

7. Verfahren gemäß Anspruch 6, dadurch gekennzeichnet, daß man eine Verbindung der Formel I herstellt, wobei
R¹ für
a) Wasserstoff,
b) Alkyl mit 2 bis 6 C-Atomen,
c) Alkyl mit 1 bis 4 C-Atomen, ein- oder mehrfach substituiert durch
1) Halogen, wie Fluor, Chlor, Brom oder Jod,
d) Phenyl,
R² für
a) Wasserstoff,
b) Alkenyl mit 2 bis 3 C-Atomen,
c) Benzyl,
R³ für
a) Pyridyl ein- oder mehrfach substituiert durch
1) Wasserstoff,
2) Halogen, wie Fluor, Chlor, Brom oder Jod,
3) Nitro,
4) Alkyl mit 1 bis 3 C-Atomen,
5) Alkoxy mit 1 bis 3 C-Atomen,
b) einen Rest der Formel II, 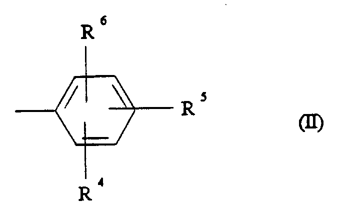 in der R⁴, R⁵ und R⁶ gleich oder verschieden sein können und für
1) Halogen, wie Fluor, Chlor, Brom oder Jod,
2) Nitro,
3) Wasserstoff,
4) Benzoyl, ein- oder mehrfach substituiert durch
4.1 Halogen, wie Fluor, Chlor, Brom oder Jod,
4.2 Methyl,
4.3 Methoxy,
4.4 Benzoyl,
5) (C₁-C₃)-Alkoxy, ein- oder mehrfach substituiert durch Halogen wie Fluor, Chlor, Brom oder Jod
6) (C₁ -C₃)-Alkyl, ein- oder mehrfach substituiert durch Halogen wie Fluor, Chlor, Brom oder Jod,
7) Hydroxy,
8) Alkylsulfonyl, mit 1 bis 3 C-Atomen in der Alkylkette,
9) in der R⁴ für Wasserstoff und R⁵ und R⁶ gemeinsam einen Methylendioxyrest bilden,
10) Cyano,
11) (C₁-C₃)-Alkylmercapto,
12) Benzoyl,
c) Pyrimidinyl, ein- oder mehrfach substituiert durch Alkyl mit 1 bis 3 C-Atomen,
d) Indolyl oder
e) Indazolinyl steht.

8. Verfahren gemäß Anspruch 6, dadurch gekennzeichnet, daß, man eine Verbindung der Formel I herstellt, wobei
R¹ für Alkyl mit 2 bis 6 C-Atomen,
R² für Wasserstoff,
R³ für
a) Pyridyl, ein- oder mehrfach substituiert durch
1) Wasserstoff,
2) Halogen, wie Fluor, Chlor, Brom oder Jod,
b) einen Rest der Formel II, 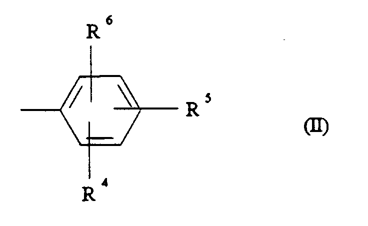 in der R⁴, R⁵ und R⁶ gleich oder verschieden sein können und für
1) Halogen, wie Fluor, Chlor, Brom oder Jod,
2) Nitro,
3) Benzoyl,
4) Benzoyl, ein- oder mehrfach substituiert durch
4.1 Halogen, wie Fluor, Chlor, Brom oder Jod,
4.2 Methyl,
5) (C₁-C₃)-Alkoxy, ein- oder mehrfach substituiert durch Halogen, wie Fluor, Chlor, Brom oder Jod
6) (C₁ -C₃)-Alkyl, ein- oder mehrfach substituiert durch Halogen wie Fluor, Chlor, Brom oder Jod,
7) Wasserstoff,
8) in der R⁴ für Wasserstoff und R⁵ und R⁶ gemeinsam einen Methylendioxyrest bilden, steht.

9. Verfahren zur Herstellung der Verbindung der Formel Ia oder der Formel Ib, 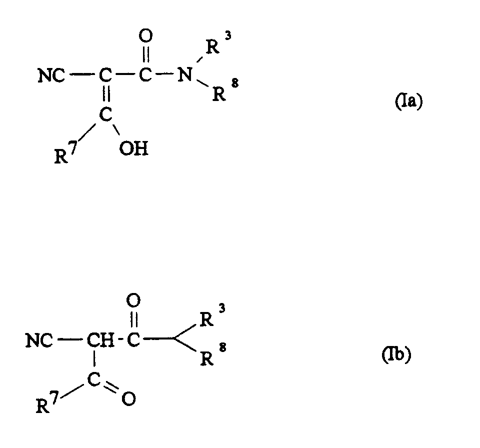 ihre gegebenenfalls stereoisomeren Formen und/oder gegebenenfalls ihre physiologisch verträglichen Salze, wobei die Reste R⁷, R⁸ und R⁹ unter
a) mit R⁷ für
1) Wasserstoff,
2) Alkyl mit 2 bis 4 C-Atomen,
mit R⁸ für
1) Wasserstoff,
2) Methyl,
mit R⁹ für
1) Phenyl,
2) Phenyl, ein- oder mehrfach substituiert durch
2.1 Halogen,
2.2. Alkyl mit 1 bis 3 C-Atomen, ein- oder mehrfach substituiert durch Halogen, wie Fluor, Chlor, Brom oder Jod, steht,
b) mit R⁷ für
1) Alkyl mit 1 bis 4 C-Atomen,
2) Wasserstoff,
3) CF₃,
mit R⁸ für
1) Wasserstoff,
2) Methyl,
3) Alkenyl mit 2 bis 3 C-Atomen,
mit R³ für
1) Pyridyl,
2) Pyridyl, ein- oder mehrfach substituiert durch
2.1 Halogen, wie Fluor, Chlor, Brom oder Jod,
2.2 Alkyl mit 1 bis 3 C-Atomen,
3) Pyrimidinyl, substituiert wie bei 2),
4) Thiazolyl, substituiert wie bei 2) und
4.1 Alkoxycarbonyl, mit 1 bis 3 C-Atomen in der Alkylkette,
5) Benzothiazolyl, substituiert wie bei 2),
6) Benzimidazolyl, substituiert wie bei 2),
7) Indazolyl, substituiert wie bei 2),
8) Phenyl, ein- oder mehrfach substituiert durch
8.1 Benzoyl,
8.2 Benzoyl, ein- oder mehrfach substituiert durch
8.2.1 Halogen, wie Fluor, Chlor, Brom oder Jod,
8.2.2 Alkyl mit 1 bis 3 C-Atomen,
8.2.3 Alkoxy mit 1 bis 3 C-Atomen,
8.2.4 Alkoxy mit 1 bis 3 C-Atomen, ein- oder mehrfach substituiert durch Halogen, wie Fluor, Chlor, Brom oder Jod,
8.3. Carboxy oder
8.4 Hydroxy, steht,
c) mit R⁷ für
1) Wasserstoff,
2) Alkyl mit 1 bis 4 C-Atomen,
mit R⁸ für
1) Wasserstoff,
2) Methyl,
mit R³ für
1) einen Rest der Formel III,
-(CH₂)₄-COOR¹⁰
in der R¹⁰ für
1.1 Wasserstoff,
1.2 Alkyl mit 1 bis 4 C-Atomen steht
d) mit R⁷ für
1) Wasserstoff,
2) Alkyl mit 1 bis 4 C-Atomen, steht,
R⁸ und R³ zusammen mit dem Stickstoff, an das sie gebunden sind, einen 4- bis 9-gliedrigen Ring bilden, substituiert durch
2.1. Carbonyl am N-Atom benachbarten C-Atom, oder
R⁸ und R³ zusammen mit Stickstoff, an das sie gebunden sind, einen Piperidinring bilden, der gegebenenfalls substituiert ist durch Alkyl mit 1 bis 3 C-Atomen,
dadurch gekennzeichnet, daß man
a) eine Verbindung der Formel V, 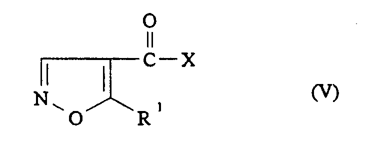 in der X für ein Halogenatom, vorzugsweise Chlor oder Brom, steht und R¹ die in Formel I angegebene Bedeutung hat,
mit dem Amin der Formel VI 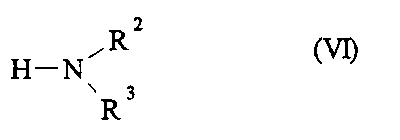 worin R² und R³ die in Formel Ia und Ib angegebene Bedeutung hat, umsetzt, oder
b) eine Verbindung der Formel VI 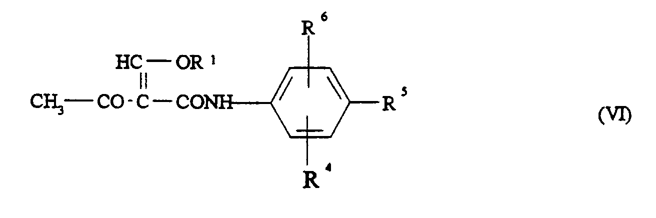 worin R¹ für (C₁-C₄)-Alkyl steht und R⁴, R⁵ und R⁶ die in Formel I angegebene Bedeutung haben, mit einer zweckmäßig mindestens äquimolaren Menge Hydroxylamin in einem organischen Lösungsmittel behandelt, oder
c) eine Verbindung der Formel V,
in der X und R¹ die obengenannte Bedeutung haben, mit einem primären aliphatischen Amin der Formel VII
H₂N - (CH₂)₄ - COOR¹⁰ (VII)
worin R¹⁰, die in Formel I angegebene Bedeutung hat, umsetzt, und
d) die gemäß den Verfahrensschritten a und c hergestellte Verbindung der Formel I in Gegenwart eines basischen Mittels in die entsprechende Verbindung der Formel Ia oder Ib umsetzt.

10. Verfahren gemäß Anspruch 9, dadurch gekennzeichnet, daß eine Verbindung der Formel Ia oder Ib hergestellt wird, wobei die Reste R⁷, R⁸ und R³ unter
a) mit R⁷ für Alkyl mit 2 bis 4 C-Atomen,
mit R⁸ für Wasserstoff,
mit R³ für
1) Phenyl,
2) Phenyl, ein- oder mehrfach substituiert durch
2.1 Halogen, wie Fluor, Chlor, Brom oder Jod,
2.2 Alkyl mit 1 bis 3 C-Atomen, ein- oder mehrfach substituiert durch Halogen, wie Fluor, Chlor, Brom oder Jod steht,
b) mit R⁷ für Alkyl mit 1 bis 4 C-Atomen,
mit R⁸ für Wasserstoff,
mit R³ für
1) Pyridyl,
2) Pyridyl, ein- oder mehrfach substituiert durch
2.1 Halogen, wie Fluor, Chlor, Brom oder Jod,
2.2 Alkyl mit 1 bis 3 C-Atomen,
3) Phenyl, ein- oder mehrfach substituiert durch
3.1 Benzoyl,
3.2 Benzoyl, ein- oder mehrfach subsitutiert durch
3.2.1 Halogen, wie Fluor, Chlor, Brom oder Jod,
3.2.2 Alkyl mit 1 bis 3 C-Atomen,
3.2.3 Alkoxy mit 1 bis 3 C-Atomen,
3.2.4 Alkoxy mit 1 bis 3 C-Atomen, ein- oder mehrfach substituiert durch Halogen, wie Fluor, Chlor, Brom oder Jod, steht,
bedeuten.

11. Verfahren gemäß der Ansprüche 6 bis 10, dadurch gekennzeichnet, daß man die Verbindung N-(4-Chlordifluormethoxy)-phenyl-5-ethylisoxazol-4-carboxamid, N-(4-Methylsulfonylphenyl)-5-methylisoxazol-4-carboxamid, N-(5-Indolyl)-5-methylisoxazol-4-carboxamid, N-(6-Indazolyl)-5-methylisoxazol-4-carboxamid, N-(5-Indazolyl)-5-methylisoxazol-4-carboxamid, N-Allyl-N-phenyl-5-methyl-isoxazol-4-carboxamid, N-(4,6-Dimethyl-2-pyridyl)-5-methylisoxazol-4-carboxamid, N-(4,6-Dimethyl-2-pyrimidinyl)-5-methylisoxazol-4-carboxamid, N-(4-Trifluormethoxyphenyl)-2-cyano-3-hydroxy-crotonsäureamid, N-[4-(1,1,2,2-Tetrafluorethoxy)-phenyl]-2-cyano-3-hydroxycrotonsäureamid, N-[4-(4-Fluorbenzoyl)-phenyl]-2-cyano-3-hydroxycrotonsäureamid oder N-(3,4-Methylendioxyphenyl)-2-cyano-3-hydroxy-4-methyl-crotonsäureamid herstellt.

12. Verwendung von mindestens einer Verbindung der Formel I, Ia oder Ib nach Anspruch 6 bis 11, ihre gegebenenfalls stereoisomeren Formen und/oder mindestens einem von deren physiologisch verträglichen Salzen zur Herstellung von Arzneimitteln zur Behandlung von Rheumaerkrankungen.

13. Verwendung von mindestens einer Verbindung der Formel I, Ia oder Ib nach Anspruch 6 bis 11, ihre gegebenenfalls stereoisomeren Formen und/oder mindestens einem von deren physiologisch verträglichen Salzen zur Herstellung von Arzneimitteln zur Behandlung von Krebserkrankungen.

14. Verwendung von einer oder mehreren Verbindungen nach Anspruch 13 zur Herstellung von Arzneimitteln zur Behandlung von Leukämie-, Sarkom-, Lymphknotentumor- und Hautkrebserkrankungen.

## Claims (Claims for the following Contracting State(s): AT, BE, CH, DE, DK, FR, GB, IT, LI, LU, NL, SE)

1. The use of at least one compound of the formula I, Ia or Ib 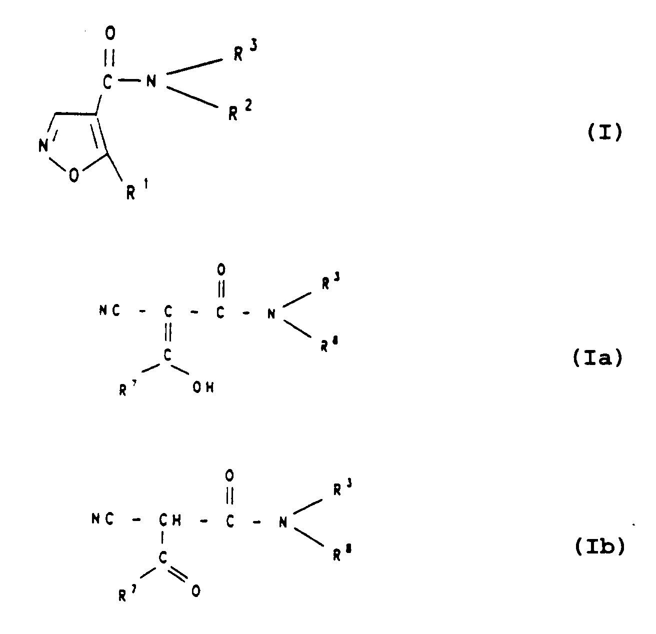 its possible stereoisomeric forms and/or, if appropriate, at least one of its physiologically tolerable salts, where
R¹ is
a) hydrogen
b) alkyl having 1 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkyl having 1 to 4 carbon atoms,
c) phenyl-(C₁-C₂)-alkyl, in particular benzyl,
d) alkenyl having 2 to 3 carbon atoms,
R³ is
a) a mono-, di- or trinuclear, unsaturated heterocyclic radical having 3 to 13 carbon atoms and 1 to 4 heteroatoms from the group comprising oxygen, sulfur and nitrogen, of which a maximum of one is different from nitrogen, in the ring system, unsubstituted or mono- or polysubstituted by
1) halogen, such as fluorine, chlorine, bromine or iodine,
2) alkyl having 1 to 3 carbon atoms,
3) alkyl having 1 to 3 carbon atoms, mono- or polysubstituted by
3.1 halogen, such as fluorine, chlorine, bromine or iodine,
4) alkoxy having 1 to 3 carbon atoms,
5) alkoxy having 1 to 3 carbon atoms, mono- or polysubstituted by
5.1 halogen, such as fluorine, chlorine, bromine or iodine,
6) nitro,
7) hydroxyl,
8) carboxyl,
9) carbamoyl,
10) an oxo group,
b) a radical of the formula II, 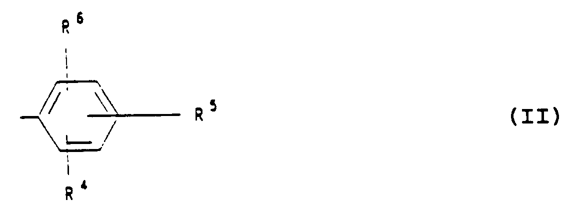 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶, together with the phenyl ring of the formula II, form a naphthalene ring,
5) in which R⁴ is hydrogen and R⁵ and R⁶ form a methylenedioxy radical,
6) alkoxy having 1 to 3 carbon atoms,
7) alkoxy having 1 to 3 carbon atoms, mono- or polysubstituted by
7.1 halogen, such as fluorine, chlorine, bromine or iodine,
8) (C₁-C₃)-alkylmercapto,
9) (C₁-C₃)-alkylmercapto, mono- or polysubstituted by
9.1 halogen, such as fluorine, chlorine, bromine or iodine,
10) halogen, such as fluorine, chlorine, bromine or iodine,
11) nitro,
12) cyano,
13) hydroxyl,
14) carboxyl,
15) (C₁-C₃)-alkylsulfonyl,
16) carbalkoxy, having 1 to 3 carbon atoms in the alkyl chain,
17) benzoyl,
18) benzoyl, mono- or polysubstituted by
18.1 halogen, such as fluorine, chlorine, bromine or iodine,
18.2 (C₁-C₃)-alkyl,
18.3 (C₁-C₃)-alkoxy,
19) phenyl,
20) phenyl, mono- or polysubstituted by
20.1 (C₁-C₃)-alkoxy,
20.2 halogen, such as fluorine, chlorine, bromine or iodine,
20.3 (C₁-C₃)-alkyl,
21) phenoxy, mono- or polysubstituted by
21.1 (C₁-C₃)-alkoxy, mono- or polysubstituted by
21.1.1 halogen, such as fluorine, chlorine, bromine or iodine,
21.2 halogen, such as fluorine, chlorine, bromine or iodine,
21.3 (C₁-C₃)-alkyl, mono- or polysubstituted by
21.3.1 halogen, such as fluorine, chlorine, bromine or iodine,
c) a radical of the formula III,
-(CH₂)ₙ-COOR¹⁰ (III)
in which R¹⁰ is
1) hydrogen
2) alkyl having 1 to 4 carbon atoms,
n is an integer from 1 to 12,
d) R² and R³, together with the nitrogen to which they are bonded, form a 4- to 9-membered ring, substituted by
1) carbonyl on the carbon atom adjacent to the nitrogen atom,
e) R² and R³, together with the nitrogen to which they are bonded, form a 5- to 6-membered ring of the formula IV, 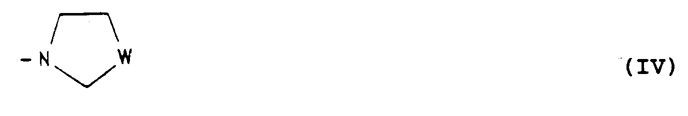 in which W is
1) -CH₂-,
2) -CH₂-CH₂-,
3) 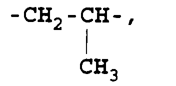
4) 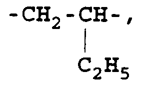
5) 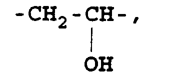
6) -CH₂-O- or
7) -CH₂-S-
R⁷ is
a) hydrogen,
b) alkyl having 1 to 17 carbon atoms,
c) alkyl having 1 to 3 carbon atoms, mono- or polysubstituted by halogen such as fluorine, chlorine, bromine or iodine,
d) phenyl-(C₁-C₂)-alkyl, in particular benzyl,
R⁸ is
a) hydrogen,
b) methyl,
c) alkenyl having 2 to 3 carbon atoms, for the production of pharmaceuticals for the treatment of carcinoses.

2. The use as claimed in claim 1, wherein
R¹ is
a) hydrogen,
b) alkyl having 1 to 6 carbon atoms,
c) alkyl having 1 to 4 carbon atoms, mono- or poly-substituted by
1) halogen, such as fluorine, chlorine, bromine or iodine,
d) phenyl,
R² is
a) hydrogen,
b) alkyl having 1 to 4 carbon atoms,
c) phenyl-(C₁-C₂)alkyl, in particular benzyl,
d) alkenyl having 2 to 3 carbon atoms,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 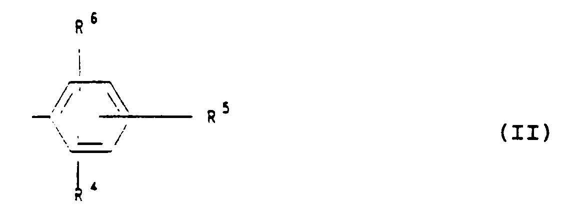 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶ form a methylenedioxy radical,
5) alkoxy having 1 to 3 carbon atoms,
6) alkoxy having 1 to 3 carbon atoms, mono- or polysubstituted by
6.1 halogen, such as fluorine, chlorine, bromine or iodine,
7) (C₁-C₃)-alkylmercapto,
8) (C₁-C₃)-alkylmercapto, mono- or polysubstituted by
8.1 halogen, such as fluorine, chlorine, bromine or iodine,
9) halogen, such as fluorine, chlorine, bromine or iodine,
10) nitro,
11) cyano,
12) (C₁-C₃)-alkylsulfonyl,
13) benzoyl,
14) benzoyl, mono- or polysubstituted by
14.1 halogen, such as fluorine, chlorine, bromine or iodine,
14.2 (C₁-C₃)-alkyl,
14.3 (C₁-C₃)-alkoxy,
15) phenoxy, mono- or polysubstituted by
15.1 (C₁-C₃)-alkoxy, mono- or polysubstituted by
15.1.1 halogen, such as fluorine, chlorine, bromine or iodine,
15.2 halogen, such as fluorine, chlorine, bromine or iodine,
15.3 (C₁-C₃)-alkyl, mono- or polysubstituted by
15.3.1 halogen, such as fluorine, chlorine, bromine or iodine,
c) a radical of the formula III,
-(CH₂)ₙ-COOR¹⁰ (III)
in which R¹⁰ is
1) hydrogen,
2) alkyl having 1 to 4 carbon atoms,
n is an integer from 1 to 12,
d) R² and R³, together with the nitrogen to which they are bonded, form a 4- to 9-membered ring, substituted by
1) carbonyl on the carbon atom adjacent to the nitrogen atom,
R⁷ is
a) hydrogen,
b) alkyl having 1 to 17 carbon atoms
c) alkyl having 1 to 3 carbon atoms, mono- or polysubstituted by halogen, such as fluorine, chlorine, bromine or iodine,
d) phenyl-(C₁-C₂)-alkyl, in particular benzyl,
R⁸ is
a) hydrogen,
b) methyl,
c) alkenyl having 2 to 3 carbon atoms.

3. The use as claimed in claim 1, wherein
R¹ is alkyl having 1 to 6 carbon atoms,
R² is hydrogen,
R³ is
a) pyridyl, mono- or polysubstituted by halogen, such as fluorine, chlorine, bromino or iodine,
b) a radical of the formula II, 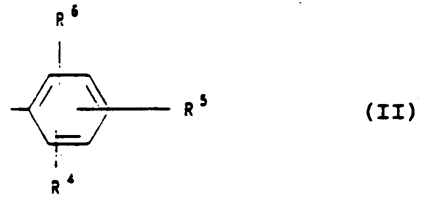 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶ form a methylenedioxy radical,
5) alkoxy having 1 to 3 carbon atoms,
6) alkoxy having 1 to 3 carbon atoms, mono- or polysubstituted by
6.1 halogen, such as fluorine, chlorine, bromine or iodine,
7) halogen, such as fluorine, chlorine, bromine or iodine,
8) nitro,
9) benzoyl,
10) benzoyl, mono- or poly-substituted by
10.1 halogen, such as fluorine, chlorine, bromine or iodine,
10.2 (C₁-C₃)-alkyl,
10.3 (C₁-C₃)-alkoxy,
11) phenoxy, mono- or polysubstituted by
11.1 (C₁-C₃)-alkoxy, mono- or polysubstituted by
11.1.1 halogen, such as fluorine, chlorine, bromine or iodine,
11.2 halogen, such as fluorine, chlorine, bromine or iodine,
11.3 (C₁-C₃)-alkyl, mono- or polysubstituted by
11.3.1 halogen, such as fluorine, chlorine, bromine or iodine,
R⁷ is alkyl having 1 to 17 carbon atoms,
R⁸ is hydrogen.

4. The use of methylisoxazole-4-carboxylic acid-(4-trifluoromethyl)anilide or N-(4-trifluoromethylphenyl)-2-cyano-3-hydroxycrotonamide for the production of pharmaceuticals for the treatment of carcinoses.

5. A process for the production of a pharmaceutical for the treatment of carcinoses, which comprises bringing at least one compound of the formula I, Ia or Ib as claimed in claim 1, its possible stereoisomeric forms and/or at least one of its physiologically tolerable salts into a suitable form for adminstration with a physiologically acceptable excipient and, if desired, other additives and/or auxiliaries.

6. The use of one or more of the compounds as claimed in claim 1 for the production of pharmaceuticals for the treatment of leukemias, sarcomas, lymph node tumors or skin carcinoses.

7. A compound of the formula I 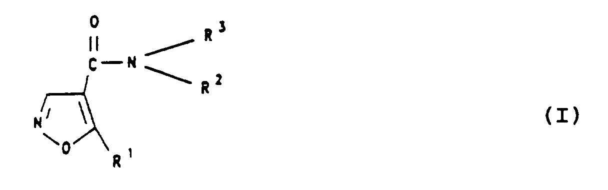 its possible stereoisomeric forms and/or, if appropriate, its physiologically tolerable salts, where
R¹ is
a) hydrogen,
b) alkyl having 2 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkenyl having 2 to 3 carbon atoms,
c) benzyl,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 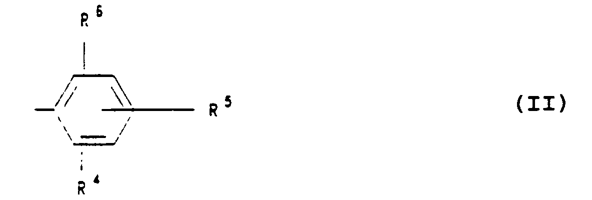 in which R⁴, R⁵ and R⁶ can be identical or different and are
1) halogen, such as fluorine, chlorine, bromine or iodine,
2) nitro,
3) hydrogen,
4) benzoyl, mono- or polysubstituted by
4.1 halogen, such as fluorine, chlorine, bromine or iodine,
4.2 methyl,
4.3 methoxy,
4.4 benzoyl,
5) (C₁-C₃)-alkoxy, mono- or polysubstituted by
5.1 halogen, such as fluorine, chlorine, bromine or iodine,
6) (C₁-C₃)-alkyl, mono- or polysubstituted by
6.1 halogen, such as fluorine, chlorine, bromine or iodine,
7) hydroxyl,
8) alkylsulfonyl, having 1 to 3 carbon atoms in the alkyl chain,
9) in which R⁴ is hydrogen and R⁵ and R⁶ together form a methylenedioxy radical,
10) cyano,
11) (C₁-C₃)-alkylmercapto,
12) benzoyl,
c) pyrimidinyl, mono- or polysubstituted by
1) alkyl having 1 to 3 carbon atoms,
d) indolyl or
e) indazolinyl.

8. A compound as claimed in claim 8, wherein
R¹ is
a) hydrogen,
b) alkyl having 2 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkenyl having 2 to 3 carbon atoms,
c) benzyl,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 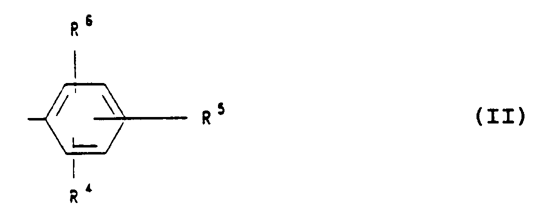 in which R⁴, R⁵ and R⁶ can be identical or different and are
1) halogen, such as fluorine, chlorine, bromine or iodine,
2) nitro,
3) hydrogen,
4) benzoyl, mono- or polysubstituted by
4.1 halogen, such as fluorine, chlorine, bromine or iodine,
4.2 methyl,
4.3 methoxy,
4.4 benzoyl,
5) (C₁-C₃)-alkoxy, mono- or polysubstituted by
5.1 halogen, such as fluorine, chlorine, bromine or iodine,
6) (C₁-C₃)-alkyl, mono- or polysubstituted by
6.1 halogen, such as fluorine, chlorine, bromine or iodine,
7) hydroxyl,
8) alkylsulfonyl, having 1 to 3 carbon atoms in the alkyl chain,
9) in which R⁴ is hydrogen and R⁵ and R⁶ together form a methylenedioxy radical,
10) cyano,
11) (C₁-C₃)-alkylmercapto,
12) benzoyl,
c) pyrimidinyl, mono- or polysubstituted by
1) alkyl having 1 to 3 carbon atoms,
d) indolyl or
e) indazolinyl.

9. A compound as claimed in claim 7, wherein
R¹ is alkyl having 2 to 6 carbon atoms,
R² is hydrogen,
R³ is
a) pyridyl, mono- or polysubstituted by
1) hydrogen,
2) halogen, such as fluorine, chlorine, bromine or iodine,
b) a radical of the formula II, 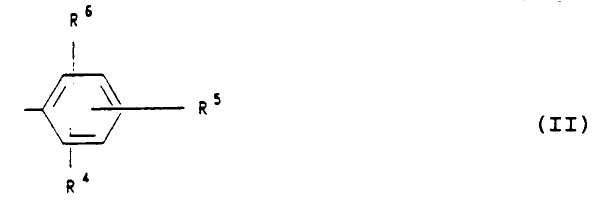 in which R⁴, R⁵ and R⁶ can be identical or different and are
1) halogen, such as fluorine, chlorine, bromine or iodine,
2) nitro,
3) benzoyl,
4) benzoyl, mono- or polysubstituted by
4.1 halogen, such as fluorine, chlorine, bromine or iodine,
4.2 methyl,
5) (C₁-C₃)-alkoxy, mono- or polysubstituted by
5.1 halogen, such as fluorine, chlorine, bromine or iodine,
6) (C₁-C₃)-alkyl, mono- or polysubstituted by
6.1 halogen, such as fluorine, chlorine, bromine or iodine,
7) hydrogen,
8) in which R⁴ is hydrogen and R⁵ and R⁶ together form a methylenedioxy radical.

10. A compound of the formula Ia or the formula Ib 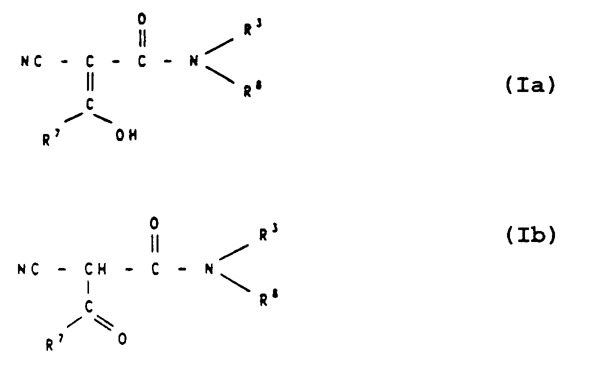 its possible stereoisomeric forms and/or, if appropriate, its physiologically tolerable salts, where the radicals R⁷, R⁸ and R³ are the following
a) where R⁷ is
1) hydrogen,
2) alkyl having 2 to 4 carbon atoms,
where R⁸ is
1) hydrogen,
2) methyl,
where R³ is
1) phenyl,
2) phenyl, mono- or polysubstituted by
2.1 halogen,
2.2 alkyl having 1 to 3 carbon atoms, mono- or polysubstituted by halogen, such as fluorine, chlorine, bromine or iodine,
b) where R⁷ is
1) alkyl having 1 to 4 carbon atoms,
2) hydrogen,
3) CF₃,
where R⁸ is
1) hydrogen,
2) methyl,
3) alkenyl having 2 to 3 carbon atoms,
where R³ is
1) pyridyl,
2) pyridyl, mono- or polysubstituted by
2.1 halogen, such as fluorine, chlorine, bromine or iodine,
2.2 alkyl having 1 to 3 carbon atoms,
3) pyrimidinyl, substituted as for 2),
4) thiazolyl, substituted as for 2) and
4.1 alkoxycarbonyl, having 1 to 3 carbon atoms in the alkyl chain,
5) benzothiazolyl, substituted as for 2),
6) benzimidazolyl, substituted as for 2),
7) indazolyl, substituted as for 2),
8) phenyl, mono- or polysubstituted by
8.1 benzoyl,
8.2 benzoyl, mono- or polysubstituted by
8.2.1 halogen, such as fluorine, chlorine, bromine or iodine,
8.2.2 alkyl having 1 to 3 carbon atoms,
8.2.3 alkoxy having 1 to 3 carbon atoms,
8.2.4 alkoxy having 1 to 3 carbon atoms, mono- or polysubstituted by halogen, such as fluorine, chlorine, bromine or iodine,
8.3 carboxyl or
8.4 hydroxyl,
c) where R⁷ is
1) hydrogen,
2) alkyl having 1 to 4 carbon atoms,
where R⁸ is
1) hydrogen,
2) methyl,
where R³ is
1) a radical of the formula III,
-(CH₂)₄-COOR¹⁰
in which R¹⁰ is
1.1 hydrogen
1.2 alkyl having 1 to 4 carbon atoms,
d) where R⁷ is
1) hydrogen,
2) alkyl having 1 to 4 carbon atoms,
R⁸ and R³, together with the nitrogen to which they are bonded, form a 4- to 9-membered ring, substituted by
2.1 carbonyl on the carbon atom adjacent to the nitrogen atom, or
R⁸ and R³, together with nitrogen to which they are bonded, form a piperidine ring optionally substituted by alkyl having 1 to 3 carbon atoms.

11. A compound as claimed in claim 10, wherein the radicals R⁷, R⁸ and R³ are the following
a) where R⁷ is alkyl having 2 to 4 carbon atoms,
where R⁸ is hydrogen,
where R³ is
1) phenyl,
2) phenyl, mono- or polysubstituted by
2.1 halogen, such as fluorine, chlorine, bromine or iodine,
2.2 alkyl having 1 to 3 carbon atoms, mono- or polysubstituted by halogen, such as fluorine, chlorine, bromine or iodine,
b) where R⁷ is alkyl having 1 to 4 carbon atoms,
where R⁸ is hydrogen,
where R³ is
1) pyridyl,
2) pyridyl, mono- or polysubstituted by
2.1 halogen, such as fluorine, chlorine, bromine or iodine,
2.2 alkyl having 1 to 3 carbon atoms,
3) phenyl, mono- or polysubstituted by
3.1 benzoyl,
3.2 benzoyl, mono- or polysubstituted by
3.2.1 halogen, such as fluorine, chlorine, bromine or iodine,
3.2.2 alkyl having 1 to 3 carbon atoms,
3.2.3 alkoxy having 1 to 3 carbon atoms,
3.2.4 alkoxy having 1 to 3 carbon atoms, mono- or polysubstituted by halogen, such as fluorine, chlorine, bromine or iodine.

12. The compound N-(4-chlorodifluoromethoxy)phenyl-5-ethylisoxazole-4-carboxamide, N-(4-methylsulfonylphenyl)-5-methylisoxazole-4-carboxamide, N-(5-indolyl)-5-methylisoxazole-4-carboxamide, N-(6-indazolyl)-5-methylisoxazole-4-carboxamide, N-(5-indazolyl)-5-methylisoxazole-4-carboxamide, N-allyl-N-phenyl-5-methylisoxazole-4-carboxamide, N-(4,6-dimethyl-2-pyridyl)-5-methylisoxazole-4-carboxamide, N-(4,6-dimethyl-2-pyrimidinyl)-5-methylisoxazole-4-carboxamide, N-(4-trifluoromethoxyphenyl)-2-cyano-3-hydroxycrotonamide, N-[4-(1,1,2,2-tetrafluoroethoxy)-phenyl]-2-cyano-3-hydroxycrotonamide, N-[4-(4-fluorobenzoyl)-phenyl]-2-cyano-3-hydroxycrotonamide or N-(3,4-methylenedioxyphenyl)-2-cyano-3-hydroxy-4-methylcrotonamide.

13. The use of at least one compound of the formula I, Ia or Ib as claimed in claims 7 to 12, its possible stereoisomeric forms and/or at least one of its physiologically tolerable salts for the production of pharmaceuticals for the treatment of rheumatic disorders.

14. A pharmaceutical which contains - or consists of - at least one compound of the formula I, Ia or Ib as claimed in claims 7 to 12, its possible stereoisomeric forms and/or at least one of its physiologically tolerable salts.

15. A process for the production of a pharmaceutical, which comprises bringing at least one compound of the formula I, Ia or Ib as claimed in claims 7 to 12, its possible stereoisomeric forms and/or at least one of its physiologically tolerable salts into a suitable form for administration with a physiologically acceptable excipient and, if desired, other additives and/or auxiliaries.

16. The use of at least one compound of the formula I, Ia or Ib as claimed in claims 7 to 12, its possible stereoisomeric forms and/or at least one of its physiologically tolerable salts for the production of pharmaceuticals for the treatment of carcinoses.

17. The use of one or more compounds as claimed in claim 16 for the production of pharmaceuticals for the treatment of leukemias, sarcomas, lymph node tumors and skin carcinoses.

## Claims (Claims for the following Contracting State(s): GR)

1. The use of at least one compound of the formula I, Ia or Ib 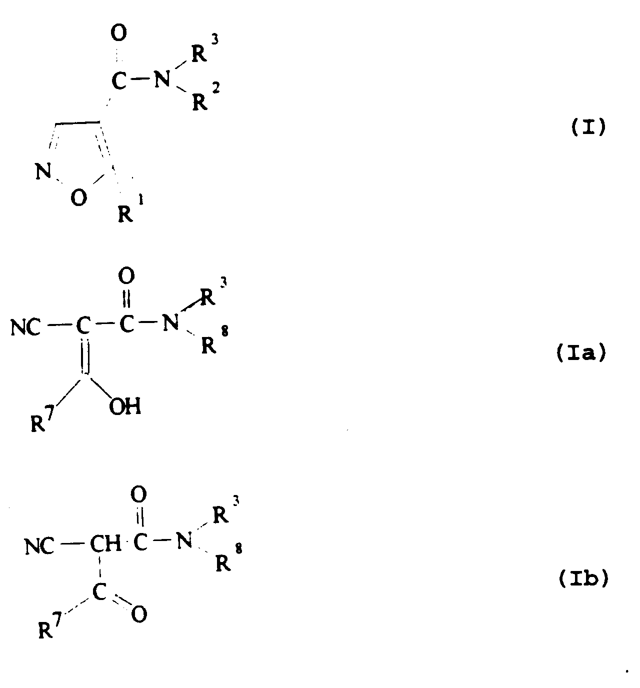 its possible stereoisomeric forms and/or, if appropriate, at least one of its physiologically tolerable salts, where
R¹ is
a) hydrogen,
b) alkyl having 1 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkyl having 1 to 4 carbon atoms,
c) phenyl-(C₁-C₂)-alkyl, in particular benzyl,
d) alkenyl having 2 to 3 carbon atoms,
R³ is
a) a mono-, di- or trinuclear, unsaturated heterocyclic radical having 3 to 13 carbon atoms and 1 to 4 heteroatoms from the group comprising oxygen, sulfur and nitrogen, of which a maximum of one is different from nitrogen, in the ring system, unsubstituted or mono- or polysubstituted by
1) halogen, such as fluorine, chlorine, bromine or iodine,
2) alkyl having 1 to 3 carbon atoms,
3) alkyl having 1 to 3 carbon atoms, mono- or polysubstituted by
3.1 halogen, such as fluorine, chlorine, bromine or iodine,
4) alkoxy having 1 to 3 carbon atoms,
5) alkoxy having 1 to 3 carbon atoms, mono- or polysubstituted by
5.1 halogen, such as fluorine, chlorine, bromine or iodine,
6) nitro,
7) hydroxyl,
8) carboxyl,
9) carbamoyl,
10) an oxo group,
b) a radical of the formula II, 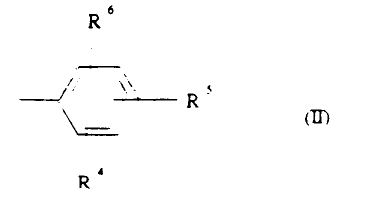 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶, together with the phenyl ring of the formula II, form a naphthalene ring,
5) in which R⁴ is hydrogen and R⁵ and R⁶ form a methylenedioxy radical,
6) alkoxy having 1 to 3 carbon atoms,
7) alkoxy having 1 to 3 carbon atoms, mono- or polysubstituted by
7.1 halogen, such as fluorine, chlorine, bromine or iodine,
8) (C₁-C₃)-alkylmercapto,
9) (C₁-C₃)-alkylmercapto, mono- or polysubstituted by
9.1 halogen, such as fluorine, chlorine, bromine or iodine,
10) halogen, such as fluorine, chlorine, bromine or iodine,
11) nitro,
12) cyano,
13) hydroxyl,
14) carboxyl,
15) (C₁-C₃)-alkylsulfonyl,
16) carbalkoxy, having 1 to 3 carbon atoms in the alkyl chain,
17) benzoyl,
18) benzoyl, mono- or polysubstituted by
18.1 halogen, such as fluorine, chlorine, bromine or iodine,
18.2 (C₁-C₃)-alkyl,
18.3 (C₁-C₃)-alkoxy,
19) phenyl,
20) phenyl, mono- or polysubstituted by
20.1 (C₁-C₃)-alkoxy,
20.2 halogen, such as fluorine, chlorine, bromine or iodine,
20.3 (C₁-C₃)-alkyl,
21) phenoxy, mono- or polysubstituted by
21.1 (C₁-C₃)-alkoxy, mono- or polysubstituted by
21.1.1 halogen, such as fluorine, chlorine, bromine or iodine,
21.2 halogen, such as fluorine, chlorine, bromine or iodine,
21.3 (C₁-C₃)-alkyl, mono- or polysubstituted by
21.3.1 halogen, such as fluorine, chlorine, bromine or iodine,
c) a radical of the formula III,
-(CH₂)ₙ-COOR¹⁰ (III)
in which R¹⁰ is
1) hydrogen
2) alkyl having 1 to 4 carbon atoms,
n is an integer from 1 to 12,
d) R² and R³, together with the nitrogen to which they are bonded, form a 4- to 9-membered ring, substituted by
1) carbonyl on the carbon atom adjacent to the nitrogen atom,
e) R² and R³, together with the nitrogen to which they are bonded, form a 5- to 6-membered ring of the formula IV, 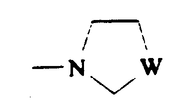 in which W is
1) -CH₂-,
2) -CH₂-CH₂-,
3) 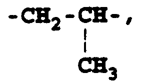
4) 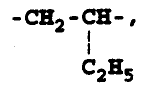
5) 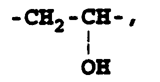
6) -CH₂-O- or
7) -CH₂-S-
R⁷ is
a) hydrogen,
b) alkyl having 1 to 17 carbon atoms,
c) alkyl having 1 to 3 carbon atoms, mono- or polysubstituted by halogen such as fluorine, chlorine, bromine or iodine,
d) phenyl-(C₁-C₂)-alkyl, in particular benzyl,
R⁸ is
a) hydrogen,
b) methyl,
c) alkenyl having 2 to 3 carbon atoms,
for the production of pharm aceuticals for the treatment of carcinoses.

2. The use as claimed in claim 1, wherein
R¹ is
a) hydrogen,
b) alkyl having 1 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkyl having 1 to 4 carbon atoms,
c) phenyl-(C₁-C₂)-alkyl, in particular benzyl,
d) alkenyl having 2 to 3 carbon atoms,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 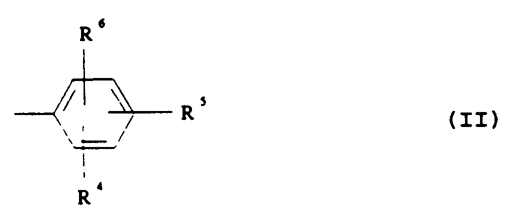 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶ form a methylenedioxy radical,
5) alkoxy having 1 to 3 carbon atoms,
6) alkoxy having 1 to 3 carbon atoms, mono- or polysubstituted by
6.1 halogen, such as fluorine, chlorine, bromine or iodine,
7) (C₁-C₃)-alkylmercapto,
8) (C₁-C₃)-alkylmercapto, mono- or polysubstituted by
8.1 halogen, such as fluorine, chlorine, bromine or iodine,
9) halogen, such as fluorine, chlorine, bromine or iodine,
10) nitro,
11) cyano,
12) (C₁-C₃)-alkylsulfonyl,
13) benzoyl,
14) benzoyl, mono- or polysubstituted by
14.1 halogen, such as fluorine, chlorine, bromine or iodine,
14.2 (C₁-C₃)-alkyl,
14.3 (C₁-C₃)-alkoxy,
15) phenoxy, mono- or polysubstituted by
15.1 (C₁-C₃)-alkoxy, mono- or polysubstituted by
15.1.1 halogen, such as fluorine, chlorine, bromine or iodine,
15.2 halogen, such as fluorine, chlorine, bromine or iodine,
15.3 (C₁-C₃)-alkyl, mono- or polysubstituted by
15.3.1 halogen, such as fluorine, chlorine, bromine or iodine,
c) a radical of the formula III,
-(CH₂)ₙ-COOR¹⁰ (III)
in which R¹⁰ is
1) hydrogen,
2) alkyl having 1 to 4 carbon atoms,
n is an integer from 1 to 12,
d) R² and R³, together with the nitrogen to which they are bonded, form a 4- to 9-membered ring, substituted by
1) carbonyl on the carbon atom adjacent to the nitrogen atom,
R⁷ is
a) hydrogen,
b) alkyl having 1 to 17 carbon atoms,
c) alkyl having 1 to 3 carbon atoms, mono- or polysubstituted by halogen, such as fluorine, chlorine, bromine or iodine,
d) phenyl-(C₁-C₂)-alkyl, in particular benzyl,
R⁸ is
a) hydrogen,
b) methyl,
c) alkenyl having 2 to 3 carbon atoms.

3. The use as claimed in claim 1, wherein
R¹ is alkyl having 1 to 6 carbon atoms,
R² is hydrogen,
R³ is
a) pyridyl, mono- or polysubstituted by halogen, such as fluorine, chlorine, bromine or iodine,
b) a radical of the formula II, 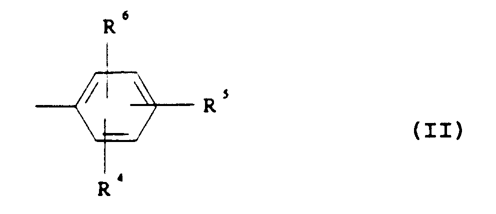 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶ form a methylenedioxy radical,
5) alkoxy having 1 to 3 carbon atoms,
6) alkoxy having 1 to 3 carbon atoms, mono- or polysubstituted by
6.1 halogen, such as fluorine, chlorine, bromine or iodine,
7) halogen, such as fluorine, chlorine, bromine or iodine,
8) nitro,
9) benzoyl,
10) benzoyl, mono- or polysubstituted by
10.1 halogen, such as fluorine, chlorine, bromine or iodine,
10.2 (C₁-C₃)-alkyl,
10.3 (C₁-C₃)-alkoxy,
11) phenoxy, mono- or polysubstituted by
11.1 (C₁-C₃)-alkoxy, mono- or polysubstituted by
11.1.1 halogen, such as fluorine, chlorine, bromine or iodine,
11.2 halogen, such as fluorine, chlorine, bromine or iodine,
11.3 (C₁-C₃)-alkyl, mono- or polysubstituted by
11.3.1 halogen, such as fluorine, chlorine, bromine or iodine,
R⁷ is alkyl having 1 to 17 carbon atoms,
R⁸ is hydrogen.

4. The use of methylisoxazole-4-carboxylic acid-(4-trifluoromethyl)anilide or N-(4-trifluoromethylphenyl)-2-cyano-3-hydroxycrotonamide for the production of pharmaceuticals for the treatment of carcinoses.

5. The use of one or more of the compounds as claimed in claim 1 for the production of pharmaceuticals for the treatment of leukemias, sarcomas, lymph node tumors or skin carcinoses.

6. A process for the preparation of a compound of the formula I 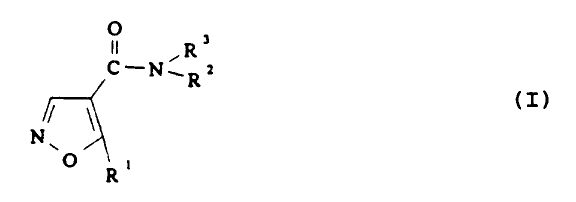 its possible stereoisomeric forms and/or, if appropriate, its physiologically tolerable salts, where
R¹ is
a) hydrogen,
b) alkyl having 2 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkenyl having 2 to 3 carbon atoms,
c) benzyl,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 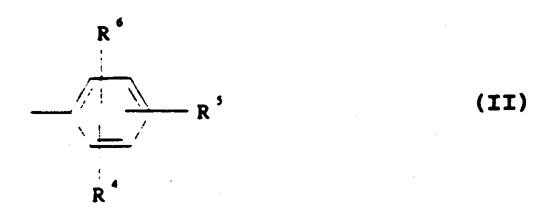 in which R⁴, R⁵ and R⁶ can be identical or different and are
1) halogen, such as fluorine, chlorine, bromine or iodine,
2) nitro,
3) hydrogen,
4) benzoyl, mono- or polysubstituted by
4.1 halogen, such as fluorine, chlorine, bromine or iodine,
4.2 methyl,
4.3 methoxy,
4.4 benzoyl,
5) (C₁-C₃)-alkoxy, mono- or polysubstituted by
5.1 halogen, such as fluorine, chlorine, bromine or iodine,
6) (C₁-C₃)-alkyl, mono- or polysubstituted by
6.1 halogen, such as fluorine, chlorine, bromine or iodine,
7) hydroxyl,
8) alkylsulfonyl, having 1 to 3 carbon atoms in the alkyl chain,
9) in which R⁴ is hydrogen and R⁵ and R⁶ together form a methylenedioxy radical,
10) cyano,
11) (C₁-C₃)-alkylmercapto,
12) benzoyl,
c) pyrimidinyl mono- or polysubstituted by
1) alkyl having 1 to 3 carbon atoms,
d) indolyl or
e) indazolinyl,
which comprises
a) reacting a compound of the formula V, 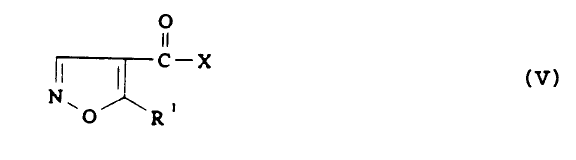 in which X is a halogen atom, preferably chlorine or bromine, and R¹ is as defined in formula I, with the amine of the formula VI 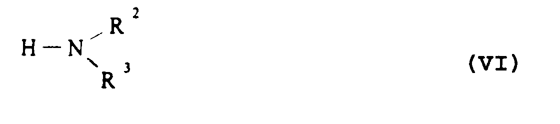 in which R² and R³ are as defined in formula I.

7. The process as claimed in claim 6, wherein a compound of the formula I is prepared, where
R¹ is
a) hydrogen,
b) alkyl having 2 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkenyl having 2 to 3 carbon atoms,
c) benzyl,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 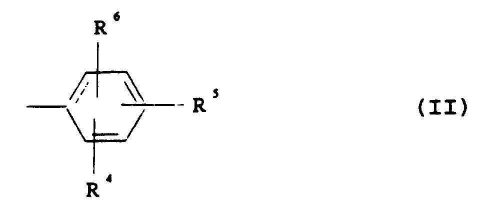 in which R⁴, R⁵ and R⁶ can be identical or different and are
1) halogen, such as fluorine, chlorine, bromine or iodine,
2) nitro,
3) hydrogen,
4) benzoyl, mono- or polysubstituted by
4.1 halogen, such as fluorine, chlorine, bromine or iodine,
4.2 methyl,
4.3 methoxy,
4.4 benzoyl,
5) (C₁-C₃)-alkoxy, mono- or polysubstituted by halogen, such as fluorine, chlorine, bromine or iodine,
6) (C₁-C₃)-alkyl, mono- or polysubstituted by halogen, such as fluorine, chlorine, bromine or iodine,
7) hydroxyl,
8) alkylsulfonyl, having 1 to 3 carbon atoms in the alkyl chain,
9) in which R⁴ is hydrogen and R⁵ and R⁶ together form a methylenedioxy radical,
10) cyano,
11) (C₁-C₃)-alkylmercapto,
12) benzoyl,
c) pyrimidinyl, mono- or polysubstituted by alkyl having 1 to 3 carbon atoms,
d) indolyl or
e) indazolinyl.

8. The process as claimed in claim 6, wherein a compound of the formula I is prepared, where
R¹ is alkyl having 2 to 6 carbon atoms,
R² is hydrogen,
R³ is
a) pyridyl, mono- or polysubstituted by
1) hydrogen,
2) halogen, such as fluorine, chlorine, bromine or iodine,
b) a radical of the formula II, 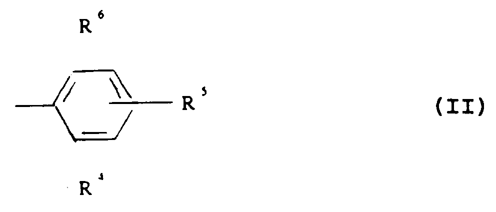 in which R⁴, R⁵ and R⁶ can be identical or different and are
1) halogen, such as fluorine, chlorine, bromine or iodine,
2) nitro,
3) benzoyl,
4) benzoyl, mono- or polysubstituted by
4.1 halogen, such as fluorine, chlorine, bromine or iodine,
4.2 methyl,
5) (C₁-C₃)-alkoxy, mono- or polysubstituted by halogen, such as fluorine, chlorine, bromine or iodine,
6) (C₁-C₃)-alkyl, mono- or polysubstituted by halogen, such as fluorine, chlorine, bromine or iodine,
7) hydrogen,
8) in which R⁴ is hydrogen and R⁵ and R⁶ together form a methylenedioxy radical.

9. A process for the preparation of a compound of the formula Ia or the formula Ib 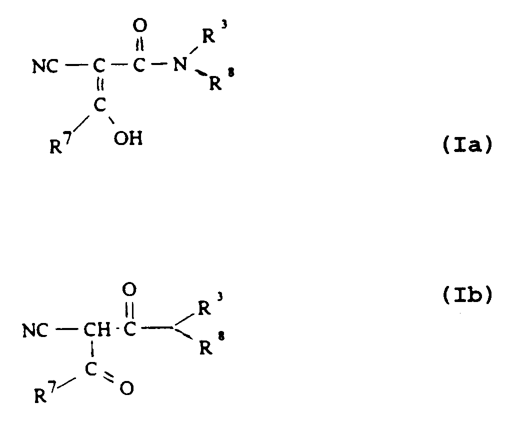 its possible stereoisomeric forms and/or, if appropriate, its physiologically tolerable salts, where the radicals R⁷, R⁸ and R³ are the following
a) where R⁷ is
1) hydrogen,
2) alkyl having 2 to 4 carbon atoms,
where R⁸ is
1) hydrogen,
2) methyl,
where R³ is
1) phenyl,
2) phenyl, mono- or polysubstituted by
2.1 halogen,
2.2 alkyl having 1 to 3 carbon atoms, mono- or polysubstituted by halogen, such as fluorine, chlorine, bromine or iodine,
b) where R⁷ is
1) alkyl having 1 to 4 carbon atoms,
2) hydrogen,
3) CF₃,
where R⁸ is
1) hydrogen,
2) methyl,
3) alkenyl having 2 to 3 carbon atoms,
where R³ is
1) pyridyl,
2) pyridyl, mono- or polysubstituted by
2.1 halogen, such as fluorine, chlorine, bromine or iodine,
2.2 alkyl having 1 to 3 carbon atoms,
3) pyrimidinyl, substituted as for 2),
4) thiazolyl, substituted as for 2) and
4.1 alkoxycarbonyl, having 1 to 3 carbon atoms in the alkyl chain,
5) benzothiazolyl, substituted as for 2),
6) benzimidazolyl, substituted as for 2),
7) indazolyl, substituted as for 2),
8) phenyl, mono- or polysubstituted by
8.1 benzoyl,
8.2 benzoyl, mono- or polysubstituted by
8.2.1 halogen, such as fluorine, chlorine, bromine or iodine,
8.2.2 alkyl having 1 to 3 carbon atoms,
8.2.3 alkoxy having 1 to 3 carbon atoms,
8.2.4 alkoxy having 1 to 3 carbon atoms, mono- or poly-substituted by halogen, such as fluorine, chlorine, bromine or iodine,
8.3 carboxyl or
8.4 hydroxyl,
c) where R⁷ is
1) hydrogen,
2) alkyl having 1 to 4 carbon atoms,
where R⁸ is
1) hydrogen,
2) methyl,
where R³ is
1) a radical of the formula III,
-(CH₂)₄-COOR¹⁰
in which R¹⁰ is
1.1 hydrogen
1.2 alkyl having 1 to 4 carbon atoms,
d) where R⁷ is
1) hydrogen,
2) alkyl having 1 to 4 carbon atoms,
R⁸ and R³, together with the nitrogen to which they are bonded, form a 4- to 9-membered ring, substituted by
2.1 carbonyl on the carbon atom adjacent to the nitrogen atom, or
R⁸ and R³, together with the nitrogen to which they are bonded, form a piperidine ring optionally substituted by alkyl having 1 to 3 carbon atoms,
which comprises
a) reacting a compound of the formula V, 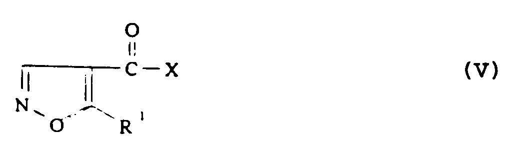 in which X is a halogen atom, preferably chlorine or bromine, and R¹ is as defined in formula I, with the amine of the formula VI 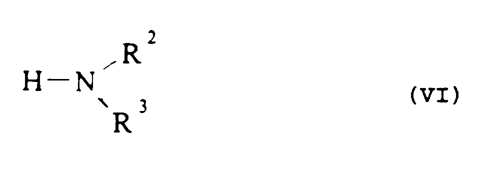 in which R² and R³ are as defined in formulae Ia and Ib, or
b) treating a compound of the formula VII 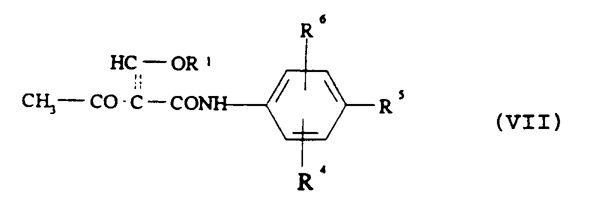 in which R¹ is (C₁-C₄)-alkyl and R⁴, R⁵ and R⁶ are as defined in formula I, with an expediently at least equimolar amount of hydroxylamine in an organic solvent, or
c) reacting a compound of the formula V, in which X and R¹ are as defined above, with a primary aliphatic amine of the formula (VIII)
H₂N-(CH₂)₄-COOR¹⁰ (VIII)
in which R¹⁰ is as defined in formula I, and
d) reacting the compound of the formula I prepared according to process steps a and c in the presence of a basic agent to give the corresponding compound of the formula Ia or Ib.

10. The process as claimed in claim 9, wherein a compound of the formula Ia or Ib is prepared, where the radicals R⁷, R⁸ and R³ are the following
a) where R⁷ is alkyl having 2 to 4 carbon atoms,
where R⁸ is hydrogen,
where R³ is
1) phenyl,
2) phenyl, mono- or polysubstituted by
2.1 halogen, such as fluorine, chlorine, bromine or iodine,
2.2 alkyl having 1 to 3 carbon atoms, monoor polysubstituted by halogen, such as fluorine, chlorine, bromine or iodine,
b) where R⁷ is alkyl having 1 to 4 carbon atoms,
where R⁸ is hydrogen,
where R³ is
1) pyridyl,
2) pyridyl, mono- or polysubstituted by
2.1 halogen, such as fluorine, chlorine, bromine or iodine,
2.2 alkyl having 1 to 3 carbon atoms,
3) phenyl, mono- or polysubstituted by
3.1 benzoyl,
3.2 benzoyl, mono- or polysubstituted by
3.2.1 halogen, such as fluorine, chlorine, bromine or iodine,
3.2.2 alkyl having 1 to 3 carbon atoms,
3.2.3 alkoxy having 1 to 3 carbon atoms,
3.2.4 alkoxy having 1 to 3 carbon atoms, mono- or polysubstituted by halogen, such as fluorine, chlorine, bromine or iodine.

11. The process as claimed in claims 6 to 10, wherein the compound N-(4-chlorodifluoromethoxy)phenyl-5-ethylisoxazole-4-carboxamide, N-(4-methylsulfonylphenyl)-5-methylisoxazole-4-carboxamide, N-(5-indolyl)-5-methylisoxazole-4-carboxamide, N-(6-indazolyl)-5-methylisoxazole-4-carboxamide, N-(5-indazolyl)-5-methylisoxazole-4-carboxamide, N-allyl-N-phenyl-5-methylisoxazole-4-carboxamide, N-(4,6-dimethyl-2-pyridyl)-5-methylisoxazole-4-carboxamide, N-(4,6-dimethyl-2-pyrimidinyl)-5-methylisoxazole-4-carboxamide, N-(4-trifluoromethoxyphenyl)-2-cyano-3-hydroxycrotonamide, N-[4-(1,1,2,2-tetrafluoroethoxy)-phenyl]-2-cyano-3-hydroxycrotonamide, N-[4-(4-fluorobenzoyl)-phenyl]-2-cyano-3-hydroxycrotonamide or N-(3,4-methylenedioxyphenyl)-2-cyano-3-hydroxy-4-methyl crotonamide is prepared.

12. The use of at least one compound of the formula I, Ia or Ib as claimed in claims 6 to 11, its possible stereoisomeric forms and/or at least one of its physiologically tolerable salts for the production of pharmaceuticals for the treatment of rheumatic disorders.

13. A process for the production of a pharmaceutical, which comprises bringing at least one compound of the formula I, Ia or Ib prepared as in claims 6 to 11, its possible stereoisomeric forms and/or at least one of its physiologically tolerable salts into a suitable form for administration with a physiologically acceptable excipient and, if desired, other additives and/or auxiliaries.

14. The use of at least one compound of the formula I, Ia or Ib as claimed in claims 6 to 11, its possible stereoisomeric forms and/or at least one of its physiologically tolerable salts for the production of pharmaceuticals for the treatment of carcinoses.

15. The use of one or more compounds as claimed in claim 14 for the production of pharmaceuticals for the treatment of leukemias, sarcomas, lymph node tumors and skin carcinoses.

## Claims (Claims for the following Contracting State(s): ES)

1. The use of at least one compound of the formula I, Ia or Ib 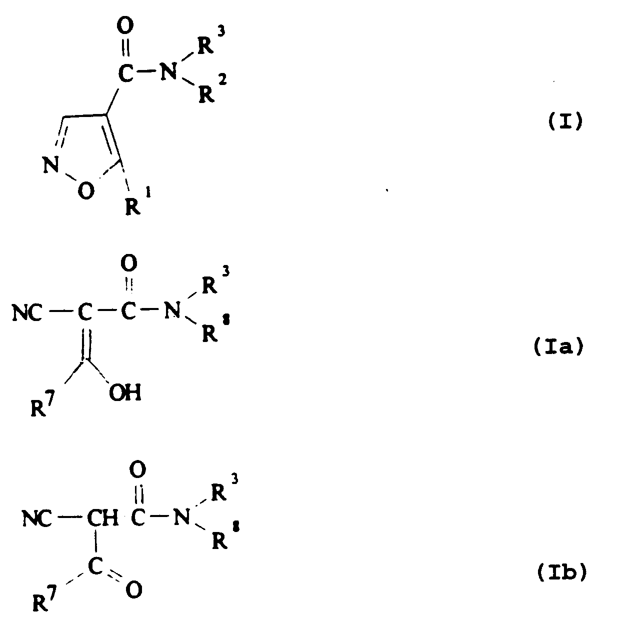 its possible stereoisomeric forms and/or, if appropriate, at least one of its physiologically tolerable salts, where
R¹ is
a) hydrogen
b) alkyl having 1 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkyl having 1 to 4 carbon atoms,
c) phenyl-(C₁-C₂)-alkyl, in particular benzyl,
d) alkenyl having 2 to 3 carbon atoms,
R³ is
a) a mono-, di- or trinuclear, unsaturated heterocyclic radical having 3 to 13 carbon atoms and 1 to 4 heteroatoms from the group comprising oxygen, sulfur and nitrogen, of which a maximum of one is different from nitrogen, in the ring system, unsubstituted or mono- or polysubstituted by
1) halogen, such as fluorine, chlorine, bromine or iodine,
2) alkyl having 1 to 3 carbon atoms,
3) alkyl having 1 to 3 carbon atoms, mono- or polysubstituted by
3.1 halogen, such as fluorine, chlorine, bromine or iodine,
4) alkoxy having 1 to 3 carbon atoms,
5) alkoxy having 1 to 3 carbon atoms, mono- or polysubstituted by
5.1 halogen, such as fluorine, chlorine, bromine or iodine,
6) nitro,
7) hydroxyl,
8) carboxyl,
9) carbamoyl,
10) an oxo group,
b) a radical of the formula II, 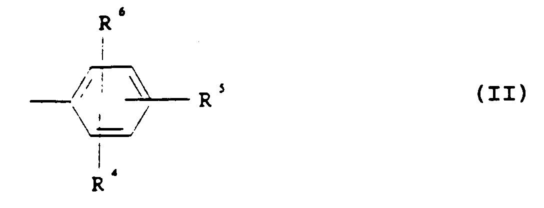 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶, together with the phenyl ring of the formula II, form a naphthalene ring,
5) in which R⁴ is hydrogen and R⁵ and R⁶ form a methylenedioxy radical,
6) alkoxy having 1 to 3 carbon atoms,
7) alkoxy having 1 to 3 carbon atoms, mono- or polysubstituted by
7.1 halogen, such as fluorine, chlorine, bromine or iodine,
8) (C₁-C₃)-alkylmercapto,
9) (C₁-C₃)-alkylmercapto, mono- or polysubstituted by
9.1 halogen, such as fluorine, chlorine, bromine or iodine,
10) halogen, such as fluorine, chlorine, bromine or iodine,
11) nitro,
12) cyano,
13) hydroxyl,
14) carboxyl,
15) (C₁-C₃)-alkylsulfonyl,
16) carbalkoxy, having 1 to 3 carbon atoms in the alkyl chain,
17) benzoyl,
18) benzoyl, mono- or polysubstituted by
18.1 halogen, such as fluorine, chlorine, bromine or iodine,
18.2 (C₁-C₃)-alkyl,
18.3 (C₁-C₃)-alkoxy,
19) phenyl,
20) phenyl, mono- or polysubstituted by
20.1 (C₁-C₃)-alkoxy,
20.2 halogen, such as fluorine, chlorine, bromine or iodine,
20.3 (C₁-C₃)-alkyl,
21) phenoxy, mono- or polysubstituted by
21.1 (C₁-C₃)-alkoxy, mono- or polysubstituted by
21.1.1 halogen, such as fluorine, chlorine, bromine or iodine,
21.2 halogen, such as fluorine, chlorine, bromine or iodine,
21.3 (C₁-C₃)-alkyl, mono- or poly-substituted by
21.3.1 halogen, such as fluorine, chlorine, bromine or iodine,
c) a radical of the formula III,
-(CH₂)ₙ-COOR¹⁰ (III)
in which R¹⁰ is
1) hydrogen
2) alkyl having 1 to 4 carbon atoms,
n is an integer from 1 to 12,
d) R² and R³, together with the nitrogen to which they are bonded, form a 4- to 9-membered ring, substituted by
1) carbonyl on the carbon atom adjacent to the nitrogen atom,
e) R² and R³, together with the nitrogen to which they are bonded, form a 5- to 6-membered ring of the formula IV, 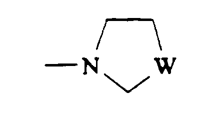 in which W is
1) -CH₂-,
2) -CH₂-CH₂-,
3) 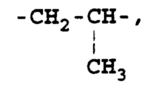
4) 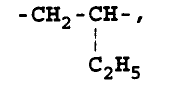
5) 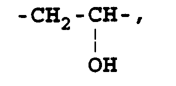
6) -CH₂-O- or
7) -CH₂-S-
R⁷ is
a) hydrogen,
b) alkyl having 1 to 17 carbon atoms,
c) alkyl having 1 to 3 carbon atoms, mono- or polysubstituted by halogen such as fluorine, chlorine, bromine or iodine,
d) phenyl-(C₁-C₂)-alkyl, in particular benzyl,
R⁸ is
a) hydrogen,
b) methyl,
c) alkenyl having 2 to 3 carbon atoms, for the production of pharm aceuticals for the treatment of carcinoses.

2. The use as claimed in claim 1, wherein
R¹ is
a) hydrogen,
b) alkyl having 1 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkyl having 1 to 4 carbon atoms,
c) phenyl-(C₁-C₂)-alkyl, in particular benzyl,
d) alkenyl having 2 to 3 carbon atoms,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 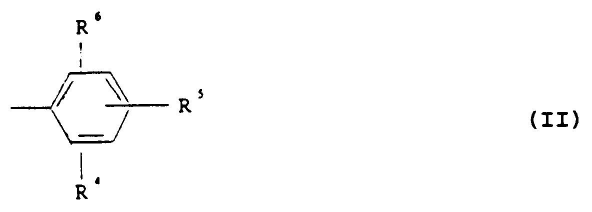 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶ form a methylenedioxy radical,
5) alkoxy having 1 to 3 carbon atoms,
6) alkoxy having 1 to 3 carbon atoms, mono- or polysubstituted by
6.1 halogen, such as fluorine, chlorine, bromine or iodine,
7) (C₁-C₃)-alkylmercapto,
8) (C₁-C₃)-alkylmercapto, mono- or polysubstituted by
8.1 halogen, such as fluorine, chlorine, bromine or iodine,
9) halogen, such as fluorine, chlorine, bromine or iodine,
10) nitro,
11) cyano,
12) (C₁-C₃)-alkylsulfonyl,
13) benzoyl,
14) benzoyl, mono- or polysubstituted by
14.1 halogen, such as fluorine, chlorine, bromine or iodine,
14.2 (C₁-C₃)-alkyl,
14.3 (C₁-C₃)-alkoxy,
15) phenoxy, mono- or polysubstituted by
15.1 (C₁-C₃)-alkoxy, mono- or polysubstituted by
15.1.1 halogen, such as fluorine, chlorine, bromine or iodine,
15.2 halogen, such as fluorine, chlorine, bromine or iodine,
15.3 (C₁-C₃)-alkyl, mono- or polysubstituted by
15.3.1 halogen, such as fluorine, chlorine, bromine or iodine,
c) a radical of the formula III,
-(CH₂)ₙ-COOR¹⁰ (III)
in which R¹⁰ is
1) hydrogen,
2) alkyl having 1 to 4 carbon atoms,
n is an integer from 1 to 12,
d) R² and R³, together with the nitrogen to which they are bonded, form a 4- to 9-membered ring, substituted by
1) carbonyl on the carbon atom adjacent to the nitrogen atom,
R⁷ is
a) hydrogen,
b) alkyl having 1 to 17 carbon atoms,
c) alkyl having 1 to 3 carbon atoms, mono- or polysubstituted by halogen, such as fluorine, chlorine, bromine or iodine,
d) phenyl-(C₁-C₂)-alkyl, in particular benzyl,
R⁸ is
a) hydrogen,
b) methyl,
c) alkenyl having 2 to 3 carbon atoms.

3. The use as claimed in claim 1, wherein
R¹ is alkyl having 1 to 6 carbon atoms,
R² is hydrogen
R³ is
a) pyridyl, mono- or polysubstituted by halogen, such as fluorine, chlorine, bromine or iodine,
b) a radical of the formula II, 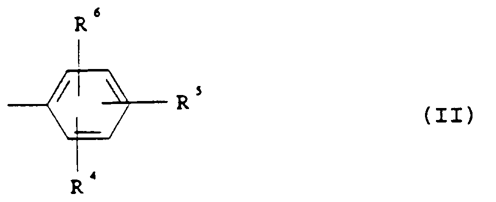 in which R⁴, R⁵ and R⁶ can be identical or different and are
1) hydrogen,
2) phenoxy,
3) alkyl having 1 to 3 carbon atoms, mono- or polysubstituted by
3.1 halogen, such as fluorine, chlorine, bromine or iodine,
4) in which R⁴ is hydrogen and R⁵ and R⁶ form a methylenedioxy radical,
5) alkoxy having 1 to 3 carbon atoms,
6) alkoxy having 1 to 3 carbon atoms, mono- or polysubstituted by
6.1 halogen, such as fluorine, chlorine, bromine or iodine,
7) halogen, such as fluorine, chlorine, bromine or iodine,
8) nitro,
9) benzoyl,
10) benzoyl, mono- or polysubstituted by
10.1 halogen, such as fluorine, chlorine, bromine or iodine,
10.2 (C₁-C₃)-alkyl,
10.3 (C₁-C₃)-alkoxy,
11) phenoxy, mono- or polysubstituted by
11.1 (C₁-C₃)-alkoxy, mono- or polysubstituted by
11.1.1 halogen, such as fluorine, chlorine, bromine or iodine,
11.2 halogen, such as fluorine, chlorine, bromine or iodine,
11.3 (C₁-C₃)-alkyl, mono- or polysubstituted by
11.3.1 halogen, such as fluorine, chlorine, bromine or iodine,
R⁷ is alkyl having 1 to 17 carbon atoms,
R⁸ is hydrogen.

4. The use of methylisoxazole-4-carboxylic acid-(4-trifluoromethyl)anilide or N-(4-trifluoromethylphenyl)-2-cyano-3-hydroxycrotonamide for the production of pharmaceuticals for the treatment of carcinoses.

5. The use of one or more of the compounds as claimed in claim 1 for the production of pharmaceuticals for the treatment of leukemias, sarcomas, lymph node tumors or skin carcinoses.

6. A process for the preparation of a compound of the formula I 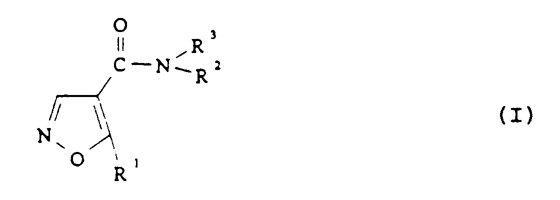 its possible stereoisomeric forms and/or, if appropriate, its physiologically tolerable salts, where
R¹ is
a) hydrogen,
b) alkyl having 2 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkenyl having 2 to 3 carbon atoms,
c) benzyl,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 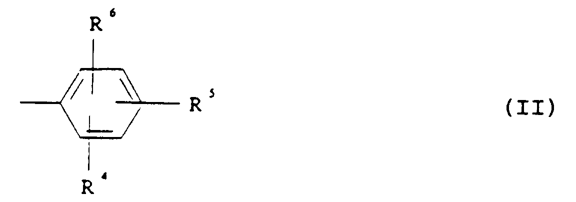 in which R⁴, R⁵ and R⁶ can be identical or different and are
1) halogen, such as fluorine, chlorine, bromine or iodine,
2) nitro,
3) hydrogen,
4) benzoyl, mono- or polysubstituted by
4.1 halogen, such as fluorine, chlorine, bromine or iodine,
4.2 methyl,
4.3 methoxy,
4.4 benzoyl,
5) (C₁-C₃)-alkoxy, mono- or polysubstituted by
5.1 halogen, such as fluorine, chlorine, bromine or iodine,
6) (C₁-C₃)-alkyl, mono- or polysubstituted by
6.1 halogen, such as fluorine, chlorine, bromine or iodine,
7) hydroxyl,
8) alkylsulfonyl, having 1 to 3 carbon atoms in the alkyl chain,
9) in which R⁴ is hydrogen and R⁵ and R⁶ together form a methylenedioxy radical,
10) cyano,
11) (C₁-C₃)-alkylmercapto,
12) benzoyl,
c) pyrimidinyl mono- or polysubstituted by
1) alkyl having 1 to 3 carbon atoms,
d) indolyl or
e) indazolinyl,
which comprises
a) reacting a compound of the formula V, 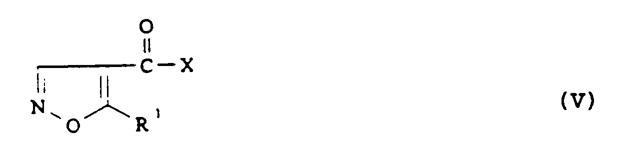 in which X is a halogen atom, preferably chlorine or bromine, and R¹ is as defined in formula I, with the amine of the formula VI 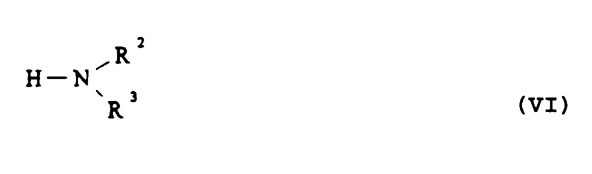 in which R² and R³ are as defined in formula I.

7. The process as claimed in claim 6, wherein a compound of the formula I is prepared, where
R¹ is
a) hydrogen,
b) alkyl having 2 to 6 carbon atoms,
c) alkyl having 1 to 4 carbon atoms, mono- or polysubstituted by
1) halogen, such as fluorine, chlorine, bromine or iodine,
d) phenyl,
R² is
a) hydrogen,
b) alkenyl having 2 to 3 carbon atoms,
c) benzyl,
R³ is
a) pyridyl, mono- or polysubstituted by
1) hydrogen,
2) halogen, such as fluorine, chlorine, bromine or iodine,
3) nitro,
4) alkyl having 1 to 3 carbon atoms,
5) alkoxy having 1 to 3 carbon atoms,
b) a radical of the formula II, 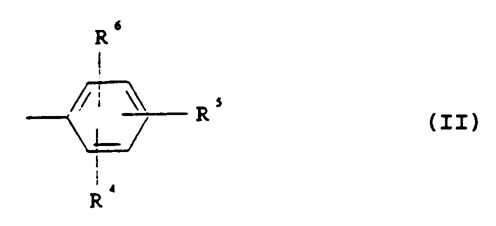 in which R⁴, R⁵ and R⁶ can be identical or different and are
1) halogen, such as fluorine, chlorine, bromine or iodine,
2) nitro,
3) hydrogen,
4) benzoyl, mono- or polysubstituted by
4.1 halogen, such as fluorine, chlorine, bromine or iodine,
4.2 methyl,
4.3 methoxy,
4.4 benzoyl,
5) (C₁-C₃)-alkoxy, mono- or poly-substituted by halogen, such as fluorine, chlorine, bromine or iodine,
6) (C₁-C₃)-alkyl, mono- or poly-substituted by halogen, such as fluorine, chlorine, bromine or iodine,
7) hydroxyl,
8) alkylsulfonyl, having 1 to 3 carbon atoms in the alkyl chain,
9) in which R⁴ is hydrogen and R⁵ and R⁶ together form a methylenedioxy radical,
10) cyano,
11) (C₁-C₃)-alkylmercapto,
12) benzoyl,
c) pyrimidinyl, mono- or polysubstituted by alkyl having 1 to 3 carbon atoms,
d) indolyl or
e) indazolinyl.

8. The process as claimed in claim 6, wherein a compound of the formula I is prepared, where
R¹ is alkyl having 2 to 6 carbon atoms,
R² is hydrogen,
R³ is
a) pyridyl, mono- or polysubstituted by
1) hydrogen,
2) halogen, such as fluorine, chlorine, bromine or iodine,
b) a radical of the formula II, 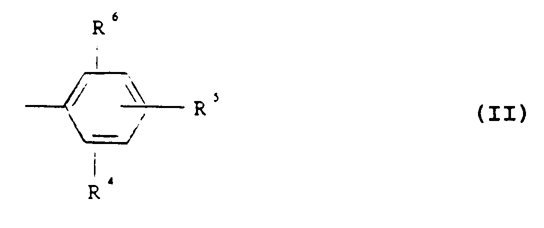 in which R⁴, R⁵ and R⁶ can be identical or different and are
1) halogen, such as fluorine, chlorine, bromine or iodine,
2) nitro,
3) benzoyl,
4) benzoyl, mono- or polysubstituted by
4.1 halogen, such as fluorine, chlorine, bromine or iodine,
4.2 methyl,
5) (C₁-C₃)-alkoxy, mono- or polysubstituted by halogen, such as fluorine, chlorine, bromine or iodine,
6) (C₁-C₃)-alkyl, mono- or polysubstituted by halogen, such as fluorine, chlorine, bromine or iodine,
7) hydrogen,
8) in which R⁴ is hydrogen and R⁵ and R⁶ together form a methylenedioxy radical.

9. A process for the preparation of a compound of the formula Ia or the formula Ib 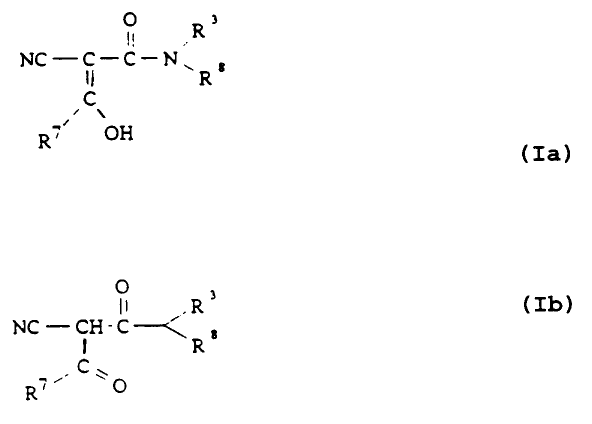 its possible stereoisomeric forms and/or, if appropriate, its physiologically tolerable salts, where the radicals R⁷, R⁸ and R³ are the following
a) where R⁷ is
1) hydrogen,
2) alkyl having 2 to 4 carbon atoms,
where R⁸ is
1) hydrogen,
2) methyl,
where R³ is
1) phenyl,
2) phenyl, mono- or polysubstituted by
2.1 halogen,
2.2 alkyl having 1 to 3 carbon atoms, mono- or polysubstituted by halogen, such as fluorine, chlorine, bromine or iodine,
b) where R⁷ is
1) alkyl having 1 to 4 carbon atoms,
2) hydrogen,
3) CF₃,
where R⁸ is
1) hydrogen,
2) methyl,
3) alkenyl having 2 to 3 carbon atoms,
where R³ is
1) pyridyl,
2) pyridyl, mono- or polysubstituted by
2.1 halogen, such as fluorine, chlorine, bromine or iodine,
2.2 alkyl having 1 to 3 carbon atoms,
3) pyrimidinyl, substituted as for 2),
4) thiazolyl, substituted as for 2) and
4.1 alkoxycarbonyl, having 1 to 3 carbon atoms in the alkyl chain,
5) benzothiazolyl, substituted as for 2),
6) benzimidazolyl, substituted as for 2),
7) indazolyl, substituted as for 2),
8) phenyl, mono- or polysubstituted by
8.1 benzoyl,
8.2 benzoyl, mono- or polysubstituted by
8.2.1 halogen, such as fluorine, chlorine, bromine or iodine,
8.2.2 alkyl having 1 to 3 carbon atoms,
8.2.3 alkoxy having 1 to 3 carbon atoms,
8.2.4 alkoxy having 1 to 3 carbon atoms, mono- or polysubstituted by halogen, such as fluorine, chlorine, bromine or iodine,
8.3 carboxyl or
8.4 hydroxyl,
c) where R⁷ is
1) hydrogen,
2) alkyl having 1 to 4 carbon atoms,
where R⁸ is
1) hydrogen,
2) methyl,
where R³ is
1) a radical of the formula III,
-(CH₂)₄-COOR¹⁰
in which R¹⁰ is
1.1 hydrogen
1.2 alkyl having 1 to 4 carbon atoms,
d) where R⁷ is
1) hydrogen,
2) alkyl having 1 to 4 carbon atoms,
R⁸ and R³, together with the nitrogen to which they are bonded, form a 4- to 9-membered ring, substituted by
2.1 carbonyl on the carbon atom adjacent to the nitrogen atom, or
R⁸ and R³, together with the nitrogen to which they are bonded, form a piperidine ring optionally substituted by alkyl having 1 to 3 carbon atoms, which comprises
a) reacting a compound of the formula V, 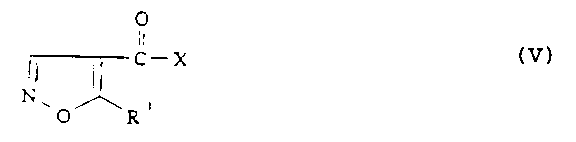 in which X is a halogen atom, preferably chlorine or bromine, and R¹ is as defined in formula I, with the amine of the formula VI 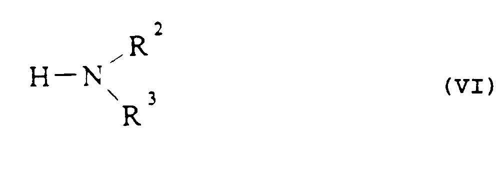 in which R² and R³ are as defined in formulae Ia and Ib, or
b) treating a compound of the formula VII 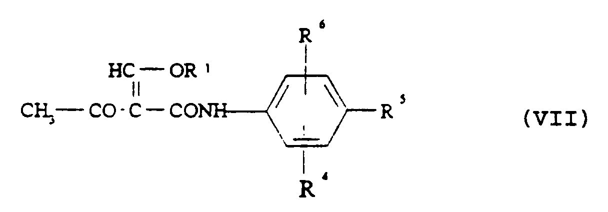 in which R¹ is (C₁-C₄)-alkyl and R⁴, R⁵ and R⁶ are as defined in formula I, with an expediently at least equimolar amount of hydroxylamine in an organic solvent, or
c) reacting a compound of the formula V, in which X and R¹ are as defined above, with a primary aliphatic amine of the formula (VIII)
H₂N-(CH₂)₄-COOR¹⁰ (VIII)
in which R¹⁰ is as defined in formula I, and
d) reacting the compound of the formula I prepared according to process steps a and c in the presence of a basic agent to give the corresponding compound of the formula Ia or Ib.

10. The process as claimed in claim 9, wherein a compound of the formula Ia or Ib is prepared, where the radicals R⁷, R⁸ and R³ are the following
a) where R⁷ is alkyl having 2 to 4 carbon atoms,
where R⁸ is hydrogen,
where R³ is
1) phenyl,
2) phenyl, mono- or polysubstituted by
2.1 halogen such as fluorine, chlorine, bromine or iodine,
2.2 alkyl having 1 to 3 carbon atoms, mono- or polysubstituted by halogen, such as fluorine, chlorine, bromine or iodine,
b) where R⁷ is alkyl having 1 to 4 carbon atoms,
where R⁸ is hydrogen,
where R³ is
1) pyridyl,
2) pyridyl, mono- or polysubstituted by
2.1 halogen, such as fluorine, chlorine, bromine or iodine,
2.2 alkyl having 1 to 3 carbon atoms,
3) phenyl, mono- or polysubstituted by
3.1 benzoyl,
3.2 benzoyl, mono- or polysubstituted by
3.2.1 halogen, such as fluorine, chlorine, bromine or iodine,
3.2.2 alkyl having 1 to 3 carbon atoms,
3.2.3 alkoxy having 1 to 3 carbon atoms,
3.2.4 alkoxy having 1 to 3 carbon atoms, mono- or polysubstituted by halogen, such as fluorine, chlorine, bromine or iodine.

11. The process as claimed in claims 6 to 10, wherein the compound N-(4-chlorodifluoromethoxy)phenyl-5-ethylisoxazole-4-carboxamide, N-(4-methylsulfonylphenyl)-5-methylisoxazole-4-carboxamide, N-(5-indolyl)-5-methylisoxazole-4-carboxamide, N-(6-indazolyl)-5-methylisoxazole-4-carboxamide, N-(5-indazolyl)-5-methylisoxazole-4-carboxamide, N-allyl-N-phenyl-5-methylisoxazole-4-carboxamide, N-(4,6-dimethyl-2-pyridyl)-5-methylisoxazole-4-carboxamide, N-(4,6-dimethyl-2-pyrimidinyl)-5-methylisoxazole-4-carboxamide, N-(4-trifluoromethoxyphenyl)-2-cyano-3-hydroxycrotonamide, N-[4-(1,1,2,2-tetrafluoroethoxy)-phenyl]-2-cyano-3-hydroxycrotonamide, N-[4-(4-fluorobenzoyl)-phenyl]-2-cyano-3-hydroxycrotonamide or N-(3,4-methylenedioxyphenyl)-2-cyano-3-hydroxy-4-methyl crotonamide is prepared.

12. The use of at least one compound of the formula I, Ia or Ib as claimed in claims 6 to 11, its possible stereoisomeric forms and/or at least one of its physiologically tolerable salts for the production of pharmaceuticals for the treatment of rheumatic disorders.

13. The use of at least one compound of the formula I, Ia or Ib as claimed in claims 6 to 11, its possible stereoisomeric forms and/or at leapt one of its physiologically tolerable salts for the production of pharmaceuticals for the treatment of carcinoses.

14. The use of one or more compounds as claimed in claim 13 for the production of pharmaceuticals for the treatment of leukemias, sarcomas, lymph node tumors and skin carcinoses.

## Revendications (Revendications pour l'(les) Etat(s) contractant(s) suivant(s): AT, BE, CH, DE, DK, FR, GB, IT, LI, LU, NL, SE)

1. Utilisation d'au moins un composé de formule I, Ia ou Ib 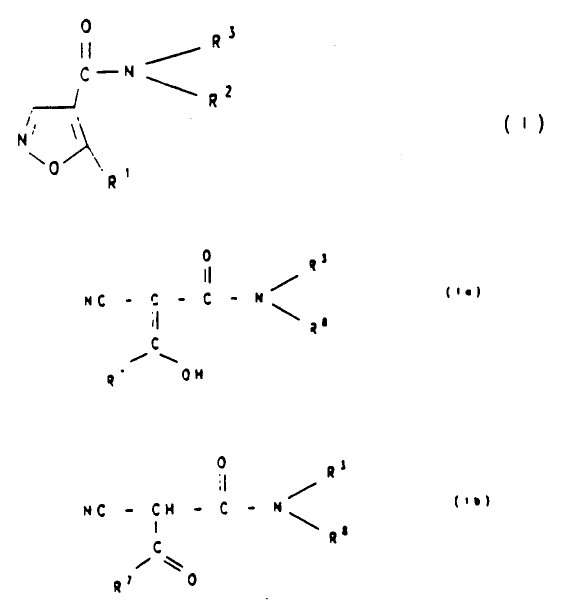 de ses formes éventuellement stéréoisomères et/ou éventuellement d'au moins un de ses sels physiologiquement acceptables,
formules dans lesquelles
R¹ représente
a) un atome d'hydrogène,
b) un groupe alkyle ayant de 1 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle.
R² représente
a) un atome d'hydrogène,
b) un groupe alkyle ayant de 1 à 4 atomes de carbone,
c) un groupe phényl-alkyle(C₁-C₂), en particulier le groupe benzyle,
d) un groupe alcényle ayant 2 ou 3 atomes de carbone,
R³ représente
a) un radical hétérocyclique insaturé mono-, bi- ou tricyclique, comportant dans le système cyclique de 3 à 13 atomes de carbone et de 1 à 4 hétéroatomes choisis parmi les atomes d'oxygène, de soufre et d'azote, dont au maximum un est différent de l'atome d'azote, non substitué ou une ou plusieurs fois substitué par
1) un ou des atomes d'halogène, tels que les atomes de fluor, chlore, brome et iode,
2) un ou des groupes alkyle ayant de 1 à 3 atomes de carbone,
3) un ou des groupes alkyle ayant de 1 à 3 atomes de carbone, une ou plusieurs fois substitués par
3.1. un ou des atomes d'halogène, tels que les atomes de fluor, chlore, brome et iode,
4) un ou des groupes alcoxy ayant de 1 à 3 atomes de carbone,
5) un ou des groupes alcoxy ayant de 1 à 3 atomes de carbone, une ou plusieurs fois substitués par
5.1. un ou des atomes d'halogène, tels que les atomes de fluor, chlore, brome et iode,
6) le groupe nitro,
7) hydroxy,
8) carboxy,
9) carbamoyle,
10) oxo,
b) un radical de formule II 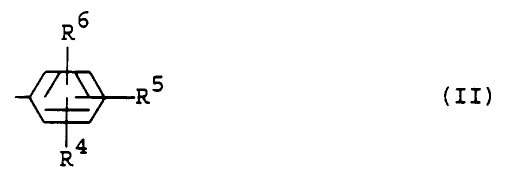 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment ensemble, avec le noyau phényle de la formule II, un cycle naphtalène,
5) dans laquelle R⁴ représente un atome d'hydrogène et R⁵ et R⁶ forment un radical méthylènedioxy,
6) un groupe alcoxy ayant de 1 à 3 atomes de carbone,
7) un groupe alcoxy ayant de 1 à 3 atomes de carbone, une ou plusieurs fois substitué par
7.1. un ou des atomes d'halogène, tels que les atomes de fluor, chlore, brome et iode,
8) un groupe alkyl(C₁-C₃)mercapto,
9) un groupe alkyl(C₁-C₃)mercapto, une ou plusieurs fois substitué par
9.1. un ou des atomes d'halogène, tels que les atomes de fluor, chlore, brome et iode,
10) un atome d'halogène, tel qu'un atome de fluor, chlore, brome ou iode,
11) le groupe nitro,
12) cyano,
13) hydroxy,
14) carboxy,
15) un groupe alkyl(C₁-C₃)sulfonyle,
16) un groupe carbalcoxy, comportant de 1 à 3 atomes de carbone dans la chaîne alkyle,
17) le groupe benzoyle,
18) un groupe benzoyle, une ou plusieurs fois substitué par
18.1. un ou des atomes d'halogène, tels que les atomes de fluor, chlore, brome et iode,
18.2. un ou des groupes alkyle en C₁-C₃,
18.3. un ou des groupes alcoxy en C₁-C₃,
19) le groupe phényle,
20) un groupe phényle, une ou plusieurs fois substitué par
20.1. un ou des groupes alcoxy en C₁-C₃,
20.2. un ou des atomes d'halogène, tels que les atomes de fluor, chlore, brome et iode,
20.3. un ou des groupes alkyle en C₁-C₃,
21) un groupe phénoxy, une ou plusieurs fois substitué par
21.1. un ou des groupes alcoxy en C₁-C₃, une ou plusieurs fois substitués par
21.1.1. un ou des atomes d'halogène, tels que les atomes de fluor, chlore, brome et iode,
21.2. un ou des atomes d'halogène, tels que les atomes de fluor, chlore, brome et iode,
21.3. un ou des groupes alkyle en C₁-C₃ une ou plusieurs fois substitués par
21.3.1. un ou des atomes d'halogène, tels que les atomes de fluor, chlore, brome et iode,
c) un radical de formule III
-(CH₂)ₙCOOR¹⁰ (III)
dans laquelle
R¹⁰ représente
1) un atome d'hydrogène,
2) un groupe alkyle ayant de 1 à 4 atomes de carbone,
n représente un nombre entier allant de 1 à 12,
d) R² et R³ forment ensemble, avec l'atome d'azote auquel ils sont liés, un cycle à 4-9 chaînons, substitué par
1) un groupe carbonyle sur l'atome de carbone contigu à l'atome d'azote,
e) R² et R³ forment ensemble, avec l'atome d'azote auquel ils sont liés, un cycle à 5-6 chaînons de formule IV 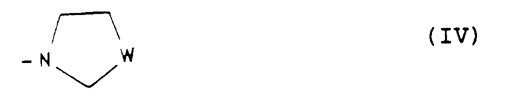 dans laquelle W représente
1) -CH₂-,
2) -CH₂-CH₂-,
3) 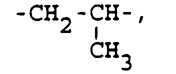
4) 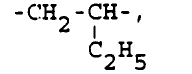
5) 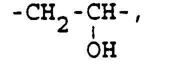
6) -CH₂-O- ou
7) -CH₂-S-,
R⁷ représente
a) un atome d'hydrogène,
b) un groupe alkyle ayant de 1 à 17 atomes de carbone,
c) un groupe alkyle ayant de 1 à 3 atomes de carbone, une ou plusieurs fois substitué par un ou des atomes d'halogène, tels que les atomes de fluor, chlore, brome et iode,
d) un groupe phényl-alkyle(C₁-C₂), en particulier le groupe benzyle,
R⁸ représente
a) un atome d'hydrogène,
b) le groupe méthyle,
c) un groupe alcényle ayant 2 ou 3 atomes de carbone, pour la fabrication de médicaments destinés au traitement de maladies cancéreuses.

2. Utilisation selon la revendication 1, caractérisée en ce que
R¹ représente
a) un atome d'hydrogène,
b) un groupe alkyle ayant de 1 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alkyle ayant de 1 à 4 atomes de carbone,
c) un groupe phényl-alkyle(C₁-C₂), en particulier le groupe benzyle,
d) un groupe alcényle ayant 2 ou 3 atomes de carbone,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
3) le groupe nitro,
4) un ou des groupes alkyle ayant de 1 à 3 atomes de carbone,
5) un ou des groupes alcoxy ayant de 1 à 3 atomes de carbone,
b) un radical de formule II 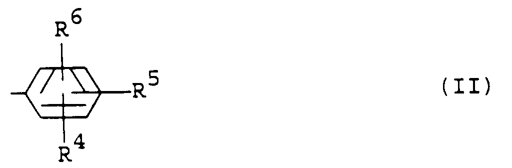 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment un radical méthylènedioxy,
5) un groupe alcoxy ayant de 1 à 3 atomes de carbone,
6) un groupe alcoxy ayant de 1 à 3 atomes de carbone, une ou plusieurs fois substitué par
6.1. un ou des atomes d'halogène, tels que les atomes de fluor, chlore, brome et iode,
7) un groupe alkyl(C₁-C₃)mercapto,
8) un groupe alkyl(C₁-C₃)mercapto, une ou plusieurs fois substitué par
8.1. un ou des atomes d'halogène, tels que les atomes de fluor, chlore, brome et iode,
9) un atome d'halogène, tel qu'un atome de fluor, chlore, brome ou iode,
10) le groupe nitro,
11) le groupe cyano,
12) un groupe alkyl(C₁-C₃)sulfonyle
13) le groupe benzoyle,
14) un groupe benzoyle, une ou plusieurs fois substitué par
14.1. un ou des atomes d'halogène, tels que les atomes de fluor, chlore, brome et iode,
14.2. un ou des groupes alkyle en C₁-C₃,
14.3. un ou des groupes alcoxy en C₁-C₃,
15) un groupe phénoxy, une ou plusieurs fois substitué par
15.1. un ou des groupes alcoxy en C₁-C₃, une ou plusieurs fois substitués par
15.1.1. un ou des atomes d'halogène, tels que les atomes de fluor, chlore, brome et iode,
15.2. un ou des atomes d'halogène, tels que les atomes de fluor, chlore, brome et iode,
15.3. un ou des groupes alkyle en C₁-C₃ une ou plusieurs fois substitués par
15.3.1. un ou des atomes d'halogène, tels que les atomes de fluor, chlore, brome et iode,
c) un radical de formule III
-(CH₂)ₙ-COOR¹⁰ (III)
dans laquelle
R¹⁰ représente
1) un atome d'hydrogène,
2) un groupe alkyle ayant de 1 à 4 atomes de carbone,
n représente un nombre entier allant de 1 à 12,
d) R² et R³ forment ensemble, avec l'atome d'azote auquel ils sont liés, un cycle à 4-9 chaînons, substitué par
1) un groupe carbonyle sur l'atome de carbone contigu à l'atome d'azote,
R⁷ représente
a) un atome d'hydrogène,
b) un groupe alkyle ayant de 1 à 17 atomes de carbone,
c) un groupe alkyle ayant de 1 à 3 atomes de carbone, une ou plusieurs fois substitué par un ou des atomes d'halogène, tels que les atomes de fluor, chlore, brome et iode,
d) un groupe phényl-alkyle(C₁-C₂), en particulier le groupe benzyle,
R⁸ représente
a) un atome d'hydrogène,
b) le groupe méthyle,
c) un groupe alcényle ayant 2 ou 3 atomes de carbone.

3. Utilisation selon la revendication 1, caractérisée que
R¹ représente un groupe alkyle ayant de 1 à 6 atomes de carbone,
R² représente un atome d'hydrogène,
R³ représente
a) un groupe pyridyle, une ou plusieurs fois substitué par un ou des atomes d'halogène, tels que les atomes de fluor, chlore, brome et iode,
b) un radical de formule II 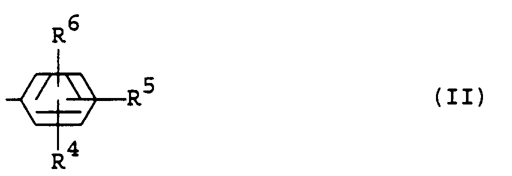 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment un radical méthylènedioxy,
5) un groupe alcoxy ayant de 1 à 3 atomes de carbone,
6) un groupe alcoxy ayant de 1 à 3 atomes de carbone, une ou plusieurs fois substitué par
6.1. un ou des atomes d'halogène, tels que les atomes de fluor, chlore, brome et iode,
7) un atome d'halogène tel qu'un atome de fluor, chlore, brome ou iode,
8) le groupe nitro,
9) le groupe benzoyle,
10) un groupe benzoyle, une ou plusieurs fois substitué par
10.1. un ou des atomes d'halogène, tels que les atomes de fluor, chlore, brome et iode,
10.2. un ou des groupes alkyle en C₁-C₃,
10.3. un ou des groupes alcoxy en C₁-C₃,
11) un groupe phénoxy, une ou plusieurs fois substitué par
11.1. un ou des groupes alcoxy en C₁-C₃, une ou plusieurs fois substitués par
11.1.1. un ou des atomes d'halogène, tels que les atomes de fluor, chlore, brome et iode,
11.2. un ou des atomes d'halogène, tels que les atomes de fluor, chlore, brome et iode,
11.3. un ou des groupes alkyle en C₁-C₃, une ou plusieurs fois substitués par
11.3.1. un ou des atomes d'halogène, tels que les atomes de fluor, chlore, brome et iode,
R⁷ représente un groupe alkyle ayant de 1 à 17 atomes de carbone,
R⁸ représente un atome d'hydrogène.

4. Utilisation du méthylisoxazole-4-(4-trifluorométhylcarboxanilide) ou du N-(4-trifluorométhylphényl)-2-cyano-3-hydroxycrotonamide, pour la fabrication de médicaments destinés au traitement de maladies cancéreuses.

5. Procédé pour la fabrication d'un médicament destiné au traitement de maladies cancéreuses, caractérisé en ce que l'on met sous une forme d'administration appropriée au moins un composé de formule I, Ia ou Ib selon la revendication 1, ses formes éventuellement isomères et/ou au moins l'un de ses sels physiologiquement acceptables, avec un véhicule physiologiquement acceptable et éventuellement d'autres additifs et/ou adjuvants.

6. Utilisation d'un ou plusieurs des composés selon la revendication 1, pour la fabrication de médicaments destinés au traitement de maladies de type leucémie, sarcome, tumeur des ganglions lymphatiques ou cancer de la peau.

7. Composé de formule I 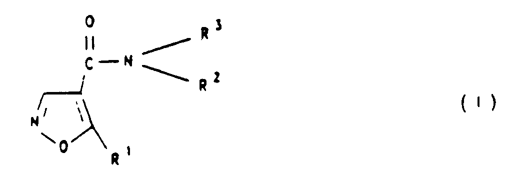 ses formes éventuellement stéréoisomères et/ou éventuellement ses sels physiologiquement acceptables, formule dans laquelle
R¹ représente
a) un atome d'hydrogène,
b) un groupe alkyle ayant de 2 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alcényle ayant 2 ou 3 atomes de carbone,
c) le groupe benzyle,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
3) le groupe nitro,
4) un ou des groupes alkyle ayant de 1 à 3 atomes de carbone,
5) un ou des groupes alcoxy ayant de 1 à 3 atomes de carbone,
b) un radical de formule II 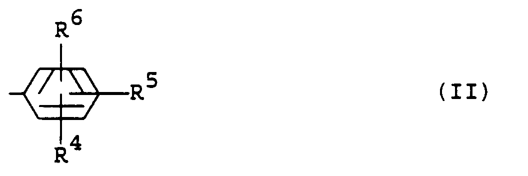 dans laquelle R⁴, R⁵, R⁶ peuvent être identiques ou différents et représentent
1) un atome d'halogène, tel qu'un atome de fluor, chlore, brome ou iode,
2) le groupe nitro,
3) un atome d'hydrogène,
4) un groupe benzoyle, une ou plusieurs fois substitué par
4.1. un ou des atomes d'halogène, tels que les atomes de fluor, chlore, brome et iode,
4.2. le groupe méthyle,
4.3. le groupe méthoxy,
4.4. le groupe benzoyle,
5) un groupe alcoxy en C₁-C₃, une ou plusieurs fois substitué par
5.1. un ou des atomes d'halogène, tels que les atomes de fluor, chlore, brome et iode,
6) un groupe alkyle en C₁-C₃, une ou plusieurs fois substitué par
6.1. un ou des atomes d'halogène, tels que les atomes de fluor, chlore, brome et iode,
7) le groupe hydroxy,
8) un groupe alkylsulfonyle ayant de 1 à 3 atomes de carbone dans la chaîne alkyle,
9) dans laquelle R⁴ représente un atome d'hydrogène et R⁵ et R⁶ forment ensemble un radical méthylènedioxy,
10) le groupe cyano,
11) un groupe alkyl(C₁-C₃)mercapto,
12) le groupe benzoyle,
c) un groupe pyrimidinyle, une ou plusieurs fois substitué par
1) un ou des groupes alkyle ayant de 1 à 3 atomes de carbone,
d) le groupe indolyle ou
e) le groupe indazolinyle.

8. Composé selon la revendication 7, caractérisé en ce que
R¹ représente
a) un atome d'hydrogène,
b) un groupe alkyle ayant de 2 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alcényle ayant 2 ou 3 atomes de carbone,
c) le groupe benzyle,
R³ représente
a) un groupe pyridyle, une ou plusieurs fois substitué par
1) un ou des atomes d'hydrogène,
2) un ou des atomes d'halogène, tels que les atomes de
fluor, chlore, brome et iode, 3) le groupe nitro,
4) un ou des groupes alkyle ayant de 1 à 3 atomes de carbone,
5) un ou des groupes alcoxy ayant de 1 à 3 atomes de carbone,
b) un radical de formule II 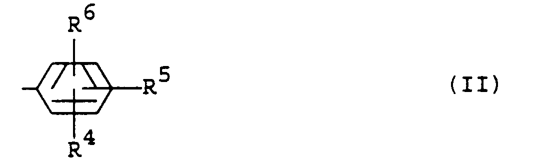 dans laquelle R⁴, R⁵, R⁶ peuvent être identiques ou différents et représentent
1) un atome d'halogène, tel qu'un atome de fluor, chlore, brome ou iode,
2) le groupe nitro,
3) un atome d'hydrogène,
4) un groupe benzoyle, une ou plusieurs fois substitué par
4.1. un ou des atomes d'halogène, tels que les atomes de fluor, chlore, brome et iode,
4.2. le groupe méthyle,
4.3. le groupe méthoxy,
4.4. le groupe benzoyle,
5) un groupe alcoxy en C₁-C₃, une ou plusieurs fois substitué par
5.1. un ou des atomes d'halogène, tels que les atomes de fluor, chlore, brome et iode,
6) un groupe alkyle en C₁-C₃, une ou plusieurs fois substitué par
6.1. un ou des atomes d'halogène, tels que les atomes de fluor, chlore, brome et iode,
7) le groupe hydroxy,
8) un groupe alkylsulfonyle ayant de 1 à 3 atomes de carbone dans la chaîne alkyle,
9) dans laquelle R⁴ représente un atome d'hydrogène et R⁵ et R⁶ forment ensemble un radical méthylènedioxy,
10) le groupe cyano,
11) un groupe alkyl(C₁-C₃)mercapto,
12) le groupe benzoyle,
c) un groupe pyrimidinyle, une ou plusieurs fois substitué par
1) un ou des groupes alkyle ayant de 1 à 3 atomes de carbone,
d) le groupe indolyle ou
e) le groupe indazolinyle.

9. Composé selon la revendication 7, caractérisé en ce que
R¹ représente un groupe alkyle ayant de 2 à 6 atomes de carbone,
R² représente un atome d'hydrogène,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
b) un radical de formule II 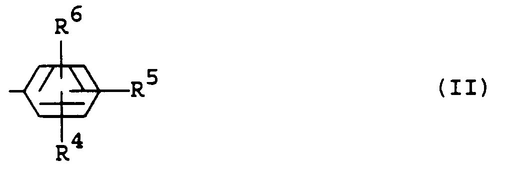 dans laquelle R⁴, R⁵, R⁶ peuvent être identiques ou différents et représentent
1) un atome d'halogène, tel qu'un atome de fluor, chlore, brome ou iode,
2) le groupe nitro,
3) le groupe benzoyle,
4) un groupe benzoyle, une ou plusieurs fois substitué par
4.1. un ou des atomes d'halogène, tels que les atomes de fluor, chlore, brome et iode,
4.2. le groupe méthyle,
5) un groupe alcoxy en C₁-C₃, une ou plusieurs fois substitué par
5.1. un ou des atomes d'halogène, tels que les atomes de fluor, chlore, brome et iode,
6) un groupe alkyle en C₁-C₃, une ou plusieurs fois substitué par
6.1. un ou des atomes d'halogène, tels que les atomes de fluor, chlore, brome et iode,
7) un atome d'hydrogène,
8) dans laquelle R⁴ représente un atome d'hydrogène et R⁵ et R⁶ forment ensemble un radical méthylènedioxy.

10. Composé de formule Ia ou de formule Ib 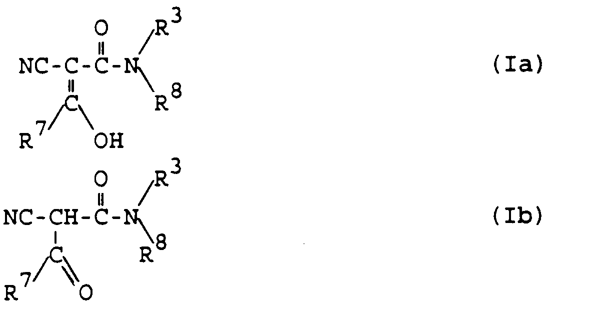 ses formes éventuellement stéréoisomères et/ou éventuellement ses sels physiologiquement acceptables, les radicaux
R⁷, R⁸ et R³ représentant
a) en ce qui concerne R⁷
1) un atome d'hydrogène,
2) un groupe alkyle ayant de 2 à 4 atomes de carbone,
en ce qui concerne R⁸
1) un atome d'hydrogène,
2) le groupe méthyle,
en ce qui concerne R³
1) le groupe phényle,
2) un groupe phényle, une ou plusieurs fois substitué par
2.1. un ou des atomes d'halogène,
2.2. un ou des groupes alkyle ayant de 1 à 3 atomes de carbone, une ou plusieurs fois substitués par un ou des atomes d'halogène, tels que les atomes de fluor, chlore, brome et iode
b) en ce qui concerne R⁷
1) un groupe alkyle ayant de 1 à 4 atomes de carbone,
2) un atome d'hydrogène,
3) CF₃,
en ce qui concerne R⁸
1) un atome d'hydrogène,
2) le groupe méthyle,
3) un groupe alcényle ayant 2 ou 3 atomes de carbone,
en ce qui concerne R³
1) le groupe pyridyle,
2) un groupe pyridyle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone,
3) un groupe pyrimidinyle substitué comme en 2),
4) un groupe thiazolyle, substitué comme en 2) et par
4.1. un ou des groupes alcoxycarbonyle ayant de 1 à 3 atomes de carbone dans la chaîne alkyle,
5) un groupe benzothiazolyle, substitué comme en 2),
6) un groupe benzimidazolyle, substitué comme en 2),
7) un groupe indazolyle, substitué comme en 2),
8) un groupe phényle, une ou plusieurs fois substitué par
8.1. un ou des groupes benzoyle,
8.2. un ou des groupes benzoyle, une ou plusieurs fois substitués par
8.2.1. un ou des atomes d'halogène, tels que les atomes de fluor, chlore, brome et iode,
8.2.2. un ou des groupes alkyle ayant de 1 à 3 atomes de carbone,
8.2.3. un ou des groupes alcoxy ayant de 1 à 3 atomes de carbone,
8.2.4. un ou des groupes alcoxy ayant de 1 à 3 atomes de carbone, une ou plusieurs fois substitués par un ou des atomes d'halogène, tels que les atomes de fluor, chlore, brome et iode,
8.3. un ou des groupes carboxy ou
8.4. un ou des groupes hydroxy,
c) en ce qui concerne R⁷
1) un atome d'hydrogène,
2) un groupe alkyle ayant de 1 à 4 atomes de carbone,
en ce qui concerne R⁸
1) un atome d'hydrogène,
2) le groupe méthyle,
en ce qui concerne R³
1) un radical de formule III
-(CH₂)₄-COOR¹⁰
dans laquelle
R¹⁰ représente
1.1. un atome d'hydrogène,
1.2. un groupe alkyle ayant de 1 à 4 atomes de carbone,
d) en ce qui concerne R⁷
1) un atome d'hydrogène,
2) un groupe alkyle ayant de 1 à 4 atomes de carbone,
R⁸ et R³ forment ensemble, avec l'atome d'azote auquel ils sont liés, un cycle à 4-9 chaînons, substitué par
2.1. un groupe carbonyle sur l'atome de carbone contigu à l'atome d'azote, ou
R⁸ et R³ forment ensemble, avec l'atome d'azote auquel ils sont liés, un cycle pipéridine,
éventuellement substitué par un groupe alkyle ayant de 1 à 3 atomes de carbone.

11. Composé selon la revendication 10, caractérisé en ce que les radicaux R⁷, R⁸ et R³ représentent
a) en ce qui concerne R⁷, un groupe alkyle ayant de 2 à 4 atomes de carbone,
en ce qui concerne R⁸, un atome d'hydrogène,
en ce qui concerne R³
1) le groupe phényle,
2) un groupe phényle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone, une ou plusieurs fois substitués par un ou des atomes d'halogène, tels que les atomes de fluor, chlore, brome et iode,
b) en ce qui concerne R⁷, un groupe alkyle ayant de 1 à 4 atomes de carbone,
en ce qui concerne R⁸, un atome d'hydrogène,
en ce qui concerne R³
1) le groupe pyridyle,
2) un groupe pyridyle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone,
3) un groupe phényle, une ou plusieurs fois substitué par
3.1. un ou des groupes benzoyle,
3.2. un ou des groupes benzoyle, une ou plusieurs fois substitués par
3.2.1. un ou des atomes d'halogène, tels que les atomes de fluor, chlore, brome et iode,
3.2.2. un ou des groupes alkyle ayant de 1 à 3 atomes de carbone,
3.2.3. un ou des groupes alcoxy ayant de 1 à 3 atomes de carbone,
3.2.4. un ou des groupes alcoxy ayant de 1 à 3 atomes de carbone, une ou plusieurs fois substitués par un ou des atomes d'halogène, tels que les atomes de fluor, chlore, brome et iode.

12. Composé qui est le N-(4-chlorodifluorométhoxy)phényl-5-éthylisoxazole-4-carboxamide, le N-(4-méthylsulfonylphényl)-5-méthylisoxazole-4-carboxamide, le N-(5-indolyl)-5-méthylisoxazole-4-carboxamide, le N-(6-indazolyl)-5-méthylisoxazole-4-carboxamide, le N-(5-indazolyl)-5-méthylisoxazole-4-carboxamide, le N-allyl-N-phényl-5-méthylisoxazole-4-carboxamide, le N-(4,6-diméthyl-2-pyridyl)-5-méthylisoxazole-4-carboxamide, le N-(4,6-diméthyl-2-pyrimidinyl)-5-méthylisoxazole-4-carboxamide, le N-(4-trifluorométhoxyphényl)-2-cyano-3-hydroxycrotonamide, le N-[4-(1,1,2,2-tétrafluoroéthoxy)phényl]-2-cyano-3-hydroxycrotonamide, le N-[4-(4-fluorobenzoyl)phényl]-2-cyano-3-hydroxycrotonamide ou le N-(3,4-méthylènedioxyphényl)-2-cyano-3-hydroxy-4-méthylcrotonamide.

13. Utilisation d'au moins un composé de formule I, Ia ou Ib selon l'une quelconque des revendications 7 à 12, de ses formes éventuellement stéréoisomères et/ou d'au moins l'un de ses sels physiologiquement acceptables, pour la fabrication de médicaments destinés au traitement de maladies rhumatismales.

14. Médicament, caractérisé par une teneur en - ou consistant en - au moins un composé de formule I, Ia ou Ib selon l'une quelconque des revendications 7 à 12, ses formes éventuellement stéréoisomères et/ou au moins l'un de ses sels physiologiquement acceptables.

15. Procédé pour la fabrication d'un médicament, caractérisé en ce que l'on met sous une forme d'administration appropriée au moins un composé de formule I, Ia ou Ib selon l'une quelconque des revendications 7 à 12, ses formes éventuellement stéréoisomères et/ou au moins l'un de ses sels physiologiquement acceptables, avec un véhicule physiologiquement acceptable et éventuellement d'autres additifs et/ou adjuvants.

16. Utilisation d'au moins un composé de formule I, Ia ou Ib selon l'une quelconque des revendications 7 à 12, de ses formes éventuellement stéréoisomères et/ou d'au moins l'un de ses sels physiologiquement acceptables, pour la fabrication de médicaments destinés au traitement de maladies cancéreuses.

17. Utilisation d'un ou plusieurs composés selon la revendication 16, pour la fabrication de médicaments destinés au traitement de maladies de type leucémie, sarcome, tumeur des ganglions lymphatiques et cancer de la peau.

## Revendications (Revendications pour l'(les) Etat(s) contractant(s) suivant(s): GR)

1. Utilisation d'au moins un composé de formule I, Ia ou Ib 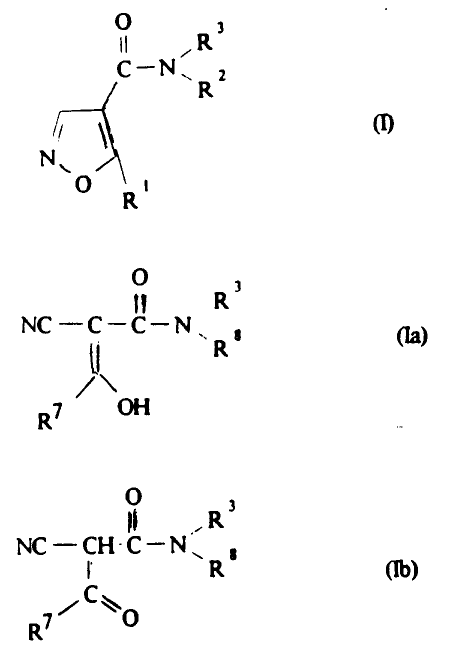 de ses formes éventuellement stéréoisomères et/ou éventuellement d'au moins un de ses sels physiologiquement acceptables,
formules dans lesquelles
R¹ représente
a) un atome d'hydrogène,
b) un groupe alkyle ayant de 1 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alkyle ayant de 1 à 4 atomes de carbone,
c) un groupe phényl-alkyle(C₁-C₂), en particulier le groupe benzyle,
d) un groupe alcényle ayant 2 ou 3 atomes de carbone,
R³ représente
a) un radical hétérocyclique insaturé mono-, bi- ou tricyclique, comportant dans le système cyclique de 3 à 13 atomes de carbone et de 1 à 4 hétéroatomes choisis parmi les atomes d'oxygène, de soufre et d'azote, dont au maximum un est différent de l'atome d'azote, non substitué ou une ou plusieurs fois substitué par
1) un ou des atomes d'halogène, tels que les atomes de fluor, chlore, brome et iode,
2) un ou des groupes alkyle ayant de 1 à 3 atomes de carbone,
3) un ou des groupes alkyle ayant de 1 à 3 atomes de carbone, une ou plusieurs fois substitués par
3.1. un ou des atomes d'halogène, tels que les atomes de fluor, chlore, brome et iode,
4) un ou des groupes alcoxy ayant de 1 à 3 atomes de carbone,
5) un ou des groupes alcoxy ayant de 1 à 3 atomes de carbone, une ou plusieurs fois substitués par
5.1. un ou des atomes d'halogène, tels que les atomes de fluor, chlore, brome et iode,
6) le groupe nitro,
7) hydroxy,
8) carboxy,
9) carbamoyle,
10) oxo,
b) un radical de formule II 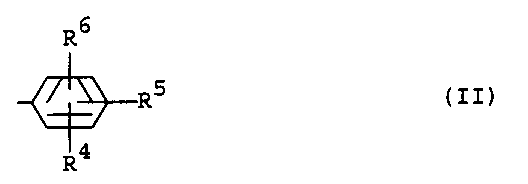 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment ensemble, avec le noyau phényle de la formule II, un cycle naphtalène,
5) dans laquelle R⁴ représente un atome d'hydrogène et R⁵ et R⁶ forment un radical méthylènedioxy,
6) un groupe alcoxy ayant de 1 à 3 atomes de carbone,
7) un groupe alcoxy ayant de 1 à 3 atomes de carbone, une ou plusieurs fois substitué par
7.1. un ou des atomes d'halogène, tels que les atomes de fluor, chlore, brome et iode,
8) un groupe alkyl(C₁-C₃)mercapto,
9) un groupe alkyl(C₁-C₃)mercapto, une ou plusieurs fois substitué par
9.1. un ou des atomes d'halogène, tels que les atomes de fluor, chlore, brome et iode,
10) un atome d'halogène, tel qu'un atome de fluor, chlore, brome ou iode,
11) le groupe nitro,
12) cyano,
13) hydroxy,
14) carboxy,
15) un groupe alkyl(C₁-C₃)sulfonyle,
16) un groupe carbalcoxy, comportant de 1 à 3 atomes de carbone dans la chaîne alkyle,
17) le groupe benzoyle,
18) un groupe benzoyle, une ou plusieurs fois substitué par
18.1. un ou des atomes d'halogène, tels que les atomes de fluor, chlore, brome et iode,
18.2. un ou des groupes alkyle en C₁-C₃,
18.3. un ou des groupes alcoxy en C₁-C₃,
19) le groupe phényle,
20) un groupe phényle, une ou plusieurs fois substitué par
20.1. un ou des groupes alcoxy en C₁-C₃,
20.2. un ou des atomes d'halogène, tels que les atomes de fluor, chlore, brome et iode,
20.3. un ou des groupes alkyle en C₁-C₃,
21) un groupe phénoxy, une ou plusieurs fois substitué par
21.1. un ou des groupes alcoxy en C₁-C₃, une ou plusieurs fois substitués par
21.1.1. un ou des atomes d'halogène, tels que les atomes de fluor, chlore, brome et iode,
21.2. un ou des atomes d'halogène, tels que les atomes de fluor, chlore, brome et iode,
21.3. un ou des groupes alkyle en C₁-C₃ une ou plusieurs fois substitués par
21.3.1. un ou des atomes d'halogène, tels que les atomes de fluor, chlore, brome et iode,
c) un radical de formule III
-(CH₂)ₙ-COOR¹⁰ (III)
dans laquelle
R¹⁰ représente
1) un atome d'hydrogène,
2) un groupe alkyle ayant de 1 à 4 atomes de carbone,
n représente un nombre entier allant de 1 à 12,
d) R² et R³ forment ensemble, avec l'atome d'azote auquel ils sont liés, un cycle à 4-9 chaînons, substitué par
1) un groupe carbonyle sur l'atome de carbone contigu à l'atome d'azote,
e) R² et R³ forment ensemble, avec l'atome d'azote auquel ils sont liés, un cycle à 5-6 chaînons de formule IV 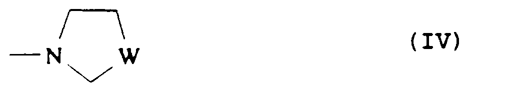 dans laquelle W représente
1) -CH₂-,
2) -CH₂-CH₂-,
3) 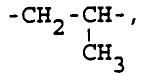
4) 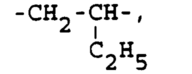
5) 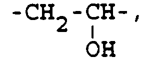
6) -CH₂-O- ou
7) -CH₂-S-,
R⁷ représente
a) un atome d'hydrogène,
b) un groupe alkyle ayant de 1 à 17 atomes de carbone,
c) un groupe alkyle ayant de 1 à 3 atomes de carbone, une ou plusieurs fois substitué par un ou des atomes d'halogène, tels que les atomes de fluor, chlore, brome et iode,
d) un groupe phényl-alkyle(C₁-C₂), en particulier le groupe benzyle,
R⁸ représente
a) un atome d'hydrogène,
b) le groupe méthyle,
c) un groupe alcényle ayant 2 ou 3 atomes de carbone, pour la fabrication de médicaments destinés au traitement de maladies cancéreuses.

2. Utilisation selon la revendication 1, caractérisée en ce que
R¹ représente
a) un atome d'hydrogène,
b) un groupe alkyle ayant de 1 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alkyle ayant de 1 à 4 atomes de carbone,
c) un groupe phényl-alkyle(C₁-C₂), en particulier le groupe benzyle,
d) un groupe alcényle ayant 2 ou 3 atomes de carbone,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
3) le groupe nitro,
4) un ou des groupes alkyle ayant de 1 à 3 atomes de carbone,
5) un ou des groupes alcoxy ayant de 1 à 3 atomes de carbone,
b) un radical de formule II 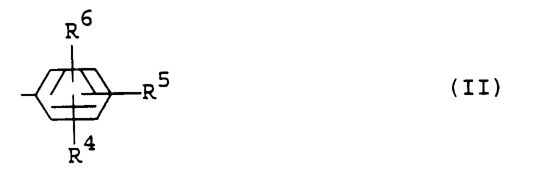 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment un radical méthylènedioxy,
5) un groupe alcoxy ayant de 1 à 3 atomes de carbone,
6) un groupe alcoxy ayant de 1 à 3 atomes de carbone, une ou plusieurs fois substitué par
6.1. un ou des atomes d'halogène, tels que les atomes de fluor, chlore, brome et iode,
7) un groupe alkyl(C₁-C₃)mercapto,
8) un groupe alkyl(C₁-C₃)mercapto, une ou plusieurs fois substitué par
8.1. un ou des atomes d'halogène, tels que les atomes de fluor, chlore, brome et iode,
9) un atome d'halogène, tel qu'un atome de fluor, chlore, brome ou iode,
10) le groupe nitro,
11) le groupe cyano,
12) un groupe alkyl(C₁-C₃)sulfonyle
13) le groupe benzoyle,
14) un groupe benzoyle, une ou plusieurs fois substitué par
14.1. un ou des atomes d'halogène, tels que les atomes de fluor, chlore, brome et iode,
14.2. un ou des groupes alkyle en C₁-C₃,
14.3. un ou des groupes alcoxy en C₁-C₃,
15) un groupe phénoxy, une ou plusieurs fois substitué par
15.1. un ou des groupes alcoxy en C₁-C₃, une ou plusieurs fois substitués par
15.1.1. un ou des atomes d'halogène, tels que les atomes de fluor, chlore, brome et iode,
15.2. un ou des atomes d'halogène, tels que les atomes de fluor, chlore, brome et iode,
15.3. un ou des groupes alkyle en C₁-C₃ une ou plusieurs fois substitués par
15.3.1. un ou des atomes d'halogène, tels que les atomes de fluor, chlore, brome et iode,
c) un radical de formule III
-(CH₂)ₙ-COOR¹⁰ (III)
dans laquelle
R¹⁰ représente
1) un atome d'hydrogène,
2) un groupe alkyle ayant de 1 à 4 atomes de carbone,
n représente un nombre entier allant de 1 à 12,
d) R² et R³ forment ensemble, avec l'atome d'azote auquel ils sont liés, un cycle à 4-9 chaînons, substitué par
1) un groupe carbonyle sur l'atome de carbone contigu à l'atome d'azote,
R⁷ représente
a) un atome d'hydrogène,
b) un groupe alkyle ayant de 1 à 17 atomes de carbone,
c) un groupe alkyle ayant de 1 à 3 atomes de carbone, une ou plusieurs fois substitué par un ou des atomes d'halogène, tels que les atomes de fluor, chlore, brome et iode,
d) un groupe phényl-alkyle(C₁-C₂), en particulier le groupe benzyle,
R⁸ représente
a) un atome d'hydrogène,
b) le groupe méthyle,
c) un groupe alcényle ayant 2 ou 3 atomes de carbone.

3. Utilisation selon la revendication 1, caractérisée que
R¹ représente un groupe alkyle ayant de 1 à 6 atomes de carbone,
R² représente un atome d'hydrogène,
R³ représente
a) un groupe pyridyle, une ou plusieurs fois substitué par un ou des atomes d'halogène, tels que les atomes de fluor, chlore, brome et iode,
b) un radical de formule II 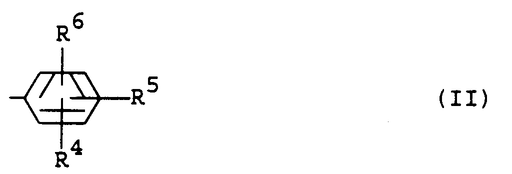 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment un radical méthylènedioxy,
5) un groupe alcoxy ayant de 1 à 3 atomes de carbone,
6) un groupe alcoxy ayant de 1 à 3 atomes de carbone, une ou plusieurs fois substitué par
6.1. un ou des atomes d'halogène, tels que les atomes de fluor, chlore, brome et iode,
7) un atome d'halogène, tel qu'un atome de fluor, chlore, brome ou iode,
8) le groupe nitro,
9) le groupe benzoyle,
10) un groupe benzoyle, une ou plusieurs fois substitué par
10.1. un ou des atomes d'halogène, tels que les atomes de fluor, chlore, brome et iode,
10.2. un ou des groupes alkyle en C₁-C₃,
10.3. un ou des groupes alcoxy en C₁-C₃,
11) un groupe phénoxy, une ou plusieurs fois substitué par
11.1. un ou des groupes alcoxy en C₁-C₃, une ou plusieurs fois substitués par
11.1.1. un ou des atomes d'halogène, tels que les atomes de fluor, chlore, brome et iode,
11.2. un ou des atomes d'halogène, tels que les atomes de fluor, chlore, brome et iode,
11.3. un ou des groupes alkyle en C₁-C₃, une ou plusieurs fois substitués par
11.3.1. un ou des atomes d'halogène, tels que les atomes de fluor, chlore, brome et iode,
R⁷ représente un groupe alkyle ayant de 1 à 17 atomes de carbone,
R⁸ représente un atome d'hydrogène.

4. Utilisation du méthylisoxazole-4-(4-trifluorométhylcarboxanilide) ou du N-(4-trifluorométhylphényl)-2-cyano-3-hydroxycrotonamide, pour la fabrication de médicaments destinés au traitement de maladies cancéreuses.

5. Utilisation d'un ou plusieurs composés selon la revendication 1, pour la fabrication de médicaments destinés au traitement de maladies de type leucémie, sarcome, tumeur des ganglions lymphatiques et cancer de la peau.

6. Procédé pour la préparation du composé de formule I 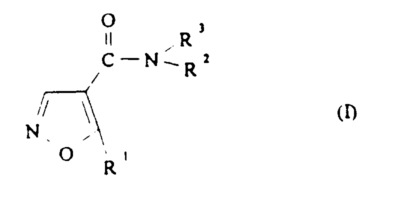 de ses formes éventuellement stéréoisomères et/ou éventuellement de ses sels physiologiquement acceptables, formule dans laquelle
R¹ représente
a) un atome d'hydrogène,
b) un groupe alkyle ayant de 2 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alcényle ayant 2 ou 3 atomes de carbone,
c) le groupe benzyle,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
3) le groupe nitro,
4) un ou des groupes alkyle ayant de 1 à 3 atomes de carbone,
5) un ou des groupes alcoxy ayant de 1 à 3 atomes de carbone,
b) un radical de formule II 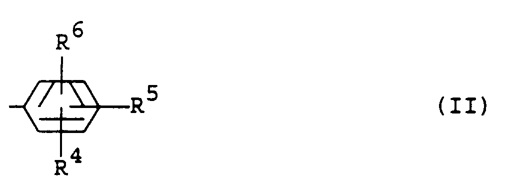 dans laquelle R⁴, R⁵, R⁶ peuvent être identiques ou différents et représentent
1) un atome d'halogène, tel qu'un atome de fluor, chlore, brome ou iode,
2) le groupe nitro,
3) un atome d'hydrogène,
4) un groupe benzoyle, une ou plusieurs fois substitué par
4.1. un ou des atomes d'halogène, tels que les atomes de fluor, chlore, brome et iode,
4.2. le groupe méthyle,
4.3. le groupe méthoxy,
4.4. le groupe benzoyle,
5) un groupe alcoxy en C₁-C₃, une ou plusieurs fois substitué par
5.1. un ou des atomes d'halogène, tels que les atomes de fluor, chlore, brome et iode,
6) un groupe alkyle en C₁-C₃, une ou plusieurs fois substitué par
6.1. un ou des atomes d'halogène, tels que les atomes de fluor, chlore, brome et iode,
7) le groupe hydroxy,
8) un groupe alkylsulfonyle ayant de 1 à 3 atomes de carbone dans la chaîne alkyle,
9) dans laquelle R⁴ représente un atome d'hydrogène et R⁵ et R⁶ forment ensemble un radical méthylènedioxy,
10) le groupe cyano,
11) un groupe alkyl(C₁-C₃)mercapto,
12) le groupe benzoyle,
c) un groupe pyrimidinyle, une ou plusieurs fois substitué par
1) un ou des groupes alkyle ayant de 1 à 3 atomes de carbone,
d) le groupe indolyle ou
e) le groupe indazolinyle,
caractérisé en ce que
a) on fait réagir un composé de formule V 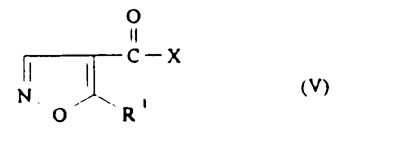 dans laquelle X représente un atome d'halogène, de préférence de chlore ou de brome, et R¹ a la signification donnée dans la formule I,
avec une amine de formule VI 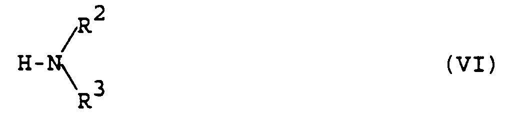 dans laquelle R² et R³ ont les significations données dans la formule I.

7. Procédé selon la revendication 6, caractérisé en ce que l'on prépare un composé de formule I dans lequel
R¹ représente
a) un atome d'hydrogène,
b) un groupe alkyle ayant de 2 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alcényle ayant 2 ou 3 atomes de carbone,
c) le groupe benzyle,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
3) le groupe nitro,
4) un ou des groupes alkyle ayant de 1 à 3 atomes de carbone,
5) un ou des groupes alcoxy ayant de 1 à 3 atomes de carbone,
b) un radical de formule II 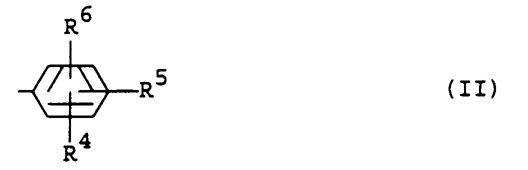 dans laquelle R⁴, R⁵, R⁶ peuvent être identiques ou différents et représentent
1) un atome d'halogène, tel qu'un atome de fluor, chlore, brome ou iode,
2) le groupe nitro,
3) un atome d'hydrogène,
4) un groupe benzoyle, une ou plusieurs fois substitué par
4.1. un ou des atomes d'halogène, tels que les atomes de fluor, chlore, brome et iode,
4.2. le groupe méthyle,
4.3. le groupe méthoxy,
4.4. le groupe benzoyle,
5) un groupe alcoxy en C₁-C₃, une ou plusieurs fois substitué par un ou des atomes d'halogène, tels que les atomes de fluor, chlore, brome et iode,
6) un groupe alkyle en C₁-C₃, une ou plusieurs fois substitué par un ou des atomes d'halogène, tels que les atomes de fluor, chlore, brome et iode,
7) le groupe hydroxy,
8) un groupe alkylsulfonyle ayant de 1 à 3 atomes de carbone dans la chaîne alkyle,
9) dans laquelle R⁴ représente un atome d'hydrogène et R⁵ et R⁶ forment ensemble un radical méthylènedioxy,
10) le groupe cyano,
11) un groupe alkyl(C₁-C₃)mercapto,
12) le groupe benzoyle,
c) un groupe pyrimidinyle, une ou plusieurs fois substitué par un ou des groupes alkyle ayant de 1 à 3 atomes de carbone,
d) le groupe indolyle ou
e) le groupe indazolinyle.

8. Procédé selon la revendication 6, caractérisé en ce que l'on prépare un composé de formule I dans lequel
R¹ représente un groupe alkyle ayant de 2 à 6 atomes de carbone,
R² représente un atome d'hydrogène,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
b) un radical de formule II 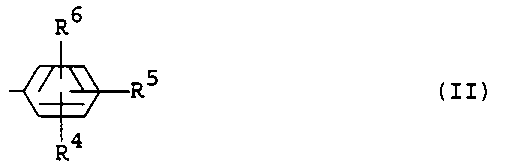 dans laquelle R⁴, R⁵, R⁶ peuvent être identiques ou différents et représentent
1) un atome d'halogène, tel qu'un atome de fluor, chlore, brome ou iode,
2) le groupe nitro,
3) le groupe benzoyle,
4) un groupe benzoyle, une ou plusieurs fois substitué par
4.1. un ou des atomes d'halogène, tels que les atomes de fluor, chlore, brome et iode,
4.2. le groupe méthyle,
5) un groupe alcoxy en C₁-C₃, une ou plusieurs fois substitué par un ou des atomes d'halogène, tels que les atomes de fluor, chlore, brome et iode,
6) un groupe alkyle en C₁-C₃, une ou plusieurs fois substitué par un ou des atomes d'halogène, tels que les atomes de fluor, chlore, brome et iode,
7) un atome d'hydrogène,
8) dans laquelle R⁴ représente un atome d'hydrogène et R⁵ et R⁶ forment ensemble un radical méthylènedioxy.

9. Procédé pour la préparation du composé de formule Ia ou de formule Ib 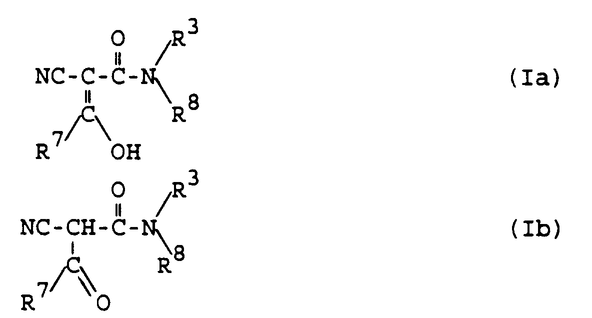 de ses formes éventuellement stéréoisomères et/ou éventuellement de ses sels physiologiquement acceptables, les radicaux R⁷, R⁸ et R³ représentant
a) en ce qui concerne R⁷
1) un atome d'hydrogène,
2) un groupe alkyle ayant de 2 à 4 atomes de carbone,
en ce qui concerne R⁸
1) un atome d'hydrogène,
2) le groupe méthyle,
en ce qui concerne R³
1) le groupe phényle,
2) un groupe phényle, une ou plusieurs fois substitué par
2.1. un ou des atomes d'halogène,
2.2. un ou des groupes alkyle ayant de 1 à 3 atomes de carbone, une ou plusieurs fois substitués par un ou des atomes d'halogène, tels que les atomes de fluor, chlore, brome et iode
b) en ce qui concerne R⁷
1) un groupe alkyle ayant de 1 à 4 atomes de carbone,
2) un atome d'hydrogène,
3) CF₃,
en ce qui concerne R⁸
1) un atome d'hydrogène,
2) le groupe méthyle,
3) un groupe alcényle ayant 2 ou 3 atomes de carbone,
en ce qui concerne R³
1) le groupe pyridyle,
2) un groupe pyridyle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone,
3) un groupe pyrimidinyle substitué comme en 2),
4) un groupe thiazolyle, substitué comme en 2) et par
4.1. un ou des groupes alcoxycarbonyle ayant de 1 à 3 atomes de carbone dans la chaîne alkyle,
5) un groupe benzothiazolyle, substitué comme en 2),
6) un groupe benzimidazolyle, substitué comme en 2),
7) un groupe indazolyle, substitué comme en 2),
8) un groupe phényle, une ou plusieurs fois substitué par
8.1. un ou des groupes benzoyle,
8.2. un ou des groupes benzoyle, une ou plusieurs fois substitués par
8.2.1. un ou des atomes d'halogène, tels que les atomes de fluor, chlore, brome et iode,
8.2.2. un ou des groupes alkyle ayant de 1 à 3 atomes de carbone,
8.2.3. un ou des groupes alcoxy ayant de 1 à 3 atomes de carbone,
8.2.4. un ou des groupes alcoxy ayant de 1 à 3 atomes de carbone, une ou plusieurs fois substitués par un ou des atomes d'halogène, tels que les atomes de fluor, chlore, brome et iode,
8.3. un ou des groupes carboxy ou
8.4. un ou des groupes hydroxy,
c) en ce qui concerne R⁷
1) un atome d'hydrogène,
2) un groupe alkyle ayant de 1 à 4 atomes de carbone,
en ce qui concerne R⁸
1) un atome d'hydrogène,
2) le groupe méthyle,
en ce qui concerne R³
1) un radical de formule III
-(CH₂)₄-COOR¹⁰
dans laquelle
R¹⁰ représente
1.1. un atome d'hydrogène,
1.2. un groupe alkyle ayant de 1 à 4 atomes de carbone,
d) en ce qui concerne R⁷
1) un atome d'hydrogène,
2) un groupe alkyle ayant de 1 à 4 atomes de carbone,
R⁸ et R³ forment ensemble, avec l'atome d'azote auquel ils sont liés, un cycle à 4-9 chaînons, substitué par
2.1. un groupe carbonyle sur l'atome de carbone contigu à l'atome d'azote, ou
R⁸ et R³ forment ensemble, avec l'atome d'azote auquel ils sont liés, un cycle pipéridine,
éventuellement substitué par un groupe alkyle ayant de 1 à 3 atomes de carbone,
caractérisé en ce que
a) on fait réagir un composé de formule V 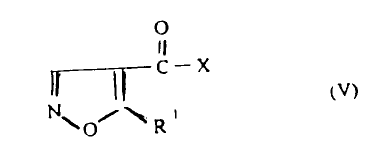 dans laquelle X représente un atome d'halogène, de préférence de chlore ou de brome, et R¹ a la signification donnée dans la formule I,
avec une amine de formule VI 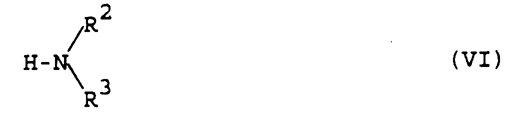 dans laquelle R² et R³ ont les significations données dans les formules Ia et Ib, ou
b) on traite un composé de formule VI 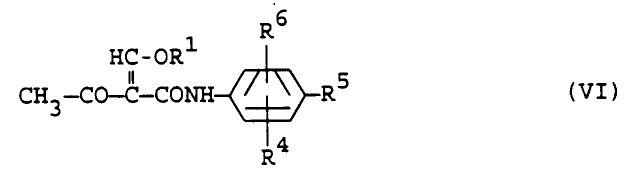 dans laquelle R¹ représente un groupe alkyle en C₁-C₄, et R⁴, R⁵ et R⁶ ont les significations données dans la formule I, par une quantité convenablement au moins équimolaire d'hydroxylamine, dans un solvant organique, ou
c) on fait réagir un composé de formule V, dans lequel X et R¹ on les significations données plus haut, avec une amine aliphatique primaire de formule VII
H₂N-(CH₂)₄-COOR¹⁰ (VII)
dans laquelle R¹⁰ a la signification donnée dans la formule I, et
d) on convertit le composé de formule I, préparé selon les étapes de processus a et c, en présence d'un agent basique, en le composé de formule Ia ou Ib correspondant.

10. Procédé selon la revendication 9, caractérisé en ce que l'on prépare un composé de formule Ia ou Ib, les radicaux R⁷, R⁸ et R³ représentant
a) en ce qui concerne R⁷, un groupe alkyle ayant de 2 à 4 atomes de carbone,
en ce qui concerne R⁸, un atome d'hydrogène,
en ce qui concerne R³
1) le groupe phényle,
2) un groupe phényle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone, une ou plusieurs fois substitués par un ou des atomes d'halogène, tels que les atomes de fluor, chlore, brome et iode,
b) en ce qui concerne R⁷, un groupe alkyle ayant de 1 à 4 atomes de carbone,
en ce qui concerne R⁸ , un atome d'hydrogène,
en ce qui concerne R³
1) le groupe pyridyle,
2) un groupe pyridyle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone,
3) un groupe phényle, une ou plusieurs fois substitué par
3.1. un ou des groupes benzoyle,
3.2. un ou des groupes benzoyle, une ou plusieurs fois substitués par
3.2.1. un ou des atomes d'halogène, tels que les atomes de fluor, chlore, brome et iode,
3.2.2. un ou des groupes alkyle ayant de 1 à 3 atomes de carbone,
3.2.3. un ou des groupes alcoxy ayant de 1 à 3 atomes de carbone,
3.2.4. un ou des groupes alcoxy ayant de 1 à 3 atomes de carbone, une ou plusieurs fois substitués par un ou des atomes d'halogène, tels que les atomes de fluor, chlore, brome et iode.

11. Procédé selon les revendications 6 à 10, caractérisé en ce que l'on prépare le composé qui est le N-(4-chlorodifluorométhoxy)phényl-5-éthylisoxazole-4-carboxamide, le N-(4-méthylsulfonylphényl)-5-méthylisoxazole-4-carboxamide, le N-(5-indolyl)-5-méthylisoxazole-4-carboxamide, le N-(6-indazolyl)-5-méthylisoxazole-4-carboxamide, le N-(5-indazolyl)-5-méthylisoxazole-4-carboxamide, le N-allyl-N-phényl-5-méthylisoxazole-4-carboxamide, le N-(4,6-diméthyl-2-pyridyl)-5-méthylisoxazole-4-carboxamide, le N-(4,6-diméthyl-2-pyrimidinyl)-5-méthylisoxazole-4-carboxamide, le N-(4-trifluorométhoxyphényl)-2-cyano-3-hydroxycrotonamide, le N-[4-(1,1,2,2-tétrafluoroéthoxy)phényl]-2-cyano-3-hydroxycrotonamide, le N-[4-(4-fluorobenzoyl)phényl]-2-cyano-3-hydroxycrotonamide ou le N-(3,4-méthylènedioxyphényl)-2-cyano-3-hydroxy-4-méthylcrotonamide.

12. Utilisation d'au moins un composé de formule I, Ia ou Ib selon l'une quelconque des revendications 6 à 11, de ses formes éventuellement stéréoisomères et/ou d'au moins l'un de ses sels physiologiquement acceptables, pour la fabrication de médicaments destinés au traitement de maladies rhumatismales.

13. Procédé pour la fabrication d'un médicament, caractérisé en ce que l'on met sous une forme d'administration appropriée au moins un composé de formule I, Ia ou Ib préparé selon l'une quelconque des revendications 6 à 11, ses formes éventuellement stéréoisomères et/ou au moins l'un de ses sels physiologiquement acceptables, avec un véhicule physiologiquement acceptable et éventuellement d'autres additifs et/ou adjuvants.

14. Utilisation d'au moins un composé de formule I, Ia ou Ib selon l'une quelconque des revendications 6 à 11, de ses formes éventuellement stéréoisomères et/ou d'au moins l'un de ses sels physiologiquement acceptables, pour la fabrication de médicaments destinés au traitement de maladies cancéreuses.

15. Utilisation d'un ou plusieurs composés selon la revendication 14, pour la fabrication de médicaments destinés au traitement de maladies de type leucémie, sarcome, tumeur des ganglions lymphatiques et cancer de la peau.

## Revendications (Revendications pour l'(les) Etat(s) contractant(s) suivant(s): ES)

1. Utilisation d'au moins un composé de formule I, Ia ou Ib 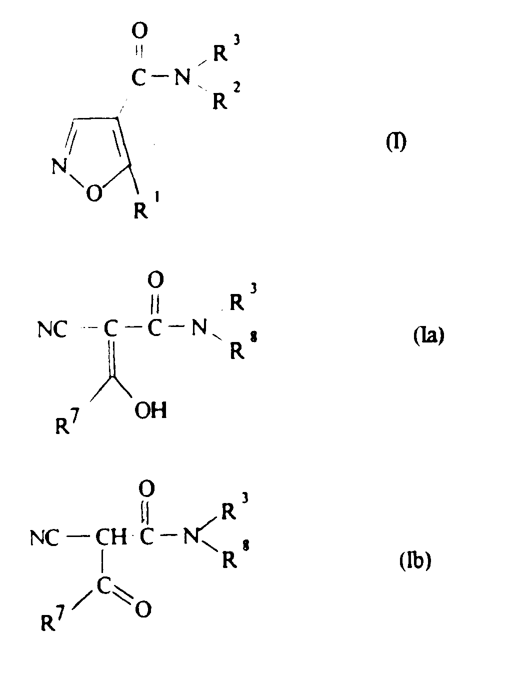 de ses formes éventuellement stéréoisomères et/ou éventuellement d'au moins un de ses sels physiologiquement acceptables,
formules dans lesquelles
R¹ représente
a) un atome d'hydrogène,
b) un groupe alkyle ayant de 1 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alkyle ayant de 1 à 4 atomes de carbone,
c) un groupe phényl-alkyle(C₁-C₂), en particulier le groupe benzyle,
d) un groupe alcényle ayant 2 ou 3 atomes de carbone,
R³ représente
a) un radical hétérocyclique insaturé mono-, bi- ou tricyclique, comportant dans le système cyclique de 3 à 13 atomes de carbone et de 1 à 4 hétéroatomes choisis parmi les atomes d'oxygène, de soufre et d'azote, dont au maximum un est différent de l'atome d'azote, non substitué ou une ou plusieurs fois substitué par
1) un ou des atomes d'halogène, tels que les atomes de fluor, chlore, brome et iode,
2) un ou des groupes alkyle ayant de 1 à 3 atomes de carbone,
3) un ou des groupes alkyle ayant de 1 à 3 atomes de carbone, une ou plusieurs fois substitués par
3.1. un ou des atomes d'halogène, tels que les atomes de fluor, chlore, brome et iode,
4) un ou des groupes alcoxy ayant de 1 à 3 atomes de carbone,
5) un ou des groupes alcoxy ayant de 1 à 3 atomes de carbone, une ou plusieurs fois substitués par
5.1. un ou des atomes d'halogène, tels que les atomes de fluor, chlore, brome et iode,
6) le groupe nitro,
7) hydroxy,
8) carboxy,
9) carbamoyle,
10) oxo,
b) un radical de formule II 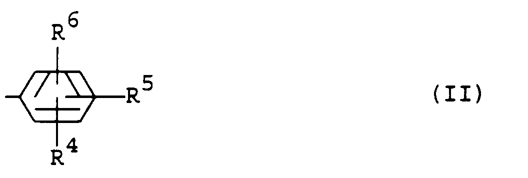 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment ensemble, avec le noyau phényle de la formule II, un cycle naphtalène,
5) dans laquelle R⁴ représente un atome d'hydrogène et R⁵ et R⁶ forment un radical méthylènedioxy,
6) un groupe alcoxy ayant de 1 à 3 atomes de carbone,
7) un groupe alcoxy ayant de 1 à 3 atomes de carbone, une ou plusieurs fois substitué par
7.1. un ou des atomes d'halogène, tels que les atomes de fluor, chlore, brome et iode,
8) un groupe alkyl(C₁-C₃)mercapto,
9) un groupe alkyl(C₁-C₃)mercapto, une ou plusieurs fois substitué par
9.1. un ou des atomes d'halogène, tels que les atomes de fluor, chlore, brome et iode,
10) un atome d'halogène, tel qu'un atome de fluor, chlore, brome ou iode,
11) le groupe nitro,
12) cyano,
13) hydroxy,
14) carboxy,
15) un groupe alkyl(C₁-C₃)sulfonyle,
16) un groupe carbalcoxy, comportant de 1 à 3 atomes de carbone dans la chaîne alkyle,
17) le groupe benzoyle,
18) un groupe benzoyle, une ou plusieurs fois substitué par
18.1. un ou des atomes d'halogène, tels que les atomes de fluor, chlore, brome et iode,
18.2. un ou des groupes alkyle en C₁-C₃,
18.3. un ou des groupes alcoxy en C₁-C₃,
19) le groupe phényle,
20) un groupe phényle, une ou plusieurs fois substitué par
20.1. un ou des groupes alcoxy en C₁-C₃,
20.2. un ou des atomes d'halogène, tels que les atomes de fluor, chlore, brome et iode,
20.3. un ou des groupes alkyle en C₁-C₃,
21) un groupe phénoxy, une ou plusieurs fois substitué par
21.1. un ou des groupes alcoxy en C₁-C₃, une ou plusieurs fois substitués par
21.1.1. un ou des atomes d'halogène, tels que les atomes de fluor, chlore, brome et iode,
21.2. un ou des atomes d'halogène, tels que les atomes de fluor, chlore, brome et iode,
21.3. un ou des groupes alkyle en C₁-C₃ une ou plusieurs fois substitués par
21.3.1. un ou des atomes d'halogène, tels que les atomes de fluor, chlore, brome et iode,
c) un radical de formule III
-(CH₂)ₙCOOR¹⁰ (III)
dans laquelle
R¹⁰ représente
1) un atome d'hydrogène,
2) un groupe alkyle ayant de 1 à 4 atomes de carbone,
n représente un nombre entier allant de 1 à 12,
d) R² et R³ forment ensemble, avec l'atome d'azote auquel ils sont liés, un cycle à 4-9 chaînons, substitué par
1) un groupe carbonyle sur l'atome de carbone contigu à l'atome d'azote,
e) R² et R³ forment ensemble, avec l'atome d'azote auquel ils sont liés, un cycle à 5-6 chaînons de formule IV 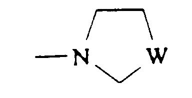 dans laquelle W représente
1) -CH₂-,
2) -CH₂-CH₂-,
3) 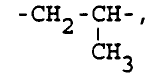
4) 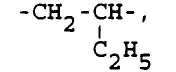
5) 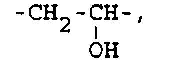
6) -CH₂-O- ou
7) -CH₂-S-,
R⁷ représente
a) un atome d'hydrogène,
b) un groupe alkyle ayant de 1 à 17 atomes de carbone,
c) un groupe alkyle ayant de 1 à 3 atomes de carbone, une ou plusieurs fois substitué par un ou des atomes d'halogène, tels que les atomes de fluor, chlore, brome et iode,
d) un groupe phényl-alkyle(C₁-C₂), en particulier le groupe benzyle,
R⁸ représente
a) un atome d'hydrogène,
b) le groupe méthyle,
c) un groupe alcényle ayant 2 ou 3 atomes de carbone, pour la fabrication de médicaments destinés au traitement de maladies cancéreuses.

2. Utilisation selon la revendication 1, caractérisée en ce que
R¹ représente
a) un atome d'hydrogène,
b) un groupe alkyle ayant de 1 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alkyle ayant de 1 à 4 atomes de carbone,
c) un groupe phényl-alkyle(C₁-C₂), en particulier le groupe benzyle,
d) un groupe alcényle ayant 2 ou 3 atomes de carbone,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
3) le groupe nitro,
4) un ou des groupes alkyle ayant de 1 à 3 atomes de carbone,
5) un ou des groupes alcoxy ayant de 1 à 3 atomes de carbone,
b) un radical de formule II 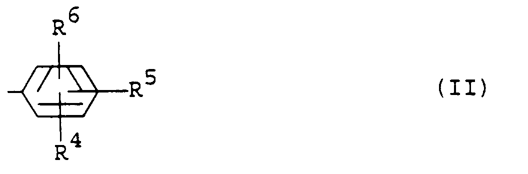 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment un radical méthylènedioxy,
5) un groupe alcoxy ayant de 1 à 3 atomes de carbone,
6) un groupe alcoxy ayant de 1 à 3 atomes de carbone, une ou plusieurs fois substitué par
6.1. un ou des atomes d'halogène, tels que les atomes de fluor, chlore, brome et iode,
7) un groupe alkyl(C₁-C₃)mercapto,
8) un groupe alkyl(C₁-C₃)mercapto, une ou plusieurs fois substitué par
8.1. un ou des atomes d'halogène, tels que les atomes de fluor, chlore, brome et iode,
9) un atome d'halogène, tel qu'un atome de fluor, chlore, brome ou iode,
10) le groupe nitro,
11) le groupe cyano,
12) un groupe alkyl(C₁-C₃)sulfonyle
13) le groupe benzoyle,
14) un groupe benzoyle, une ou plusieurs fois substitué par
14.1. un ou des atomes d'halogène, tels que les atomes de fluor, chlore, brome et iode,
14.2. un ou des groupes alkyle en C₁-C₃,
14.3. un ou des groupes alcoxy en C₁-C₃,
15) un groupe phénoxy, une ou plusieurs fois substitué par
15.1. un ou des groupes alcoxy en C₁-C₃, une ou plusieurs fois substitués par
15.1.1. un ou des atomes d'halogène, tels que les atomes de fluor, chlore, brome et iode,
15.2. un ou des atomes d'halogène, tels que les atomes de fluor, chlore, brome et iode,
15.3. un ou des groupes alkyle en C₁-C₃ une ou plusieurs fois substitués par
15.3.1. un ou des atomes d'halogène, tels que les atomes de fluor, chlore, brome et iode,
c) un radical de formule III
-(CH₂)ₙ-COOR¹⁰ (III)
dans laquelle
R¹⁰ représente
1) un atome d'hydrogène,
2) un groupe alkyle ayant de 1 à 4 atomes de carbone,
n représente un nombre entier allant de 1 à 12,
d) R² et R³ forment ensemble, avec l'atome d'azote auquel ils sont liés, un cycle à 4-9 chaînons, substitué par
1) un groupe carbonyle sur l'atome de carbone contigu à l'atome d'azote,
R⁷ représente
a) un atome d'hydrogène,
b) un groupe alkyle ayant de 1 à 17 atomes de carbone,
c) un groupe alkyle ayant de 1 à 3 atomes de carbone, une ou plusieurs fois substitué par un ou des atomes d'halogène, tels que les atomes de fluor, chlore, brome et iode,
d) un groupe phényl-alkyle(C₁-C₂), en particulier le groupe benzyle,
R⁸ représente
a) un atome d'hydrogène,
b) le groupe méthyle,
c) un groupe alcényle ayant 2 ou 3 atomes de carbone.

3. Utilisation selon la revendication 1, caractérisée que
R¹ représente un groupe alkyle ayant de 1 à 6 atomes de carbone,
R² représente un atome d'hydrogène,
R³ représente
a) un groupe pyridyle, une ou plusieurs fois substitué par un ou des atomes d'halogène, tels que les atomes de fluor, chlore, brome et iode,
b) un radical de formule II 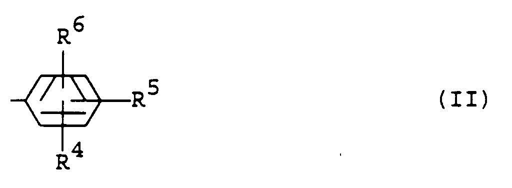 dans laquelle R⁴, R⁵ et R⁶ peuvent être identiques ou différents et représentent
1) un atome d'hydrogène,
2) le groupe phénoxy,
3) un groupe alkyle ayant de 1 à 3 atomes de carbone, une ou plusieurs fois substitué par
3.1. un ou des atomes d'halogène, tels que les atomes de fluor, chlore, brome et iode,
4) dans laquelle R⁴ représente un atome d'hydrogène et R⁵ et R⁶ forment un radical méthylènedioxy,
5) un groupe alcoxy ayant de 1 à 3 atomes de carbone,
6) un groupe alcoxy ayant de 1 à 3 atomes de carbone, une ou plusieurs fois substitué par
6.1. un ou des atomes d'halogène, tels que les atomes de fluor, chlore, brome et iode,
7) un atome d'halogène, tel qu'un atome de fluor, chlore, brome ou iode,
8) le groupe nitro,
9) le groupe benzoyle,
10) un groupe benzoyle, une ou plusieurs fois substitué par
10.1. un ou des atomes d'halogène, tels que les atomes de fluor, chlore, brome et iode,
10.2. un ou des groupes alkyle en C₁-C₃,
10.3. un ou des groupes alcoxy en C₁-C₃,
11) un groupe phénoxy, une ou plusieurs fois substitué par
11.1. un ou des groupes alcoxy en C₁-C₃, une ou plusieurs fois substitués par
11.1.1. un ou des atomes d'halogène, tels que les atomes de fluor, chlore, brome et iode,
11.2. un ou des atomes d'halogène, tels que les atomes de fluor, chlore, brome et iode,
11.3. un ou des groupes alkyle en C₁-C₃, une ou plusieurs fois substitués par
11.3.1. un ou des atomes d'halogène, tels que les atomes de fluor, chlore, brome et iode,
R⁷ représente un groupe alkyle ayant de 1 à 17 atomes de carbone,
R⁸ représente un atome d'hydrogène.

4. Utilisation du méthylisoxazole-4-(4-trifluorométhylcarboxanilide) ou du N-(4-trifluorométhylphényl)-2-cyano-3-hydroxycrotonamide, pour la fabrication de médicaments destinés au traitement de maladies cancéreuses.

5. Utilisation d'un ou plusieurs composés selon la revendication 1, pour la fabrication de médicaments destinés au traitement de maladies de type leucémie, sarcome, tumeur des ganglions lymphatiques et cancer de la peau.

6. Procédé pour la préparation du composé de formule I 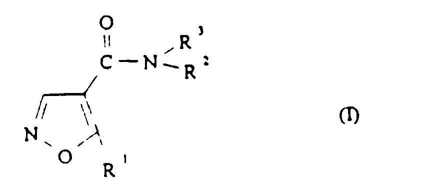 de ses formes éventuellement stéréoisomères et/ou éventuellement de ses sels physiologiquement acceptables,
formule dans laquelle
R¹ représente
a) un atome d'hydrogène,
b) un groupe alkyle ayant de 2 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alcényle ayant 2 ou 3 atomes de carbone,
c) le groupe benzyle,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
3) le groupe nitro,
4) un ou des groupes alkyle ayant de 1 à 3 atomes de carbone,
5) un ou des groupes alcoxy ayant de 1 à 3 atomes de carbone,
b) un radical de formule II 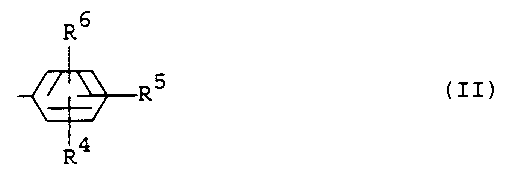 dans laquelle R⁴, R⁵, R⁶ peuvent être identiques ou différents et représentent
1) un atome d'halogène, tel qu'un atome de fluor, chlore, brome ou iode,
2) le groupe nitro,
3) un atome d'hydrogène,
4) un groupe benzoyle, une ou plusieurs fois substitué par
4.1. un ou des atomes d'halogène, tels que les atomes de fluor, chlore, brome et iode,
4.2. le groupe méthyle,
4.3. le groupe méthoxy,
4.4. le groupe benzoyle,
5) un groupe alcoxy en C₁-C₃, une ou plusieurs fois substitué par
5.1. un ou des atomes d'halogène, tels que les atomes de fluor, chlore, brome et iode,
6) un groupe alkyle en C₁-C₃, une ou plusieurs fois substitué par
6.1. un ou des atomes d'halogène, tels que les atomes de fluor, chlore, brome et iode,
7) le groupe hydroxy,
8) un groupe alkylsulfonyle ayant de 1 à 3 atomes de carbone dans la chaîne alkyle,
9) dans laquelle R⁴ représente un atome d'hydrogène et R⁵ et R⁶ forment ensemble un radical méthylènedioxy,
10) le groupe cyano,
11) un groupe alkyl(C₁-C₃)mercapto,
12) le groupe benzoyle,
c) un groupe pyrimidinyle, une ou plusieurs fois substitué par
1) un ou des groupes alkyle ayant de 1 à 3 atomes de carbone,
d) le groupe indolyle ou
e) le groupe indazolinyle,
caractérisé en ce que
a) on fait réagir un composé de formule V 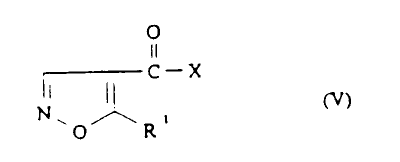 dans laquelle X représente un atome d'halogène, de préférence de chlore ou de brome, et R¹ a la signification donnée dans la formule I,
avec une amine de formule VI 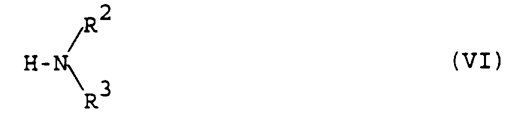 dans laquelle R² et R³ ont les significations données dans la formule I.

7. Procédé selon la revendication 6, caractérisé en ce que l'on prépare un composé de formule I dans lequel
R¹ représente
a) un atome d'hydrogène,
b) un groupe alkyle ayant de 2 à 6 atomes de carbone,
c) un groupe alkyle ayant de 1 à 4 atomes de carbone, une ou plusieurs fois substitué par
1) un ou des atomes d'halogène, tels que les atomes de fluor, chlore, brome et iode,
d) le groupe phényle,
R² représente
a) un atome d'hydrogène,
b) un groupe alcényle ayant 2 ou 3 atomes de carbone,
c) le groupe benzyle,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
3) le groupe nitro,
4) un ou des groupes alkyle ayant de 1 à 3 atomes de carbone,
5) un ou des groupes alcoxy ayant de 1 à 3 atomes de carbone,
b) un radical de formule II 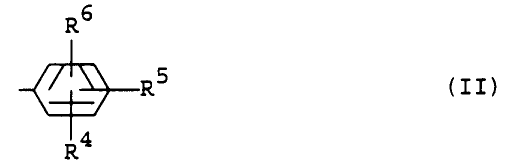 dans laquelle R⁴, R⁵, R⁶ peuvent être identiques ou différents et représentent
1) un atome d'halogène, tel qu'un atome de fluor, chlore, brome ou iode,
2) le groupe nitro,
3) un atome d'hydrogène,
4) un groupe benzoyle, une ou plusieurs fois substitué par
4.1. un ou des atomes d'halogène, tels que les atomes de fluor, chlore, brome et iode,
4.2. le groupe méthyle,
4.3. le groupe méthoxy,
4.4. le groupe benzoyle,
5) un groupe alcoxy en C₁-C₃, une ou plusieurs fois substitué par un ou des atomes d'halogène, tels que les atomes de fluor, chlore, brome et iode,
6) un groupe alkyle en C₁-C₃, une ou plusieurs fois substitué par un ou des atomes d'halogène, tels que les atomes de fluor, chlore, brome et iode,
7) le groupe hydroxy,
8) un groupe alkylsulfonyle ayant de 1 à 3 atomes de carbone dans la chaîne alkyle,
9) dans laquelle R⁴ représente un atome d'hydrogène et R⁵ et R⁶ forment ensemble un radical méthylènedioxy,
10) le groupe cyano,
11) un groupe alkyl(C₁-C₃)mercapto,
12) le groupe benzoyle,
c) un groupe pyrimidinyle, une ou plusieurs fois substitué par un ou des groupes alkyle ayant de 1 à 3 atomes de carbone,
d) le groupe indolyle ou
e) le groupe indazolinyle.

8. Procédé selon la revendication 6, caractérisé en ce que l'on prépare un composé de formule I dans lequel
R¹ représente un groupe alkyle ayant de 2 à 6 atomes de carbone,
R² représente un atome d'hydrogène,
R³ représente
a) un groupe pyridyle, une ou plusieurs fois substitué par
1) un ou des atomes d'hydrogène,
2) un ou des atomes d'halogène, tels que les atomes de fluor, chlore, brome et iode,
b) un radical de formule II 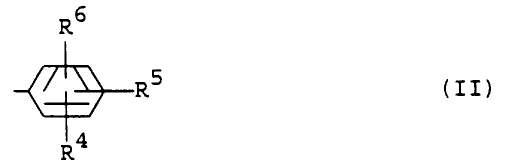 dans laquelle R⁴, R⁵, R⁶ peuvent être identiques ou différents et représentent
1) un atome d'halogène, tel qu'un atome de fluor, chlore, brome ou iode,
2) le groupe nitro,
3) le groupe benzoyle,
4) un groupe benzoyle, une ou plusieurs fois substitué par
4.1. un ou des atomes d'halogène, tels que les atomes de fluor, chlore, brome et iode,
4.2. le groupe méthyle,
5) un groupe alcoxy en C₁-C₃, une ou plusieurs fois substitué par un ou des atomes d'halogène, tels que les atomes de fluor, chlore, brome et iode,
6) un groupe alkyle en C₁-C₃, une ou plusieurs fois substitué par un ou des atomes d'halogène, tels que les atomes de fluor, chlore, brome et iode,
7) un atome d'hydrogène,
8) dans laquelle R⁴ représente un atome d'hydrogène et R⁵ et R⁶ forment ensemble un radical méthylènedioxy.

9. Procédé pour la préparation du composé de formule Ia ou de formule Ib 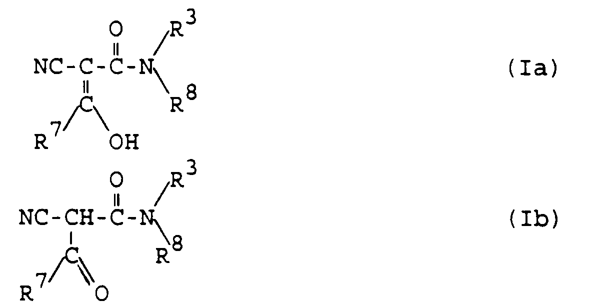 de ses formes éventuellement stéréoisomères et/ou éventuellement de ses sels physiologiquement acceptables, les radicaux R⁷, R⁸ et R³ représentant
a) en ce qui concerne R⁷
1) un atome d'hydrogène,
2) un groupe alkyle ayant de 2 à 4 atomes de carbone,
en ce qui concerne R⁸
1) un atome d'hydrogène,
2) le groupe méthyle,
en ce qui concerne R³
1) le groupe phényle,
2) un groupe phényle, une ou plusieurs fois substitué par
2.1. un ou des atomes d'halogène,
2.2. un ou des groupes alkyle ayant de 1 à 3 atomes de carbone, une ou plusieurs fois substitués par un ou des atomes d'halogène, tels que les atomes de fluor, chlore, brome et iode
b) en ce qui concerne R⁷
1) un groupe alkyle ayant de 1 à 4 atomes de carbone,
2) un atome d'hydrogène,
3) CF₃,
en ce qui concerne R⁸
1) un atome d'hydrogène,
2) le groupe méthyle,
3) un groupe alcényle ayant 2 ou 3 atomes de carbone,
en ce qui concerne R³
1) le groupe pyridyle,
2) un groupe pyridyle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone,
3) un groupe pyrimidinyle substitué comme en 2),
4) un groupe thiazolyle, substitué comme en 2) et par
4.1. un ou des groupes alcoxycarbonyle ayant de 1 à 3 atomes de carbone dans la chaîne alkyle,
5) un groupe benzothiazolyle, substitué comme en 2),
6) un groupe benzimidazolyle, substitué comme en 2),
7) un groupe indazolyle, substitué comme en 2),
8) un groupe phényle, une ou plusieurs fois substitué par
8.1. un ou des groupes benzoyle,
8.2. un ou des groupes benzoyle, une ou plusieurs fois substitués par
8.2.1. un ou des atomes d'halogène, tels que les atomes de fluor, chlore, brome et iode,
8.2.2. un ou des groupes alkyle ayant de 1 à 3 atomes de carbone,
8.2.3. un ou des groupes alcoxy ayant de 1 à 3 atomes de carbone,
8.2.4. un ou des groupes alcoxy ayant de 1 à 3 atomes de carbone, une ou plusieurs fois substitués par un ou des atomes d'halogène, tels que les atomes de fluor, chlore, brome et iode,
8.3. un ou des groupes carboxy ou
8.4. un ou des groupes hydroxy,
c) en ce qui concerne R⁷
1) un atome d'hydrogène,
2) un groupe alkyle ayant de 1 à 4 atomes de carbone,
en ce qui concerne R⁸
1) un atome d'hydrogène,
2) le groupe méthyle,
en ce qui concerne R³
1) un radical de formule III
-(CH₂)₄-COOR¹⁰
dans laquelle
R¹⁰ représente
1.1. un atome d'hydrogène,
1.2. un groupe alkyle ayant de 1 à 4 atomes de carbone,
d) en ce qui concerne R⁷
1) un atome d'hydrogène,
2) un groupe alkyle ayant de 1 à 4 atomes de carbone,
R⁸ et R³ forment ensemble, avec l'atome d'azote auquel ils sont liés, un cycle à 4-9 chaînons, substitué par
2.1. un groupe carbonyle sur l'atome de carbone contigu à l'atome d'azote, ou
R⁸ et R³ forment ensemble, avec l'atome d'azote auquel ils sont liés, un cycle pipéridine,
éventuellement substitué par un groupe alkyle ayant de 1 à 3 atomes de carbone,
caractérisé en ce que
a) on fait réagir un composé de formule V 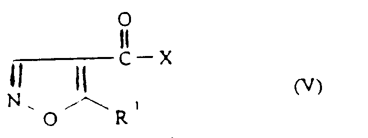 dans laquelle X représente un atome d'halogène, de préférence de chlore ou de brome, et R¹ a la signification donnée dans la formule I,
avec une amine de formule VI 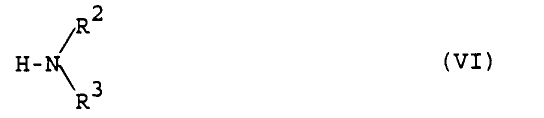 dans laquelle R² et R³ ont les significations données dans les formules Ia et Ib.
b) on traite un composé de formule VI 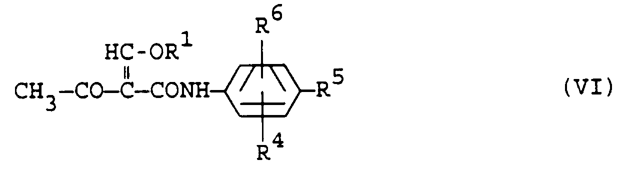 dans laquelle R¹ représente un groupe alkyle en C₁-C₄, et R⁴, R⁵ et R⁶ ont les significations données dans la formule I, par une quantité convenablement au moins équimolaire d'hydroxylamine, dans un solvant organique, ou
c) on fait réagir un composé de formule V, dans lequel X et R¹ on les significations données plus haut, avec une amine aliphatique primaire de formule VII
H₂N-(CH₂)₄-COOR¹⁰ (VII)
dans laquelle R¹⁰ a la signification donnée dans la formule I, et
d) on convertit le composé de formule I, préparé selon les étapes de processus a et c, en présence d'un agent basique, en le composé de formule Ia ou Ib correspondant.

10. Procédé selon la revendication 9, caractérisé en ce que l'on prépare un composé de formule Ia ou Ib, les radicaux R⁷, R⁸ et R³ représentant
a) en ce qui concerne R⁷, un groupe alkyle ayant de 2 à 4 atomes de carbone,
en ce qui concerne R⁸, un atome d'hydrogène,
en ce qui concerne R³
1) le groupe phényle,
2) un groupe phényle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone, une ou plusieurs fois substitués par un ou des atomes d'halogène, tels que les atomes de fluor, chlore, brome et iode,
b) en ce qui concerne R⁷, un groupe alkyle ayant de 1 à 4 atomes de carbone,
en ce qui concerne R⁸, un atome d'hydrogène,
en ce qui concerne R³
1) le groupe pyridyle,
2) un groupe pyridyle, une ou plusieurs fois substitué par
2.1. un ou des atomes d'halogène, tels que les atomes de fluor, chlore, brome et iode,
2.2. un ou des groupes alkyle ayant de 1 à 3 atomes de carbone,
3) un groupe phényle, une ou plusieurs fois substitué par
3.1. un ou des groupes benzoyle,
3.2. un ou des groupes benzoyle, une ou plusieurs fois substitués par
3.2.1. un ou des atomes d'halogène, tels que les atomes de fluor, chlore, brome et iode,
3.2.2. un ou des groupes alkyle ayant de 1 à 3 atomes de carbone,
3.2.3. un ou des groupes alcoxy ayant de 1 à 3 atomes de carbone,
3.2.4. un ou des groupes alcoxy ayant de 1 à 3 atomes de carbone, une ou plusieurs fois substitués par un ou des atomes d'halogène, tels que les atomes de fluor, chlore, brome et iode.

11. Procédé selon les revendications 6 à 10, caractérisé en ce que l'on prépare le composé qui est le N-(4-chlorodifluorométhoxy)phényl-5-éthylisoxazole-4-carboxamide, le N-(4-méthylsulfonylphényl)-5-méthylisoxazole-4-carboxamide, le N-(5-indolyl)-5-méthylisoxazole-4-carboxamide, le N-(6-indazolyl)-5-méthylisoxazole-4-carboxamide, le N-(5-indazolyl)-5-méthylisoxazole-4-carboxamide, le N-allyl-N-phényl-5-méthylisoxazole-4-carboxamide, le N-(4,6-diméthyl-2-pyridyl)-5-méthylisoxazole-4-carboxamide, le N-(4,6-diméthyl-2-pyrimidinyl)-5-méthylisoxazole-4-carboxamide, le N-(4-trifluorométhoxyphényl)-2-cyano-3-hydroxycrotonamide, le N-[4-(1,1,2,2-tétrafluoroéthoxy)phényl]-2-cyano-3-hydroxycrotonamide, le N-[4-(4-fluorobenzoyl)phényl]-2-cyano-3-hydroxycrotonamide ou le N-(3,4-méthylènedioxyphényl)-2-cyano-3-hydroxy-4-méthylcrotonamide.

12. Utilisation d'au moins un composé de formule I, Ia ou Ib selon l'une quelconque des revendications 6 à 11, de ses formes éventuellement stéréoisomères et/ou d'au moins l'un de ses sels physiologiquement acceptables, pour la fabrication de médicaments destinés au traitement de maladies rhumatismales.

13. Utilisation d'au moins un composé de formule I, Ia ou Ib selon l'une quelconque des revendications 6 à 11, de ses formes éventuellement stéréoisomères et/ou d'au moins l'un de ses sels physiologiquement acceptables, pour la fabrication de médicaments destinés au traitement de maladies cancéreuses.

14. Utilisation d'un ou plusieurs composés selon la revendication 13, pour la fabrication de médicaments destinés au traitement de maladies de type leucémie, sarcome, tumeur des ganglions lymphatiques et cancer de la peau.
